# NEW EUROPEAN PATENT SPECIFICATION

(11) **EP 0 322 926 B2**
(45) Date of publication and mention of the opposition decision: **31.03.2004**
(45) Mention of the grant of the patent: 11.08.1999
(21) Application number: 88121915.8
(22) Date of filing: 30.12.1988
(51) Int. Cl.: G01N 33/53, G01N 33/58, G01N 33/542

(54) **Assays utilizing improved chemiluminescent esters, thioesters and amides**
Für Tests verwendbare chemilumineszierende Ester, Thioester und Amide
Essais utilisant des esters, des thioesters et des amides chimioluminescents

(30) Priority: 31.12.1987 US 140040; 29.12.1988 US 291843
(43) Date of publication of application: 05.07.1989
(60) Divisional application: 98123411.5
(73) Proprietor: Nichols Institute Diagnostics, San Juan Capistrano, California 92675 (US)
(72) Inventor: McCapra, Frank, Seaford East Sussex (GB); Beheshti, Iraj, Edina, MN 55435 (US); Hart, Russell C., Minnetonka, MN 55343 (US); Koelling, Harlen, Waverly, IA 50677 (US); Patel, Ashokkumar, Minnetonka, MN 55344 (US); Ramakrishnan, Kastooriranganathan, Plymouth, MN 55441 (US)
(74) Representative: Cornish, Kristina Victoria Joy

(56) References cited:
- EP-A- 0 082 636
- EP-A- 0 257 541
- EP-A- 0 263 657
- EP-A- 0 273 115
- EP-A- 0 309 230
- EP-A- 0 324 202
- CLINICAL CHEMISTRY, vol. 29, no. 8, August 1983, pages 1474-1479, Winston, US; I. WEEKS et al.: "Acridinium esters as high-specific-activity labels in immunoassay"
- McCapra et al. Proc.R.Soc.Lond.215.1982. pp 247-272
- "The Acridines". A Albert, Edward Arnold. London 1966
- "Comprehensive Chemical Kinetics", Ester Formation and Hydrolysis and related Reactions.
- March J., "Advanced Organic Chemistry : Reaction mechanisms and Structure"

## Description

### FIELD OF THE INVENTION

This invention relates to assays and immunoassays utilizing chemiluminescent conjugates as the label.

### BACKGROUND OF THE INVENTION

Certain compounds give off light or "chemiluminesce" upon treatment with peroxide or molecular oxygen at high pH. Light is produced by the decay of a chemical intermediate which is formed by the attack of peroxide or molecular oxygen at an sp² or sp³ hybridized carbon center. The carbon center which is attacked can be part of a chain or a ring or ring system.

The characteristics and behavior of some of these chemiluminescent compounds is more fully described in McCapra, "Chemiluminescence of Organic Compounds," in Progress in Organic Chemistry, vol. 8, Carruthers and Sutherland ed. (Wiley & Sons 1973) which is incorporated herein by reference.

Chemiluminescent compounds have been used as labels in prototype assays including immunoassays for many years. Examples of such use can be found in U.S. Patent Nos. 4,383,031, 4,380,580, 4,226,993 and many others. In general, however, these compounds have not been used in commercial assays, because of their lack of stability in aqueous solutions. This lack of stability is especially important for the subclass of chemiluminescent compounds comprising certain esters, thioesters and amides. These compounds react according to the general reaction illustrated below for esters: 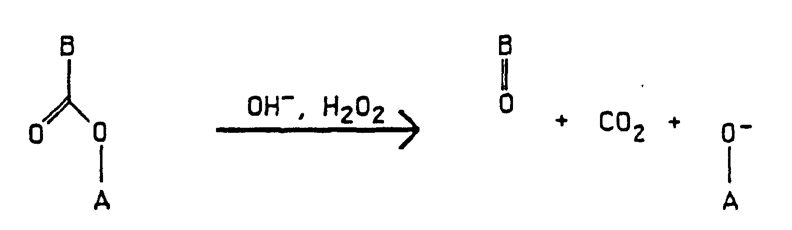 where A= an aryl ring or ring system and B= a heterocyclic ring or ring system. The compounds of this subclass tend to "lose their chemiluminescence" due to the premature hydrolysis of the ester, thioester or amide linkage. Once the linkage is hydrolyzed the compound no longer produces chemiluminescence efficiently. If the compound is being used as a label in an assay, the label will gradually lose its capacity to chemiluminesce, thus producing unreliable assay results.

It is therefore desirable to provide chemiluminescent ester, thioester and amide compounds for use in assays which are not as susceptible to hydrolysis and thus show increased stability in aqueous solution.

An article by Weeks *et al* [*Clin. Chem.,* 29/8, 1474-1479, (1983)] discusses the synthesis of a chemiluminescent acridinium ester derivative that is said to be capable of covalent association, under mild conditions, with antibodies to yield stable immuno reactive derivatives of high specific chemiluminescence activity.

EP-A-0263657 discloses various polysubstituted aryl acridinium esters. These are said to provide highly stable labels for use in a chemiluminescent assay.

EP-A-0309230 discloses diagnostic assay methods and labels for detecting a medium when the analyte is a member of a specific binding pair. In a particular system chemiluminescent acridinium ester labelled probes are used in a hybridisation assay for detecting target polynucleotide sequences.

EP-A-0257541 discloses certain chemiluminescent acridinium derivatives and their uses in luminescent immunoenzyme assays.

EP-A-0273115 discloses various acridinium sulfonylamides and isomers thereof. Methods of synthesis are also disclosed. The N-sulfonyl-9-acridinium carboxamide and isomer are said to be useful in chemiluminescent assays, when conjugated to antigens, haptens antibodies or nucleic acids.

### SUMMARY OF THE INVENTION

In accordance with the present invention, specific binding assays are disclosed which utilise a chemiluminescent compound, i.e., moiety, which has increased stability in aqueous solution. The chemiluminescent moiety is an ester, thioester or amide in which the ester, thioester or amide linkage is between (1) a heterocyclic ring or ring system containing a carbon atom to which the linkage is attached, wherein the heteroatom within the heterocyclic ring or ring system is in an oxidation state which renders such carbon atom susceptible to attack by peroxide or molecular oxygen to form an intermediate which decays to produce chemiluminescence, and (2) and aryl ring or ring system. The aryl ring or ring system contains at least one substituted six-member ring. The substituted six-member ring has two or more substituent groups, where at least two of said two or more substituent groups sterically hinder hydrolysis of said linkage. One or more of the substituent groups which sterically hinder hydrolysis of said linkage may be an electron withdrawing group. The substituted six-member ring may have one or more additional substituent groups in addition to the substituent groups which sterically hinder hydrolysis of the linkages. Such additional substituent groups may also be an electron withdrawing group.

The carbon atom in the heterocyclic ring or ring system, to which the linkage is attached, may also have a secondary substituent of the formula RₙX-, where X is selected from the group consisting of O, N, S and C, where R is any group, and where n is a number such that X has proper valency. Other chemiluminescent moieties are disclosed which are characterized by a heterocyclic ring or ring system and a secondary substituent of the formula RnX-, with the ester, thioester or amide linkage being between the heterocyclic ring or ring system and a leaving group. The disclosed chemiluminescent moieties can also include substituents at peri positions within the heterocyclic ring or ring system.

The chemiluminescent compounds provided by the present invention are disclosed in claims 1 to 38. They may be bound to specific binding partners to provide conjugates as set out in claims 39 and 40.

Also in accordance with the present invention, are compositions and kits and assay kits comprising the compounds/conjugates referred to above, as set out in claims 41 and 42. Specific binding assays are also included. These are set out in claims 43 to 69.

### DESCRIPTION OF THE PREFERRED AND ALTERNATIVE EMBODIMENTS

The chemiluminescent moieties of the present invention have either of the two following schematic formulae: 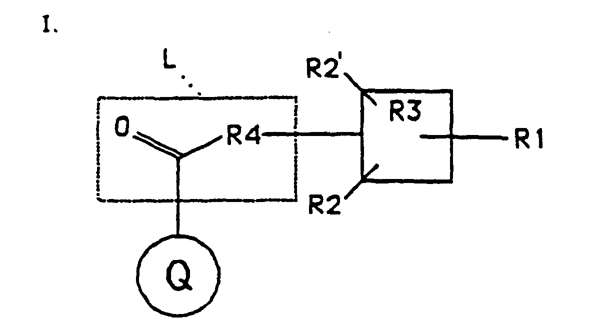 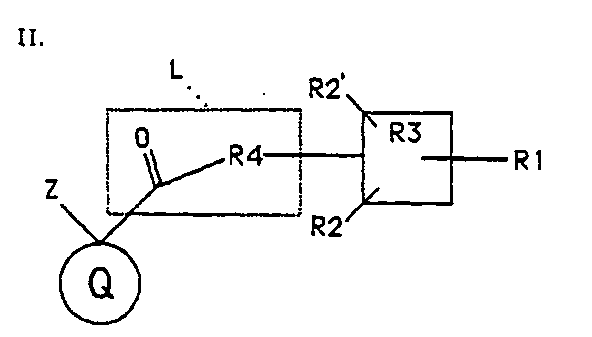 Within the schematic formulae, the dashed box labelled "L" contains the ester, thioester or amide "linkage" which is between two substituted rings or ring systems represented by the circle labelled "Q" and the solid box labelled "R3". Whether the linkage L is an ester, thioester or amide is determined by R4 being -O-, -S- or -N(SO₂CF₃)-, respectively. Preferably, the linkage is an ester linkage.

Q is a heterocyclic ring or ring system to which the ester, thioester or amide linkage L is attached at a carbon atom within the heterocyclic ring or ring system which (1) is either sp² or sp³ hybridized, and (2) is susceptible to attack by peroxide or molecular oxygen to form an intermediate that decays to produce chemiluminescence. Whether the carbon atom is rendered susceptible to such attack is determined by the oxidation state of the heteroatom within the heterocyclic ring or ring system. If the carbon to which the linkage is attached is sp² hybridized, the heteroatom must be in a positive oxidation state (i.e., have a positive charge; for example, as obtained by N-alkylation or N-oxidation). If the carbon to which the linkage is attached is sp³ hybridized, the heteroatom must be in a neutral oxidation state (i. e., uncharged). When the heteroatom is nitrogen, proper oxidation states can only be achieved if the nitrogen is substituted with an alkyl group (including a functionalized group), an aryl group including a functionalized group),-O- (if the nitrogen is in a positive oxidation state) or -OH (if the nitrogen is in a neutral oxidation state). When the heteroatom is in these "proper" oxidation states, the carbon atom will be susceptible to attack by peroxide or molecular oxygen to produce the chemiluminescent intermediate.

Heterocyclic rings or ring systems in which the heteroatom is in a positive oxidation state include without limitation acridinium, benz[a]acridinium, benz[b]acridinium, benz[c]acridinium, a 1,2,4-triazole cation, an isooxazole cation, an isothioazole cation, a 1,2-azole cation, an imidazole cation, a benzimidazole cation, quinolinium, isoquinolinium, quinolizinium, a cyclic substituted quinolinium, pyridinium, pyrimidininium, pyridazinium, pyrazininium, phenanthridinium, and quinoxalinium. Rings or ring systems in which the heteroatom is in a neutral oxidation state include the reduced forms of the foregoing. These rings or ring systems are derived from the following rings or ring systems:

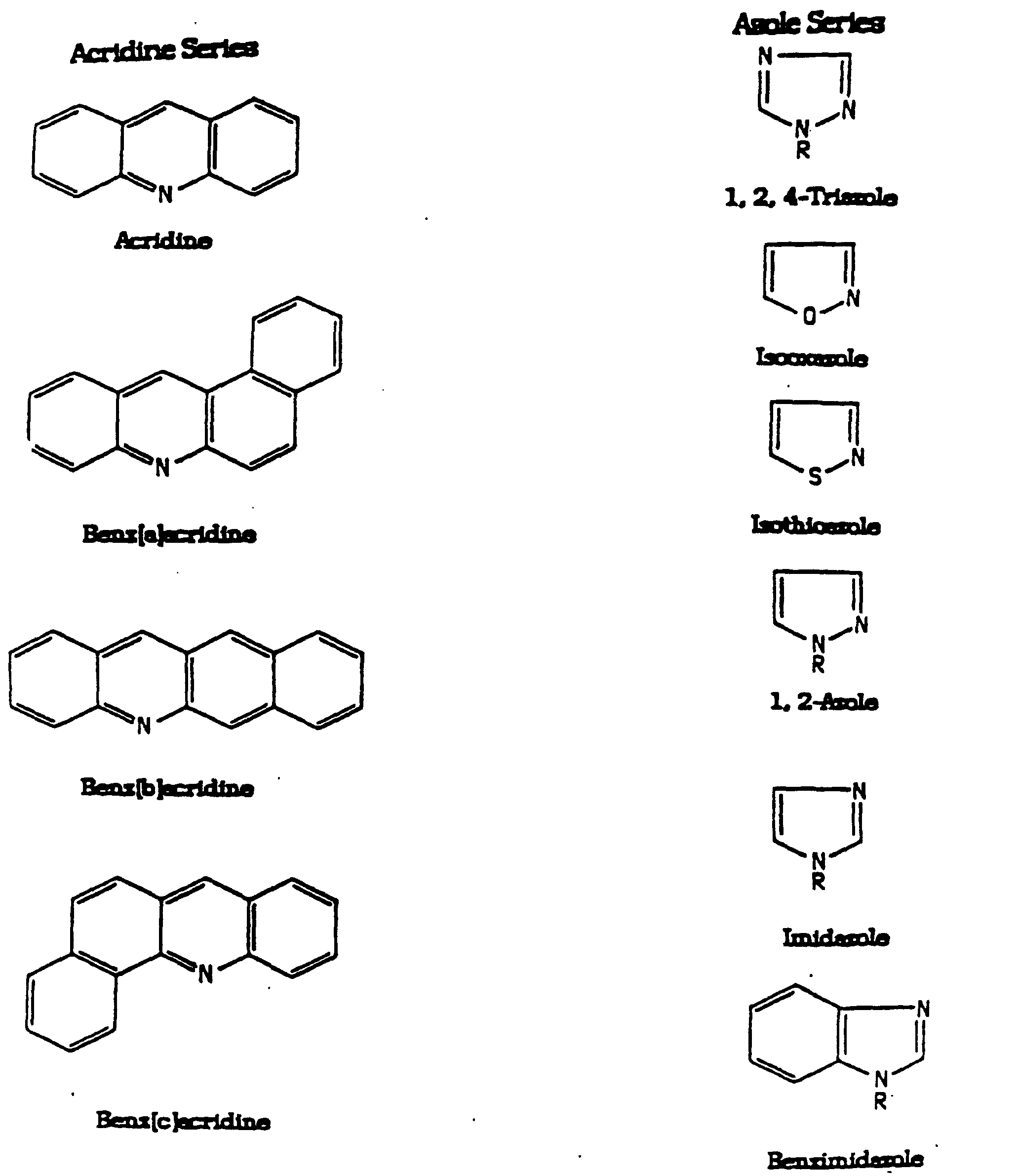

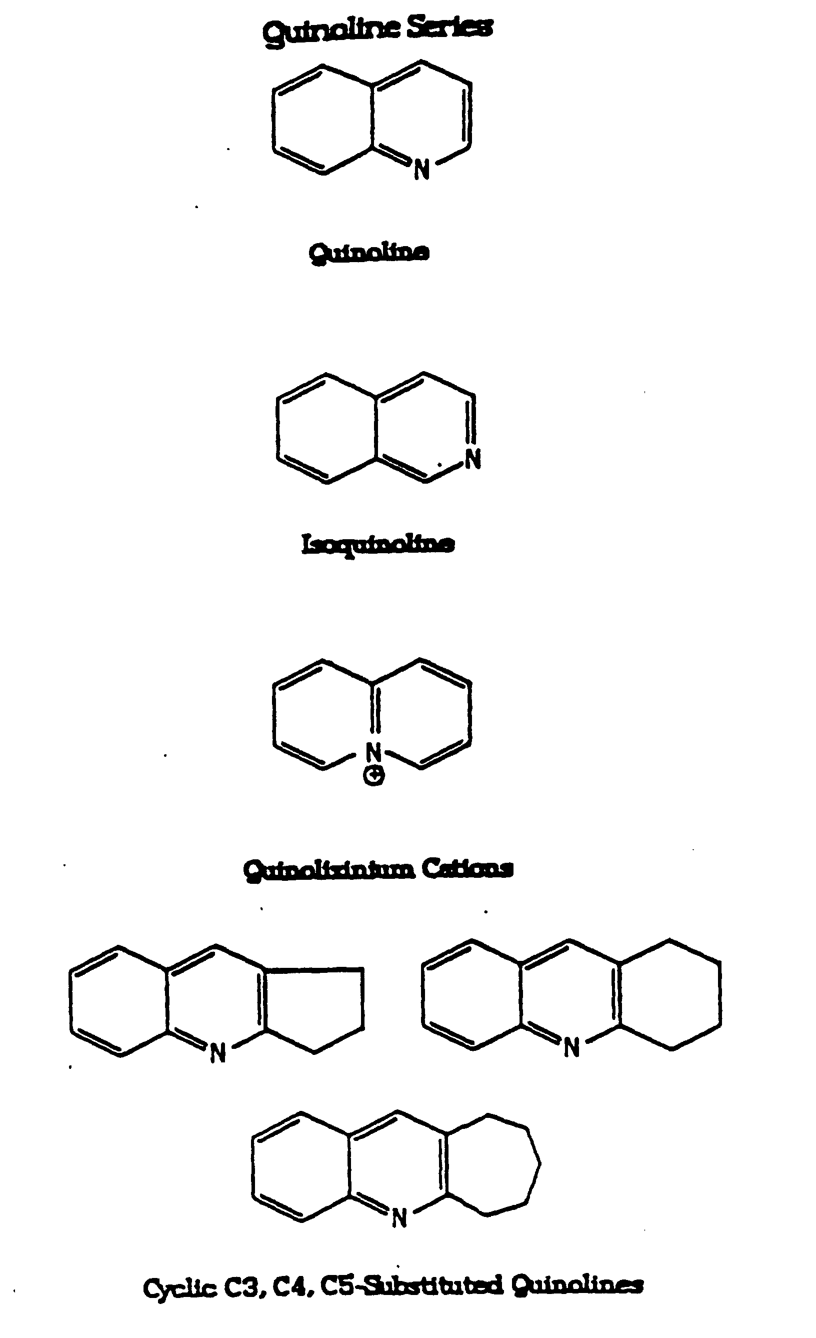

The heterocyclic ring or ring system may be free of optional substitutions not shown in the schematic formulae. Alternatively, the heterocyclic ring or ring system may be optionally substituted at any position, including the heteroatom, with groups not shown in the schematic formula. Such rings and ring systems, whether optionally substituted or free of such optional substitutions, are considered herein to be within the meaning of "heterocyclic ring or ring system."

Possible optional substitutions may be functional or non-functional. Functional optional substitutions include those for the purposes of producing peri-interactions around the linkage L, increasing the solubility of the moiety, increasing the chemiluminescent efficiency of the moiety and, preferably, attaching the moiety to protein and other material. Groups useful for attaching the moiety to protein and other material include but are not limited to optionally functionalized alkyl, alkenyl, alkynyl, alkylamino, oxoalkyl, thioalkyl or alkyloxocarbonyl chains (branched or unbranched) or optionally functionalized aryl groups. The aryl groups can be joined to the heterocyclic ring or ring system by an alkyl, alkenyl, alkynyl, alkylamino, oxoalkyl, thioalkyl or alkyloxocarbonyl chain. Other chains, in addition to those listed, are well known in the art. Optional functionalities include without limitation any of the following:
- CO₂R, where R= a hydrogen, alkyl or aryl 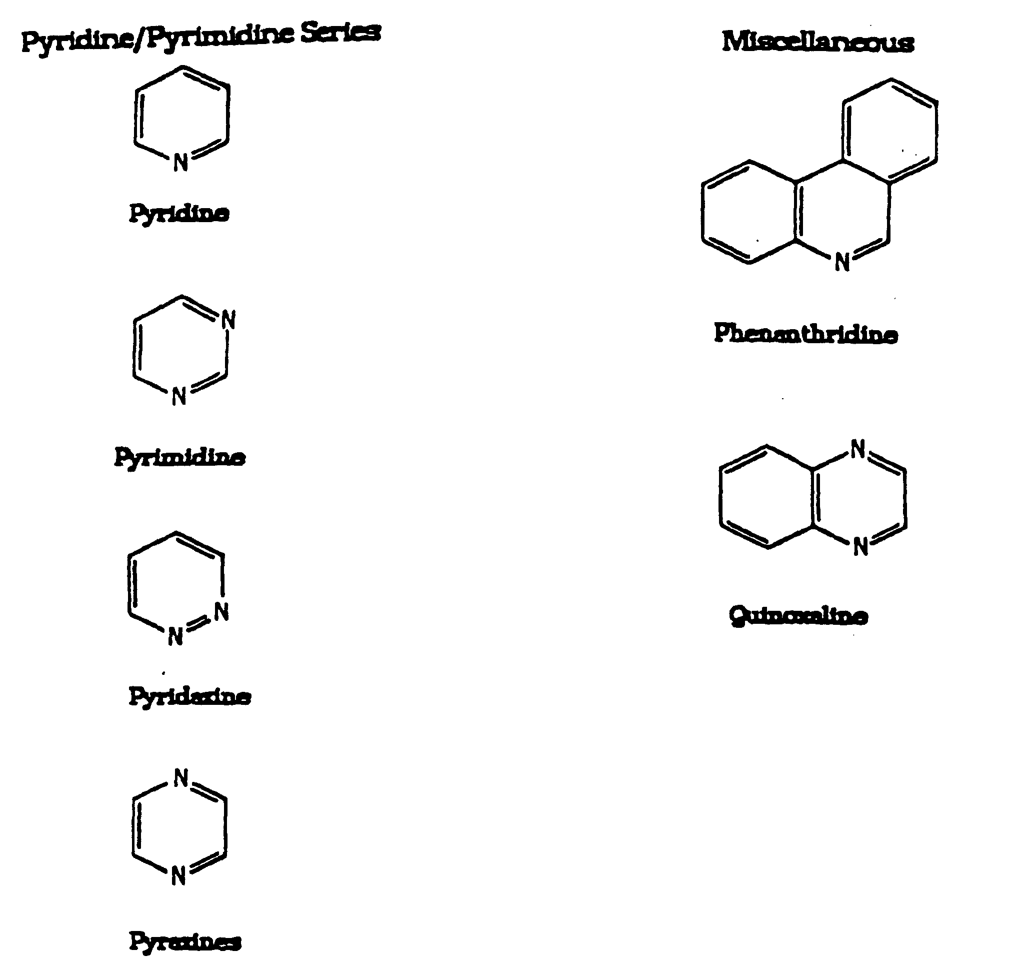 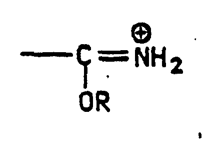
where R= a residue of a functional alcohol
- SO₂Cl
- NCS 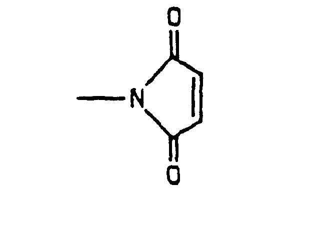 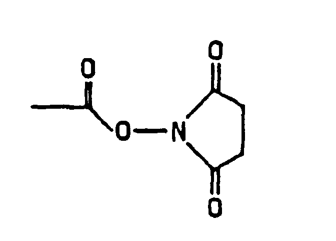 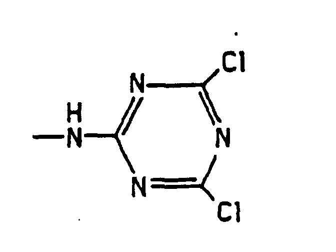 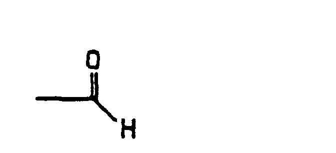
- N(CH₃)(CH₂)ₘCl, where m is greater than or equal to 1
- N₃ and other photolabile functionalities
- NH₂.
or ions, sugars, polyamines and polyoxy-compounds (e.g., polyoxyethylene, poloxybutylene, etc.). Other chains, groups and functionalities useful for attaching compounds of the present invention to protein are discussed in Ji, "Bi-functional Reagents," Meth. Enzymology 91:580 (1983), which is incorporated herein by reference. Methods if joining such attaching groups to protein and other materials utilize both covalent bonding and weak chemical forces and are well known in the art.

Peri substituents, which can cause peri-interactions, include any group which can cause steric hindrance with respect to the carbon to which the ester, thioester or amide linkage is attached and/or with respect to the carbon within the ester, thioester or amide linkage. Preferred peri substituents include short alkyl groups (e.g., methyl, ethyl) and aryl groups (e.g., phenyl). The peri substituents, if present, are located on carbon atoms within the heterocyclic ring or ring system which are "adjacent to" the carbon to which the ester, thioester or amide linkage is attached. Moieties can include more than one peri substituent. For example, peri substituents can be placed in the following positions:
(a) in acridiniums and acridans: on C1 and C8;
(b) in phenanthridiniums and reduced phenanthridiniums: on C7; and
(c) in quinoliniums and reduced quinoliniums: on C3.

The aryl ring or ring system, represented by R3, includes at least one substituted six-member ring. The ester, amide or thioester linkage is directly attached to such six-member ring. R3 may include but is not limited to phenyl, naphthalene and anthracene, which have the following structures: 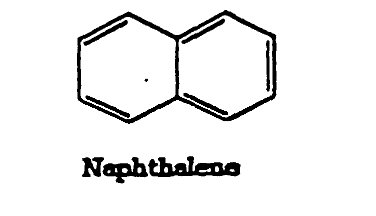 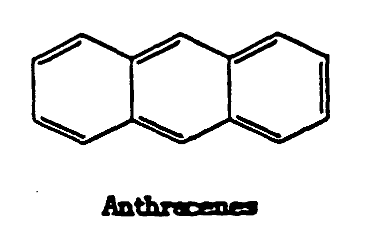 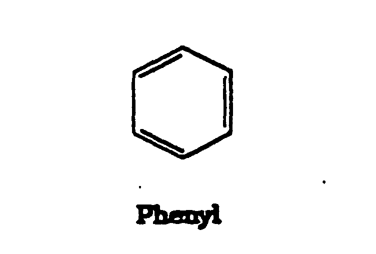 R3 may be substituted at any position including but not limited to the substitutions for R1, R2 and R2' described below A substitution, including R1, R2 and R2', can also be used to attach the moiety to protein or other material. Appropriate groups for such purpose are discussed above.

R2 and R2' are bulky groups which are located on R3 so as to sterically hinder the hydrolysis of the linkage L between R3 and the heterocyclic ring or ring system Q. Preferably, where R3 is a phenyl ring with the ester linkage being attached at position 1, R2 and R2' are located at the 2 and 6 (ortho) positions. R2 and R2' may be identical or differ from one another, and either may include:
an alkyl or optionally functionalized alkyl group
an aryl or optionally functionalized aryl group
   - OR, where R= alkyl or aryl
   - NR₂, where R= alkyl or aryl
   - NR₃⁺, where R= alkyl or aryl
   - COO⁻
   - COOH
   - COOR, where R= alkyl or aryl
   - COR, where R= alkyl or aryl
   - (CF₂)ₙCF₃, where n=0 to 10
   - CN
   - NH₃⁺
   - NO₂
   - N(CH₃)₃⁺
   - SR, where R= alkyl or aryl
   - SR₂⁺, where R= alkyl or aryl
   - SO₂R, where R= alkyl or aryl
a halogen.
The required steric hinderance can also be provided by other rings within a multi-ring R3 which are "adjacent" to the six-member ring to which the ester linkage is attached. For example, if R3= naphthalene and an ester linkage is attached at the 1 position, R2 could be a methyl group at the 2 position and R2' is the "adjacent" ring containing carbons 7-10 In such cases, the adjacent ring is considered herein to be substitution (on the six-member ring within R3) which sterically hinders the hydrolysis of the linkage.

R1 may be any electron withdrawing group on R3, but is preferably selected from -NO₂, -SO₂Cl, -Br, -N(CH₃)₃ and -H. As used throughout this specification and the appended claims, an "electron withdrawing group" shall meal any group which has a sigmaₚ value greater than or equal to 0.0. Tables of sigmaₚ values for various groups can be found in Hansch et al, J. Med. Chem. 16(11):1209-1213 (1977) and Hansch and Leo, "Substituent Constants for Cor relation Analysis in Chemistry and Biology," Ch. 6, pp. 49-52 (John Wiley & Sons, New York 1979), both of which are incorporated herein by reference.

In some moieties, R1 and one or both of R2 and R2' are electron withdrawing groups (which may be identica or differ from one another). If more than one group among R1, R2 and R2' are electron withdrawing groups, it is preferred that the additive sigmaₚ value for only the groups which are electron withdrawing groups (i.e., excluding those groups with sigmaₚ < 0.0) be less than or equal to about 1.0. Some moieties having an additive sigmaₚ value greater than 1.0 are unstable.

The moieties of the present invention may also be attached to protein and other material through substitutions on R3. Some electron withdrawing groups can be used as a means of attachment. Of the preferred electron withdrawing groups, -SO₂Cl can be used to attach a moiety to protein or other material. Alternatively, any of the groups listed above for attachment to the heterocyclic ring or ring system Q can also be used if they can be attached as substitutions to R3. Methods of joining such attaching groups to protein and other materials utilize both covalent bonding and weak chemical forces and are well known in the art.

In schematic formula II above, Z is a secondary substituent group attached to the carbon atom to which the ester, thioester or amide linkage is attached when such carbon atom is sp³ hybridized. Z may include but is not limited to
- H
   a halogen
- CN
- OR
- NR₂
- NR₃⁺
- SR
- SR₂⁺
- S₂R
- NRCO₂R
- NHNR₂
- NRNR₂
- NHOR
- ONR₂
- CR(CN)₂
- CR(COR)₂
- CR₂NO₂
- C≡COR.
Generally, in addition to a hydrogen and a halogen, Z can be any nucleophilic group of the general formula RₙX, where X= O, N, S or C (with appropriate changes in n to accommodate valency changes in X) and where R= any substituent (preferably, alkyl, aryl, an amino acid or a sugar, optionally substituted) and where multiple R groups can optionally form cyclic structures (e.g., in the Z groups: 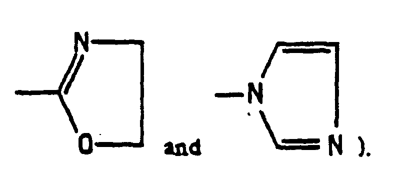 Rₙ and RₙX can also be ( or be derived from) a drug or steroid molecule.

In compounds in which Z= RₙX, Z serves as a "blocking" moiety which prevents or deters hydrolysis of the ester, thioester or amide linkage, thus decreasing the likelihood that such compounds will be unstable. The blocking effect is caused by (1) the steric bulk of the RₙX group which physically blocks attack by chemical species which would induce or increase hydrolysis of the ester, thioester or amide linkage (e.g., species which would form a pseudo base at carbon 9 of an acridinium compound), and (2) electronic effects produced by the change of the carbon atom from sp² to sp³ hybridization (which cause the ester, thioester or amide linkage to behave more like an aliphatic ester, thioester or amide). When compounds including an RₙX group are triggered to produce chemiluminescence they must first be treated with acid which cleaves the RₙX group, thus allowing induction of the chemiluminescent reaction upon the addition of peroxide, base or other triggering agent (acid treatment is not required for compounds where Z= -H or a halogen). The character and structure of the RₙX group is limited by only two factors: (1) the size of the RₙX group must be such that the group can be added to compounds of the present invention during synthesis as described below or by other synthetic procedures (i.e., the RₙXH species, from which the RₙX group derives during synthesis, cannot be so large that steric effects make it impossible to synthesize a compound of the present invention having such RₙX group), and (2) the RₙX group must be of a steric and chemical character such that it can be cleaved from the molecule with acid before triggering the chemiluminescent reaction.

The above-described improved chemiluminescent moieties are useful in a broad range of specific binding assays for the presence of analyte in a sample. "Presence" shall mean herein the qualitative and/or quantitative detection of an analyte. Such assays may be directed at any analyte which may be detected by use of the improved chemiluminescent moiety in conjunction with specific binding reactions. These assays include, without limitation, immunoassays, protein binding assays and nucleic acid hybridization assays.

In a typical immunoassay, the analyte is immunoreactive and its presence in a sample may be determined by virtue of its immunoreaction with an assay reagent. In a typical protein binding assay, the presence of analyte in a sample is determined by the specific binding reactivity of the analyte with an assay reagent where the reactivity is other than immunoreactivity. Examples of this include enzyme-substrate recognition and the binding affinity of avidin for biotin. In the typical nucleic acid hybridization assay, the presence of analyte in a sample is determined by a hybridization reaction of the analyte with an assay reagent. Analyte nucleic acid (usually present as double stranded DNA or RNA) is usually first converted to a single stranded form and immobilized onto a carrier (e.g., nitrocellulose paper). The analyte nucleic acid may alternatively be electrophoresed into a gel matrix. The immobilized analyte may then be hybridized (i.e., specifically bound) by a complementary sequence of nucleic acid.

The foregoing specific binding assays may be performed in a wide variety of assay formats. These assay formats fall within two broad categories. In the first category, the assay utilizes a chemiluminescent conjugate which comprises the improved chemiluminescent moiety attached to a specific binding material. "Specific binding material" means herein any material which will bind specifically by an immunoreaction, protein binding reaction, nucleic acid hybridization reaction, and any other reaction in which the material reacts specifically with a restricted class of biological, biochemical or chemical species. In this category of assays, the chemiluminescent conjugate participates in a specific binding reaction and the presence of analyte in the sample is proportional to the formation of one or more specific binding reaction products containing the chemiluminescent conjugate. The assay is performed by allowing the requisite specific binding reactions to occur under suitable reaction conditions. The formation of specific binding reaction products containing the chemiluminescent conjugate is determined by measuring the chemiluminescence of such products containing the chemiluminescent conjugate or by measuring the chemiluminescence of unreacted or partially reacted chemiluminescent conjugate not contained in such products.

This first category of assay formats is illustrated by sandwich assays, competitive assays, surface antigen assays, sequential saturation assays, competitive displacement assays and quenching assays.

In a sandwich format, the specific binding material to which the chemiluminescent moiety is attached, is capable of specifically binding with the analyte. The assay further utilizes a reactant which is capable of specifically binding with the analyte to form a reactant-analyte-chemiluminescent conjugate complex. The reactant may be attached to a solid phase, including without limitation, dip sticks, beads, tubes, paper or polymer sheets. In such cases, the presence of analyte in a sample will be proportional to the chemiluminescence of the solid phase after the specific binding reactions are completed. Such assay formats are discussed further in U.S Patent Nos. 4,652,533, 4,383,031, 4, 380, 580 and 4,226,993.

In a competitive format, the assay utilizes a reactant which is capable of specifically binding with the analyte to form an analyte-reactant complex and with the specific binding material, to which the chemiluminescent moiety is attached, to form a chemiluminescent conjugate-reactant complex. The reactant may be attached to a solid phase, or alternatively reaction products containing the reactant may be precipitated by use of a second antibody or by other known means. In this competitive format, the presence of analyte is "proportional," i.e., inversely proportional, to the chemiluminescence of the solid phase or precipitate. A further discussion of this assay format may be found in the immediately above mentioned U.S. patents.

In another assay format, the analyte may occur on or be bound to a larger biological, biochemical or chemical species. This type of format is illustrated by a surface antigen assay. In this format, the specific binding material is capable of specifically binding with the analyte and the presence of analyte is proportional to the analyte-chemiluminescent conjugate complex formed as a reaction product. This is illustrated by attaching the chemiluminescent moiety to an antibody which is specific to a surface antigen on a cell. The presence of the cell surface antigen will be indicated by the chemiluminescence of the cells after the completion of the reaction. The cells themselves may be used in conjunction with a filtration system to separate the analyte-chemiluminescent conjugate complex which is formed on the surface of the cell from unreacted chemiluminescent conjugate. This is discussed further in U.S. Patent No. 4,652,533.

The improved chemiluminescent moiety may be used in additional assay formats known in the art including without limitation sequential saturation and competitive displacement, both of which utilize a chemiluminescent conjugate where both (1) the specific binding material, to which the moiety is attached, and (2) the analyte specifically bind with a reactant. In the case of sequential saturation, the analyte is reacted with the reactant first, followed by a reaction of the chemiluminescent conjugate with remaining unreacted reactant. In the case of competitive displacement, the chemiluminescent conjugate competitively displaces analyte which has already bound to the reactant.

In a quenching format, the assay utilizes a reactant which is capable of specifically binding with the analyte to form an analyte-reactant complex and with the specific binding material, to whic the chemiluminescent moiety is attached, to form a chemiluminescent conjugate-reactant complex. A quenching moiety is attached to the reactant. When brought into close proximity to the chemiluminescent moiety, the quenching moiety reduces or quenches the chemiluminescence of the chemiluminescent moiety. In this quenching format, the presence of analyte is proportional to the chemiluminescence of the chemiluminescent moiety. A further discussion of this format may be found in U.S. Patent Nos. 4,220,450 and 4,277,437.

In consideration of the above discussed assay formats, and in the formats to be discussed below, the order in which assay reagents are added and reacted may vary widely as is well known in the art. For example, in a sandwich assay, the reactant bound to a solid phase may be reacted with an analyte contained in a sample and after this reaction the solid phase containing complexed analyte may be separated from the remaining sample. After this separation step, the chemiluminescent conjugate may be reacted with the complex on the solid phase. Alternatively, the solid phase, sample and chemiluminescent conjugate may be added together simultaneously and reacted prior to separation. As a still further but less preferred alternative, the analyte in the sample and the chemiluminescent conjugate may be reacted prior to addition of the reactant on the solid phase. Similar variations in the mixing and reaction steps are possible for competitive assay formats as well as other formats known in the art. "Allowing under suitable conditions substantial formation" of specific binding reaction products shall herein include the many different variations on the order of addition and reaction of assay reagents.

In the second category of assay formats, the assay utilizes an unconjugated improved chemiluminescent moiety. The presence of analyte in the sample is proportional to the formation of one or more specific binding reaction products which do not themselves contain the chemiluminescent moiety. Instead, the chemiluminescent moiety chemiluminesces in proportion to the formation of such reaction products.

In one example of this second category of assays, the assay utilizes a reactant capable of binding with the analyte to form an analyte-reactant complex which causes the chemiluminescent moiety to chemiluminesce. This is illustrated by a simple enzyme-substrate assay in which the analyte is the substrate glucose and the reactant is the enzyme glucose oxidase. Formation of the enzyme-substrate complex triggers the chemiluminescent moiety. Such enzyme-substrate assay for glucose is disclosed in U.S. Patent Nos. 3,964,870 and 4,427,770. This enzyme-substrate assay is a specific binding assay in the sense that the substrate specifically binds to the active site of the enzyme in much the same way that an antigen binds to an antibody. In this assay, the enzyme specifically binds with the substrate which results in the production of peroxide which, in turn, causes the chemiluminescent moiety to chemiluminesce.

Also included in the second category of assays are those assays in which the formation of the reaction products promotes or inhibits chemiluminescence by the chemiluminescent moiety in a less direct manner. In this assay, a first reactant, which is cross reactive with the analyte, is attached to an enzyme such as glucose oxidase close to its active site. A second reactant which is specific for both the analyte and the immunoreactive material is added to the sample and the altered enzyme in the presence of the substrate (i.e., glucose). When the second reactant binds to the first reactant located near the active site on the enzyme, the second reactant blocks the active site in a way that the substrate cannot bind to the enzyme at the active site or the binding of the substrate at the active site is significantly decreased. The second reactant blocking the enzyme in this manner inhibits the enzyme from producing peroxide which, in turn, would have triggered the chemiluminescent moiety. Analyte in the sample, however, will tie up the second reactant, thus preventing the second reactant from inhibiting the production of peroxide. The presence of analyte will be proportional to the chemiluminescence of the moiety.

The assays contained in the above two categories of assay formats may be heterogeneous or homogeneous. In heterogeneous assays, the reaction products, whose formation is proportional to the presence of analyte in the sample, are separated from other products of the reaction. Separation can be achieved by any means, including without limitation, separation of a liquid phase from a solid phase by filtration, microfiltration, double antibody precipitation, centrifugation, size exclusion chromatography, removal of a solid phase (e.g., a dip stick) from a sample solution or electrophoresis. For example, in a sandwich assay the reactant-analyte-chemiluminescent conjugate complex is separated from unreacted chemiluminescent conjugate. In a surface antigen assay, the analyte-chemiluminescent conjugate complex is separated form unreacted chemiluminescent conjugate. In a competitive assay, the reactant-chemiluminescent conjugate complex is separated from unreacted chemiluminescent conjugate. In a sequential saturation assay and in a competitive displacement assay, the reactant-chemiluminescent conjugate complex is separated from unreacted chemiluminescent conjugate. Alternatively, in homogeneous assays the reaction products are not separated. After the assay reagents have been allowed to react, the chemiluminescence may be measured from the whole assay mixture whether such mixture is in solution , on a solid phase or distributed between various membrane layers of a dip stick or other solid support. The glucose assay using glucose oxidase and a chemiluminescent moiety illustrates a simple homogeneous assay in which separation is unnecessary. The quenching assay illustrates a more complex homogeneous assay in which separation is unnecessary. It is contemplated that either category of assay formats may give rise to either heterogeneous or homogeneous formats.

Finally, "measuring the chemiluminescence" shall include, where relevant, the act of separating those specific binding reaction products, the formation of which are proportional to the presence of analyte in the sample, from other reaction products. It shall also include, where relevant, the acts of (i) treating the chemiluminescent moiety with acid to cleave a Z (i.e., RₙX) group from the moiety, and/or (ii) triggering the chemiluminescent moiety to chemiluminesce in the case of those assay formats in which the formation of the reaction products does not itself trigger the chemiluminescent moiety.

### SYNTHESIS OF MOIETIES

The following examples show the synthesis of certain chemiluminescent moieties of the present invention.

### Example 1

A preferred chemiluminescnet moiety of the present invention is (2,6-dimethyl-4-nitro)phenyl-3-(3-succinimidyl-oxycarbonyl)propyloxy-N-methyl-acridinium-9-carboxylate fluorosulfonate (or other counter ions such as chloride, trifluoroacetate, sulfate, etc.) which has the following formula: 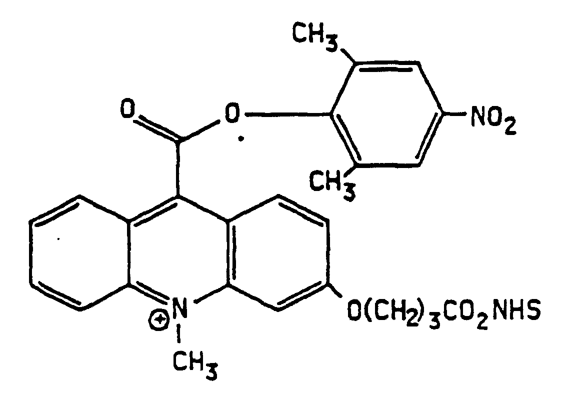 (2,6-dimethyl-4-nitro) phenyl-3-(3-succinimidyl-oxycarbonyl)propyloxy-N-methyl-acridinium-9-carboxylate fluorosulfonate is synthesized according to the following scheme: 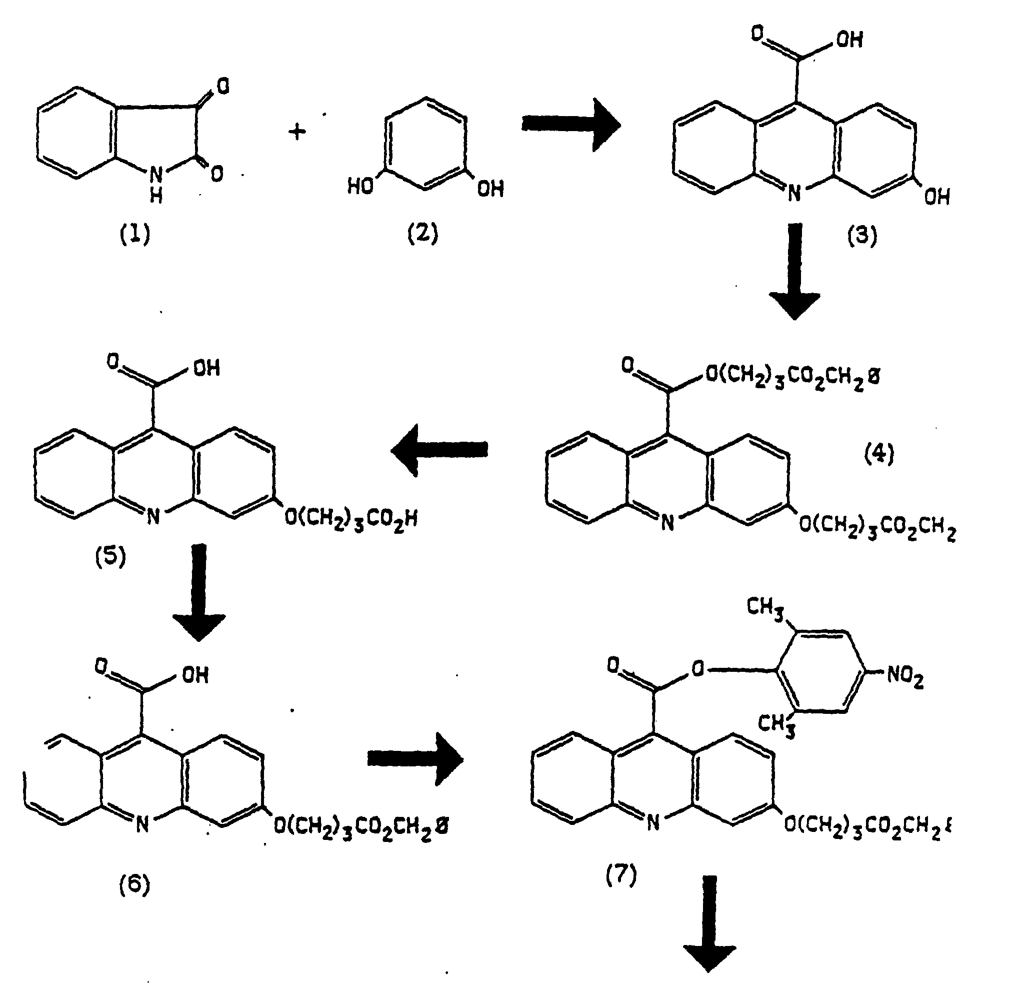 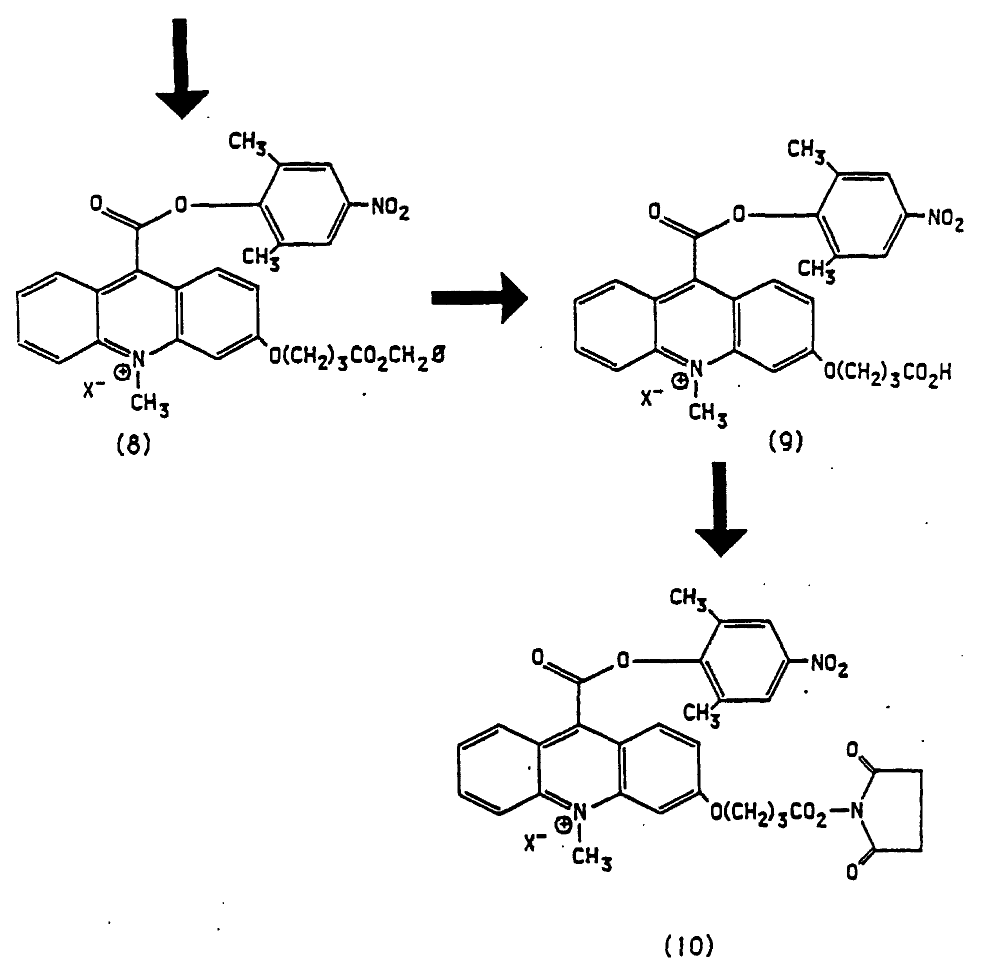 Condensation of isatin (1) with resorcinol (2) provides the 3-hydroxy acridine-9-carboxylic acid (3). Reaction with benzyl-4-bromo butyrate gives the ester (4) with the 3-hydroxy group etherified. Hydrolysis using base removes both the benzyl groups resulting in the dicarboxylic acid (5). Selective rebenzylation of the carboxylic function of the propyloxy group gives 9-carboxylic acid (6). Esterification with 2,6-dimethyl-4-nitrophenol and methylation of the acridine nitrogen gives (8). Deprotection of the carboxyl group with HBr and condensation with N-hydroxysuccinimide using DCC provides (2,6-dimethyl-4-nitro) phenyl-3-(3-succinimidyi-oxycarbonyl) propyloxy-N-methyl-acridinium-9-carboxylate fluorosulfonate. These reactions are described in further detail in the following.

4-bromobutyryl chloride (13.8 g, 75 mmole) was placed in a 100 ml round bottom flask. The flask was cooled to -20° C using a dry ice/ carbon tetrachloride bath. Ethyl acetate (50 ml) containing N-methylmorpholine (7.58 g, 75 mmole, 8.2 ml) was added carefully. Using an addition funnel, benzyl alcohol (6.97 g, 6.67 ml, 6.64 mmoles) was added dropwise. After the addition the bath was removed and the mixture was stirred for 2 hours. The product was transferred to a separatory funnel using ethyl acetate (50 ml), washed once with sodium bicarbonate (10%), then twice with water, and dried with anhydrous sodium sulfate. Evaporation of solvents gave benzyl-4-bromo-butyrate as an oil (yield = 91 %).

Isatin (1) (1.88 g) was slowly added to a solution of potassium hydroxide (5.07 g, 0.09 mole) dissolved in water (3.5 ml). The reaction flask was heated to about 50°C in an oil bath. About 10 ml more water was added dropwise. Resorcinal (2) (10 g, 0.089 mole) was added and the temperature was raised to 100° C as stirring was continued, resulting in the formation of a molten mixture. More isatin (1) (1.88 g) was added. The reaction flask (3-necked round bottom) was attached to a nitrogen inlet and the water vapors were flushed out by the stream of nitrogen. The mixture was stirred for 4 hours at 125° C. Water (70 ml) was added and the contents were dissolved by continued stirring. After transferring the mixture to an erlenmeyer flask the volume was brought up to 200 ml with water. The pH was adjusted to 2.0 with concentrated hydrochloric acid. Filtration and washing of the solids with water gave the crude acridine acid. It was then dissolved in 2N NaOH (100 ml) and the solution was filtered through celite. The celite bed was washed with 200 ml of 1 N NaOH. The filtrate was acidified with concentrated HCl to pH 2.0. The precipitate of 2-hydroxy-acridine-9-carboxylic acid (3) was filtered, washed with water and was dried in vacuum over P₂O₅ (yield = 42%).

3-hydroxy-9-acridine carboxylic acid (3) (4 g, 0.017 mole), benzyl-4-bromobutyrate (14.6 g, 0.057 mole) and cesium carbonate (22.16 g, 0.068 mole) were dissolved in DMSO (125 ml) in a 250 ml round bottom flask. The flask was warmed to about 50° C in an oil bath. After stirring the mixture at that temperature for 1 hour, the mixture was poured into water (1 liter). The precipitated product was extracted with chloroform after making the aqueous suspension basic with sodium bicarbonate. Drying and evaporation of chloroform gave 3-(3-benzyloxycarbonyl)-propyloxy-9-(3-benzyloxy-carbonyl-propyl) acridine carboxylate (4) which was chromatographed on a silica gel column using chloroform as solvent. Fractions with R_{f} value of 0.36 on TLC with CHCl₃/EtOAc, 9/1, were pooled. The solvents were evaporated (yield = 55%).

3-(3-benzyloxycarbonyl)-propyloxy-9-(3-benzyloxycarbonyl propyl) acridine carboxylate (4) (4.93 g, 8.3 mmole) was added to a mixture of 2N NaOH (300 ml) and methanol (300 ml). The mixture was stirred at room temperature for 48 hours. The methanol was removed on a rotary evaporator and the solution was acidified with concentrated hydrochloric acid to pH 6.0. The precipitated solids were filtered, washed with water and dissolved in ethyl acetate. The solution was dried and then the solvents were evaporated to give 3-(3-carboxy) propyloxy-acridine-9-carboxylic acid (5) (yield = 92.8%).

3-(3-carboxy)propyloxy-acridine-9-carboxylic acid (5) (1.5 g, 4.6 mmole) was dissolved in DMAP (80 ml, 1,3-dimethyl-3,4,5,6-tetrahydro-2(1H) pyrimidone) with warming. Benzyl alcohol (0.5 ml, 0.52 g, 4.8 mmole), 1,3-dicyclohexylcarbodiimide (1.04 g, 5.0 mmole) and N,N-dimethyl aminopyridine (0.2 g, 1.6 mmole) were added to the reaction which was previously cooled in a bath of dry ice/CCl₄. The mixture was stirred for 15 hours with a nitrogen inlet at room temperature. The mixture was added to saturated sodium chloride (320 ml). 3-(3-benzyloxycarbonyl) propyloxy-acridine-9-carboxylic acid (6) was filtered and was washed with a small amount of water. The product was chromatographed on a silica gel column using CHCl₃/MeOH, 95/5 as solvent (yield = 26%).

3-(3-benzyloxycarbonyl) propyloxy-acridine-9-carboxylic acid (6) (0.5 g, 1.2 mmole) and p-toluene sulfonyl chloride (0.46 g, 2.4 mmole) were dissolved in pyridine (20 ml). The solution was cooled in a bath of dry ice/CCl₄ for 15 minutes. 2,6-dimethyl-4-nitrophenol (0.2 gm, 1.2 mmole) was added and the cooling bath was removed and the mixture was stirred for 15 hours at room temperature. It was added to water (450 ml) and the pH was adjusted to 2.0 with concentrated hydrochloric acid. The product was filtered, washed with water and was dried in vacuum. The crude product was chromatographed on a silica gel column using chloroform as solvent. Fractions with an R_{f} value of 0.8 on TLC with CHCl₃/EtOAc, 1:1, were pooled. Evaporation of solvents gave (2,6-dimethyl-4- nitro) phenyl-3-(3-benzyloxycarbonyl)-propyloxy-acridine-9-carboxylate (7) (yield = 47%).

The acridine (7) (0.32 g, 0.56 mmole) was dissolved in anhydrous methylene chloride (4 ml) and methyl fluorosulfate (0.27 ml, 3.36 mmole, 6 molar equivalent) was added. The mixture was stirred for 15 hours at room temperature. Anhydrous ether (20 ml) was added. (2,6-dimethyl-4-nitro)phenyl-3-(3-benzyloxycarbonyl) propyloxy-acridinium-9-carboxylate fluorosulfonate (8) was filtered and washed with ether (50 ml). The yield was quantitative.

The benzyl-protected acridinium ester (8) (250 ng) was treated with 30% HBr/Acetic acid (3 ml) for 2 hours at 55° C. Anhydrous ether (20 ml) was added to precipitate the product. Filtration and washing of the solids with ether gave (2,6-dimethyl-4-nitro)phenyl-3-(3-carboxyl) propyloxy-acridinium-9-carboxylate fluorosulfonate (9). Crystallization from acetonitrile provided the pure compound (yield - 80%).

The deprotected acridinium (9) (67 mg, 0.13 mmole) in a 50 ml 2-necked round bottom flask was dissolved in anhydrous acetonitrile (10 ml). Dicyclohexylcarbodiimide (DCC, 33 mg, 0.16 mmole) was added and the mixture stirred for 45 minutes at room temperature. N-hydroxysuccinimide (17 mg, 0.15 mmole) was added and reaction continued for 2.5 hours. More DCC (14 mg) and N-hydroxysuccinimide (8 mg) were added and followed again by the same amounts after 1.5 hours. After 1.5 hours after the last addition, glacial acetic acid (1.7 1) was added to quench excess DCC. The solvent was removed in vacuo.

The crude product was purified on a semi-preparative C₁₈-Dynamax HPLC column (commercially available from Rainin Instrument Co., Inc., Woburn. Massachusetts) using CH₃CN/H₂O (0.1% Trifluoroacetic acid) 60/40, as the mobile phase at a flow rate of 1.8 ml/min, using 360 nm for detection. The fraction at retention time 9.4 minutes was collected and the solvents were removed in vacuo. The (2,6-dimethyl-4-nitro)-phenyl-3-(3-succinimidyl-oxycarbonyl) propyioxy-acridinium-9-carboxylate fluorosulfonate (10) was dried under vacuum in a dessicator containing phosphorus pentoxide (yield - 33%). MS: FAB, thioglycerol matrix, 586 (M⁺). HPLC: Rainin C₁₈ Dynamax (10 mm x 25 mm), CH₃CN/H₂O (0.1 % trifluoroacetic acid), 60:40, flow rate 1.8 ml/min, retention time 9.4 min, detected at 360 nm.

### Example 2

Another preferred moiety of the present invention is (2,6-dimethoxy-3-chlorosulfonyl)phenyl-N-methyl-acridinium-9-carboxylate fluorosulfonate which has the following formula: 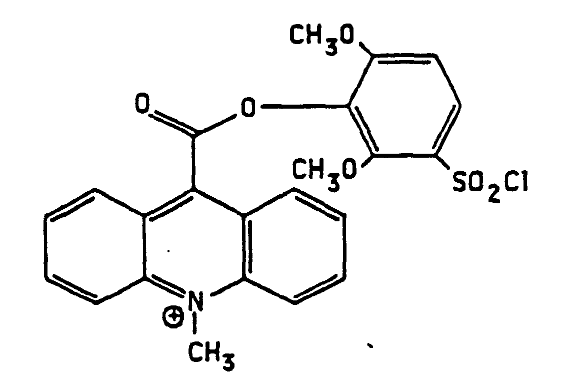 (2,6-dimethoxy-3-chlorosulfonyl)phenyl-N-methyl-acridinium-9-carboxylate fluorosulfonate is synthesized according to the following scheme: 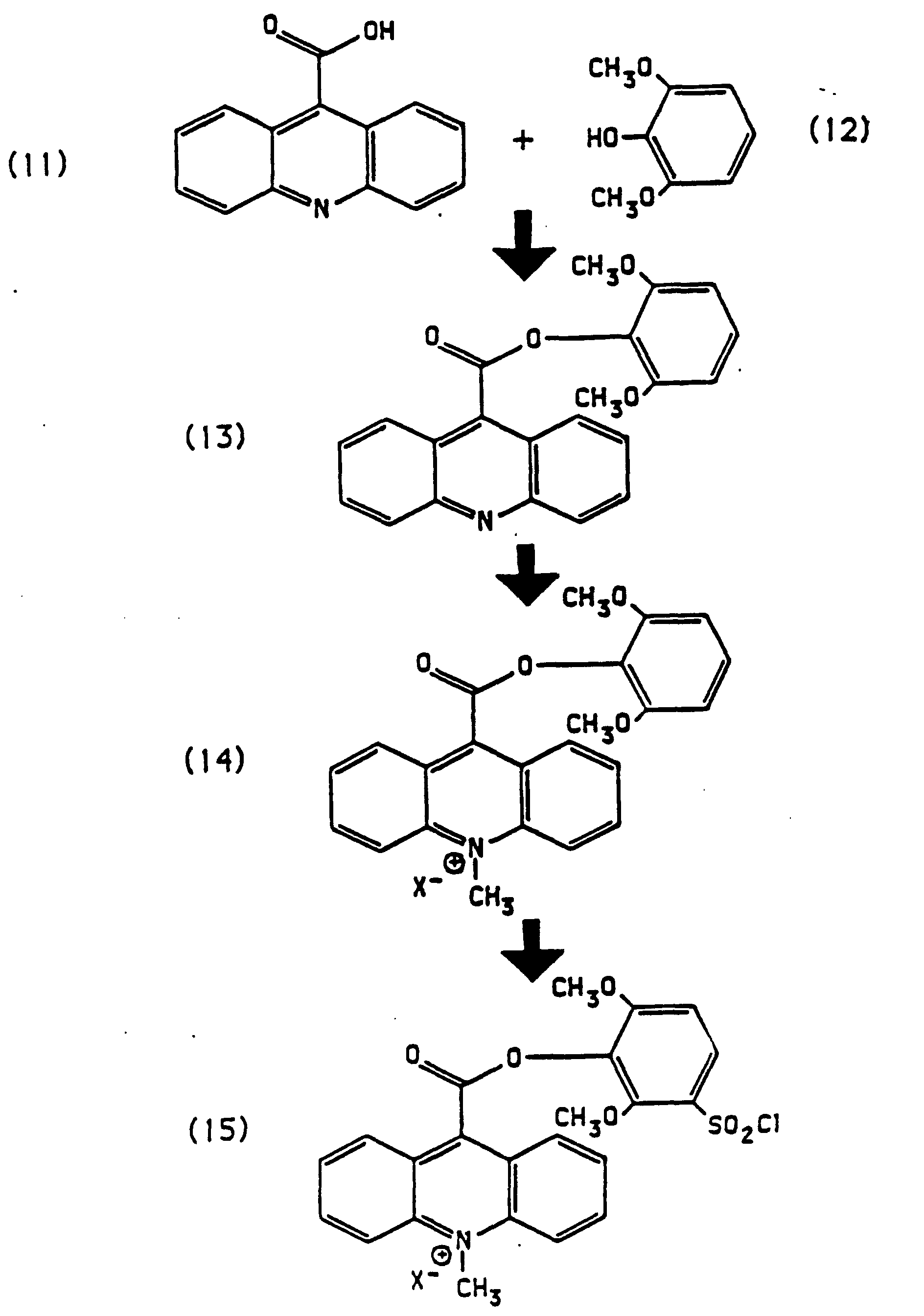 Esterification of acridine-9-carboxylic acid (11) with 2,6-dimethoxyphenol (12) via the acid chloride provides the ester (13). Methylation of the acridine nitrogen with methylfluorosulfate and subsequent chlorosulfonation with chlorosulfonic acid gives the label (15). These reactions are described in further detail in the following.

Acridine-9-carboxylic acid (11) (6.10 g, 0.027 moles) in a 250 ml round bottom flask was mixed with thionyl chloride (130 ml) and the mixture was refluxed for 2 hours with stirring. The excess thionyl chloride was removed in a rotary evaporator. The residue was treated with benzene (75 ml) and the solvent was removed in vacuo to remove traces of thionyl chloride. The residue of acridine-9-carbonyl chloride (11) was mixed with pyridine (130 ml) and 2,6-dimethoxyphenol (12) (4.16 g, 0.027 moles) was added. The mixture was warmed using a water bath (about 60° C) to dissolve all the solids. After 15 hours of stirring at room temperature the mixture was poured into 1 liter of water The suspension was acidified with concentrated hydrochloric acid to pH 2.0. The solid product was filtered, washed with water and dissolved in chloroform. Drying (anhydrous sodium sulfate) and evaporation of chloroform gave (2,6-dimethoxy)phenyl-acridine-9-carboxylate (13). This was chromatographed on a silica gel column using CHCl₃/EtOAc, 98:2 as solvent. The fractions with R_{f} value of 0.19 on TLC with the same solvent were pooled and evaporation of the solvents gave the pure ester (13) (yield = 30%).

(2,6-dimethoxy)phenyl-acridine-9-carboxylate (13) (2.01 g, 5.6 mmole) was dissolved in dichloromethane (110 ml, anhydrous) in a 250 ml round bottom flask. Methyl fluorosulfate (4.60 ml, 6.48 g, 56 mmoles) was added and the mixture was stirred at room temperature for 15 hours. Anhydrous ether (100 ml) was added and the precipitated bright yellow solids were filtered after stirring the suspension for 0.5 hours. The solid was washed well with ether (about 100 ml) and then with pentane (50 ml). The acridinium was recrystallized from acetonitrile to provide pure 2,6-dimethoxy-phenyl-acridinium-9-carboxylate fluorosulfonate (14) (yield = 81 %).

In a dry two neck 25 ml round bottom flask were placed the (2,6-dimethoxy)phenyl-10-methyl acridinium-9-carboxylate fluoro-sulfonate (14) (101.7 mg, 0.215 mmole), a magnetic stirring bar and anhydrous CH₂Cl₂ (5 ml). The suspension was stirred and cooled to -20° C in a CCl₄/dry ice bath. Chlorosulfonic acid (72 1,0.125 g, 1.07 mmole) was added and stirring continued at -20°C for 30 minutes. The reaction mixture was then allowed to warm slowly to room temperature and stirred for an additional 2 hours. Anhydrous ether (5 ml) was added to the reaction flask causing the formation of a light yellow precipitate. It was filtered and washed thoroughly with ether. Drying under vacuum gave (2,6-dimethoxy-3-chlorosulfonyl)phenyl acridinium-9-carboxylate fluorosulfonate (15) (yield = 93.4%). MS: FAB, dithiothreitol/dithioerythrytol matrix, 472 (M⁺).

### Example 3

Another preferred moiety of the present invention is (2,6-dimethyl-4-bromo)phenyl-N-methyl-acridinium-9-carboxylate fluorosulfonate which has the following formula:

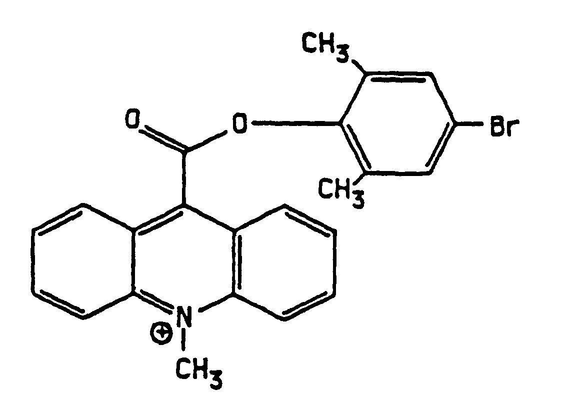

9-(2,6-dimethyl-4-bromo)phenyl-N-methyl-acridinium-9-carboxylate fluorosulfonate was synthesized by the same esterification procedure as (2,6-dimethoxy-3-chlorosulfonyl)phenyl-N-methyl-acridinium-9-carboxylatefluorosulfonate, with the substiution of 2,6-dimethyl-4-bromo-phenol for the substituted phenol employed in the (2,6-dimethoxy-3-chlorosulfonyl)phenyl-N-methyl-acridinium-9-carboxylate fluorosulfonate synthesis.

### Example 4

Another preferred moiety of the present invention is (2,6-dimethyl-3-chlorosulfonyl)phenyl-N-methyl-acridinium-9-carboxylate fluorosulfonate which has the following formula:

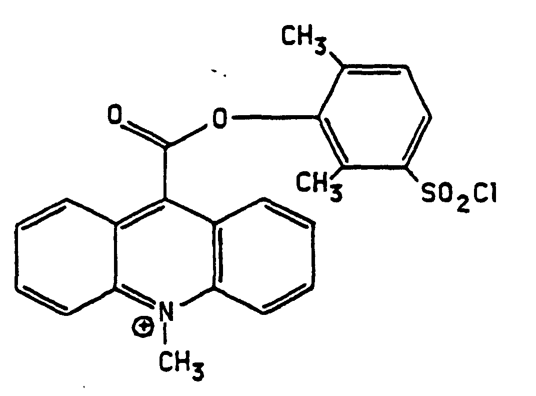

(2,6-dimethyl-3-chlorosulfonyl)phenyl-N-methyl-acridinium-9-carboxylate fluorosulfonate was synthesized by the same method as (2,6-dimethoxy-3-chlorosulfonyl)phenyl-N-methyl-acridinium-9 -carboxylate fluorosulfonate with the substitution of 2,6-dimethylphenol for 2,6-dimethoxyphenol in the esterification step.

### Example 5

Another moiety of the present invention is (2,6-dimethyl-4-nitro)phenyl-3-(3-succinimidyloxycarbonyl)propyloxy-9,10-dihydro-N-methyl-acridan-9-carboxylate which has the following formula: 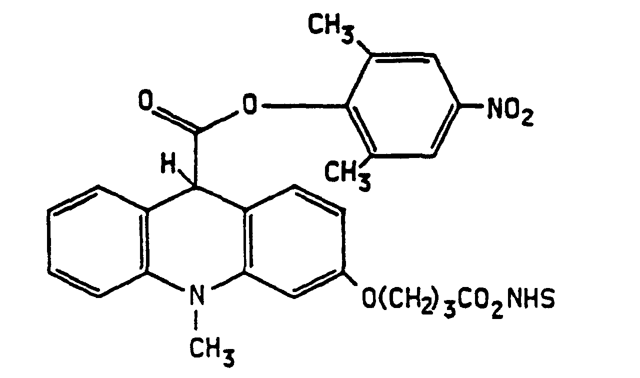 (2,6-dimethyl-4-nitro)phenyl-3-(3-succinimidyloxycarbonyl)propyloxy-9,10-dihydro-N-methyl-acridan-9-carboxylate was synthesized from the acridinium acid (9). Reduction of the acid (9) with sodium cyanoborohydride gives the acrid an which is then converted to the NHS ester by the mixed anhydride method. These reactions are described in further detail in the following.

The acridinium acid (9) (210 mg, 0.37 mmole) was dissolved in a 1:1 mixture of acetonitrile and 0.1 M phosphate buffer, pH 5.2 (60 ml). A solution of sodium cyanoborohydride (190 mg) in acetonitrile (5 ml) was added dropwise to the acridinium solution. This results in the bleaching of the yellow color of the solution. Stirring was continued for 15 minutes. Acetonitrile (100 ml) was added and the solvents were removed in a rotary evaporator. The residue as a suspension in water is extracted with ethylacetate. The organic layer was washed with water and dried. Removal of solvents gave (2,6-dimethyl-4-nitro)phenyl-3-(3-carboxyl)propyloxy-9,10-dihydro-acridan-9-carboxylate (yield = 90%).

The acridan acid (125 mg, 0.255 mmole) and N-methylmorpholine (28 l) were dissolved in anhydrous acetonitrile (15 ml). The mixture was cooled in a CCl₄/dry ice bath under nitrogen. Isobutylchloroformate (35 l) was added, the mixture was stirred for 3 minutes and N-hydroxysuccinimide (35 mg) dissolved in acetonitrile (2 ml) was added. After stirring at -20°C for 15 minutes the CCl₄/dry ice bath was removed and the reaction allowed to warm up to room temperature. After 2 hours the solvents were evaporated and the residue extracted into ethyl acetate. The insoluble N-methylmorpholine hydrochloride salt was removed by filtration. The filtrate was concentrated and hexane (20 ml) was added. Cooling results in crystallization of (2,6-dimethyl-4-nitro)phenyl-3-(3-succinimidyloxy-carbonyl)propyloxy-9, 1 0-dihydro-N-methyl-acridan-9-carboxylate. The crystals were finally filtered and washed with hexane (yield = 70%). MS: FAB, dithiothreitol/dithioerythrytol matrix, 588 (M⁺ +1). HPLC: Waters C₁₈ Novapak (3.9 mm x 15 mm)(commercially available from Millipore Corporation, Waters Chromatography Division, Milford, Massachusetts), CH₃CN/N₂O (0.1% trifluoracetic acid) 60:40, flow rate 1.0 ml/min, retention time 6.34 min, detected at 280 nm.

### Example 6

Another moiety of the present invention is (2,6-dimethyl-4-nitro)phenyl-N-methyl-acridinium-9-carboxylate-3-oxo-butyrimidate chloride, hydrochloride which has the following formula: 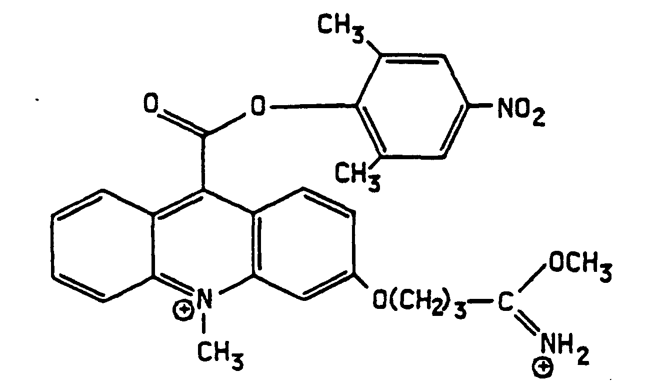 (2,6-dimethyl-4-nitro)phenyl-N-methyl-acridinium-9-carboxylate-3-oxobutyrimidate chloride, hydrochloride is synthesized according to the following scheme: 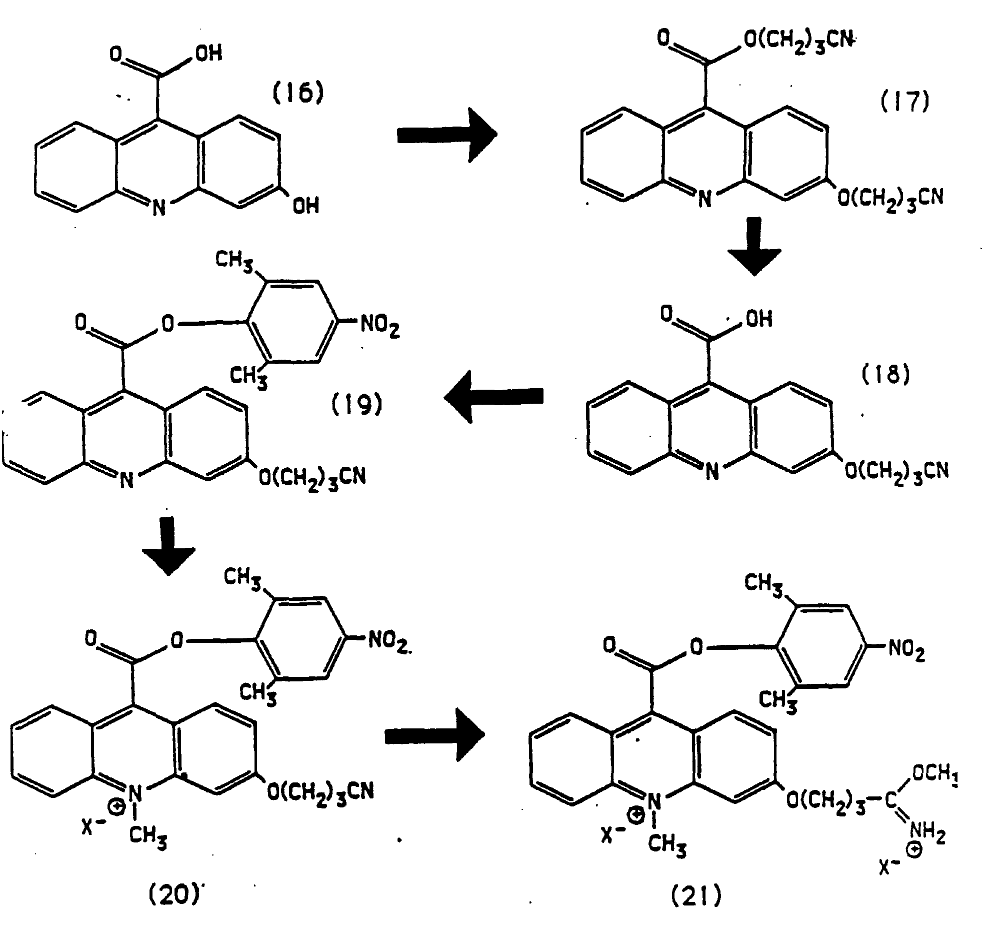 Reaction of 3-hydroxy-9-acridine carboxylic acid (16) with 4-bromobutyronitrile gives an ester (17). Hydrolysis of the ester and reesterification with 2,6-dimethyl-4-nitrophenol provides (19). Methylation with methyl fluorosulfate and conversion of the cyano group to the imidate ester using hydrogen chloride gas and methanol provides (2,6-dimethyl-4-nitro)phenyl-N-methyl-acridinium-9-carboxylate-3-oxo-butyrimidate chloride, hydrochloride (21). These reaction are described in further detail in the following.

3-Hydroxy-9-acridine carboxylic acid (16) (2 g, 8.4 mmole), 4-bromobutyronitrile (5.87 ml, 8.74 g, 34 mmole) and cesium carbonate (11.08 g, 34 mmole) were dissolved in anhydrous DMSO (50 ml) in a 100 ml round bottom flask. The mixture was warmed to about 50° C in a water bath with stirring. After 3 hours the mixture was poured into water (600 ml). The solids were filtered and were dissolved in chloroform. Drying and evaporation of the solvent gave 3-(3-cyano)propoxyl-acridine-9-carboxylic acid-(3-cyano)propyl ester (17). It was then dissolved in toluene (50 ml) and cyclohexane (150 ml) was added. The pure product (17) separated, and was then filtered and dried. The dried product was purified by thin layer chromatography with ethylacetate as the mobile phase (R_{f} = 0.58) (yield = 78.6%).

The cyanopropyl ester (17) (3.73 g, 10 mmole) was dissolved in a mixture of 0.5N NaOH (90 ml) and methanol (90 ml) and stirred in a water bath at 60° C using a reflex condenser for 2.5 hours. The methanol was removed in a rotary evaporator and the product was extracted with ethyl acetate after acidifying the aqueous phase with concentrated hydrochloric acid. Drying and evaporation of the solvent provides 3-(3-cyano) propoxyl-acridine-9-carboxylic acid (18) (yield = 80%).

The carboxylic acid (18) (4.62 g, 15 mmole) was dissolved in pyridine (130 ml) and the solution was cooled in a CCl₄/dry ice bath. p-toluenesulfonyl chloride (5.8 g, 30 mmole) was added and the bath was removed. After 15 minutes of stirring at room temperature, 2,6-dimethyl-4-nitrophenol (2.8 g, 16.8 mmole) was added. After 18 hours at room temperature, water (10 ml) was added and the solvents were removed in vacuo. The residue was dissolved in chloroform (200 ml) and the organic layer was washed with saturated sodium bicarbonate (2 x 100 ml), water (2 x 100 ml), 1 N HCl (1 x 100 ml) and finally with water (2 x 100 ml). Drying and evaporation of the solvent gave (2,6-dimethyl-4-nitro)phenyl-3-(3-cyano) propoxyl-acridine-9-carboxylate (19) which was chromatographed in a silica gel column using ethylacetate/hexane (7:3) as solvent (yield = 74.5%).

The ester (19) (1.6 g, 3.52 mmole) was dissolved in dry methylene chloride (50 ml) and under nitrogen methyl fluorosulfate (1.6 ml, 17.6 mmole) was added. The solution was stirred at room temperature for 20 hours. Anhydrous ether (100 ml) was added and the precipitated (2,6-dimethyl-4-nitro)phenyl-3-(3-cyano) propoxyl-acridinium-9-carboxylate fluorosulfonate (20) was filtered, washed with ether and dried in vacuo (yield - 84.7%).

The acridinium ester (20) (4 mg, 7.4 x 10⁻³ mmole) was dissolved in methanol (0.5 ml) in a 5 ml 2-necked flask. Anhydrous hydrogen chloride gas was bubbled carefully for 10 minutes. Anhydrous ether (3 ml) was added. The precipitated (2,6-dimethyl-4-nitro)phenyl-N-methyl-acridinium-9-carboxylate-3-oxo-butyrimidate chloride, hydrochloride (21) was collected and washed with ether. The solid was dried in vacuum and was stored in a dessicator containing phosphorus pentoxide.

### Example 7

Another moiety of the present invention is (2,6-dimethyl-4-nitro)phenyl-N-methyl-phenanthridinium-6-carboxylate fluorosulfonate which has the following formula: 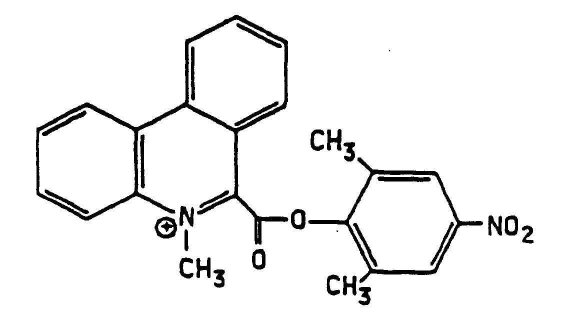 (2,6-dimethyl-4-nitro)phenyl-N-methyl-phenanthridinium-6-carboxylate fluorosulfonate is synthesized according to the following scheme:

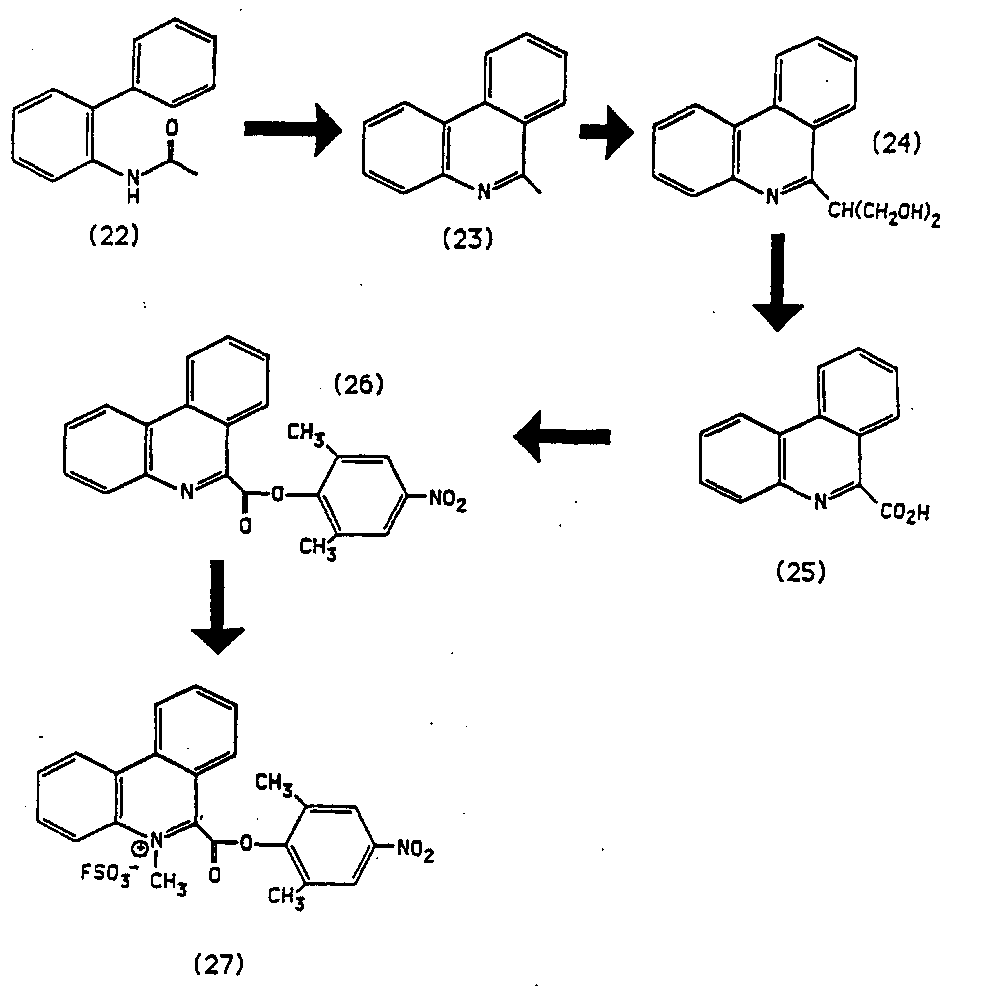

2-aminobiphenyl (16.9 g, 0.1 mol) was dissolved in anhydrous pyridine (30 ml) and acetic anhydride (10.5 ml, 0.11 mol) was added. The solution was shaken briefly and cooled to room temperature and let stand for 15 hours. After the addition of water (50 ml) N-acetyl-2-aminobiphenyl (22) was filtered off and recrystallized from aqueous ethanol to give 19.6 g of white needles (yield = 93%).

N-acetyl-2-aminobiphenyl (22) (19 g, 0.09 mol) was gently refluxed with freshly distilled phosphoryl chloride (45 ml, 0.49 mol) for 80 minutes. The solution was then cooled in ice and the precipitate (6-methylphenanthridine-hydrochloride) was filtered off, dissolved in water and made alkaline with aqueous ammonia. The solution was then extracted with ether (4 x 75 ml). The extract was dried over sodium sulfate and the ether was removed in vacuo. The resulting yellow oil was dissolved in boiling cyclohexane (400 ml) and on cooling formed white needles of 6-methylphenanthridine (23) (yield = 63%).

6-(2-hydroxy-1-hydroxymethylethyl)-phenanthridine (24) was prepared by treating 6-methylphenanthridine (23) with formaldehyde according to the method of Morgan and Walls, J. Chem. Soc. 34:2447 (1931), which is incorporated herein by reference. 6-(2-hydroxy-1-hydroxymethylethyl)-phenanthridine (24) formed as white needles (yield = 57%).

A mixture of 6-(2-hydroxy-1-hydroxymethyl)-phenanthridine (24) (6 g, 31 mmoles) and finely powdered selenium dioxide (3.8 g, 34 mmoles) was refluxed in ethyl acetate (125 ml) for 10 hours. The deep red solution was then filtered while hot through celite, before evaporating to dryness. The resulting solid was digested in warm 1M hydrochloric acid (125 ml), filtered and partially neutralized with sodium bicarbonate. The initial red precipitate was filtered off before completely neutralizing the solution. The resulting pale yellow solid was filtered off and recrystallized from acetone/pet. ether to give 2.7 g of 6-formylphenanthridine (yield = 42%).

6-carboxyphenanthridine (25) was prepared by chromic acid oxidation of 6-formylphenanthridine according to the method of Morgan and Walls, J. Chem. Soc. 34:2447 (1931), which is incorporated herein by reference The product (25) formed as a white powder (yield = 60%).

6-carboxyphenanthridine (25) (662 mg, 3 mmoles) was dissolved in anhydrous pyridine (14 ml) and cooled to 0°C. p-toluene-sulfonyl chloride (1.15 g, 6 mmoles) was added followed by 2,6-dimethyl-4-nitro-phenol (501 mg, 3 mmoles) and the mixture was allowed to stand overnight at 4°C. The resulting brown solution was stirred into iced water. The precipitate was filtered off and was chromatographed on a silica gel using chloroform/hexane (1: 1) to obtain (2,6-dimethyl-4-nitro)phenyl-phenanthridine-6-carboxylate (26) (yield = 60%).

In a dry two neck 5 ml round bottom flask the ester (26) (369 mg, 1 mmole) was suspended in anhydrous methylene chloride (5 ml). The suspension was cooled in a dry ice/CCl₄ bath under nitrogen. Chlorosulfonic acid (342 I, 6 mmole) was added and stirring continued at -20°C for 30 minutes. The mixture was then allowed to warm slowly to room temperature and stirred for an additional 2 hours. Anhydrous ether (20 ml) was added and the precipitated solids were filtered and washed with ether. Drying gave (2,6-dimethyl-4-nitro)phenyl-N-methyl-phenanthridinium-6-carboxylate fluorosulfonate (27) (yield = 90%).

### Example 8

Another moiety of the present invention is (2,6-dimethyl-4-nitro)phenyl-5,6-dihydro-N-methyl-phenanthridinium-6-carboxylate fluorosulfonate which has the following formula:

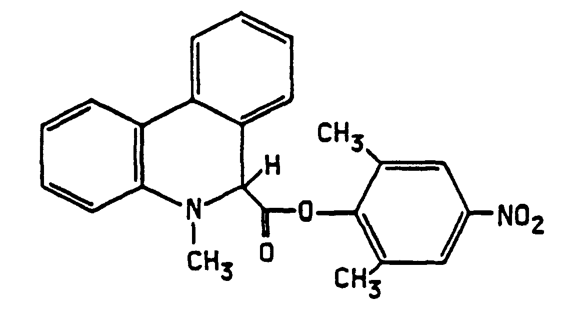

(2,6-dimethyl-4-nitro)phenyl-5,6-dihydro-N-methyl-phenanthridinium-6-carboxylate fluorosulfonate is synthesized from the unreduced phenanthridinium analog (27). The phenanthridinium (27) (398 mg, 0.8 mmole) was dissolved in a 1:1 mixture of acetonitrile and 0.1 M phosphate buffer, pH 5.2 (80 ml). A solution of sodium cyanoborohydride (410 mg) in acetonitrile (10 ml) was added dropwise to the phenanthridinium solution. This resulted in the bleaching of the yellow color of the solution. Stirring was continued for 15 minutes. Acetonitrile (100 ml) was added and the solvents were removed in a rotary evaporator. The residue was suspended in water and extracted with ethylacetate. The organic layer was washed with water and dried. Removal of solvents gave (2,6-dimethyl-4-nitro)phenyl-5,6-dihydro-N-methyl-phenanthridinium-6-carboxylate-6-carboxylate fluorosulfonate (yield = 90%).

### Example 9

Another moiety of the present invention is (2,6-dimethoxy-3-chlorosulfonyl-phenyl)-2-phenyl-N-methyl-quinolinium-4-carboxylate fluorosulfonate which has the following formula: 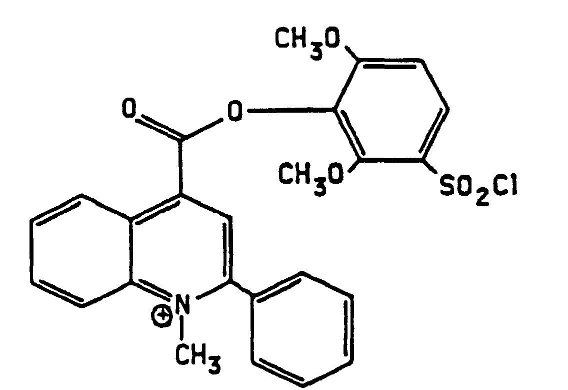 (2,6-dimethoxy-3-chlorosulfonyl-phenyl)-2-phenyl-N-methyl-quinolinium-4-carboxylate fluorosulfonate is synthesized according to the following scheme:

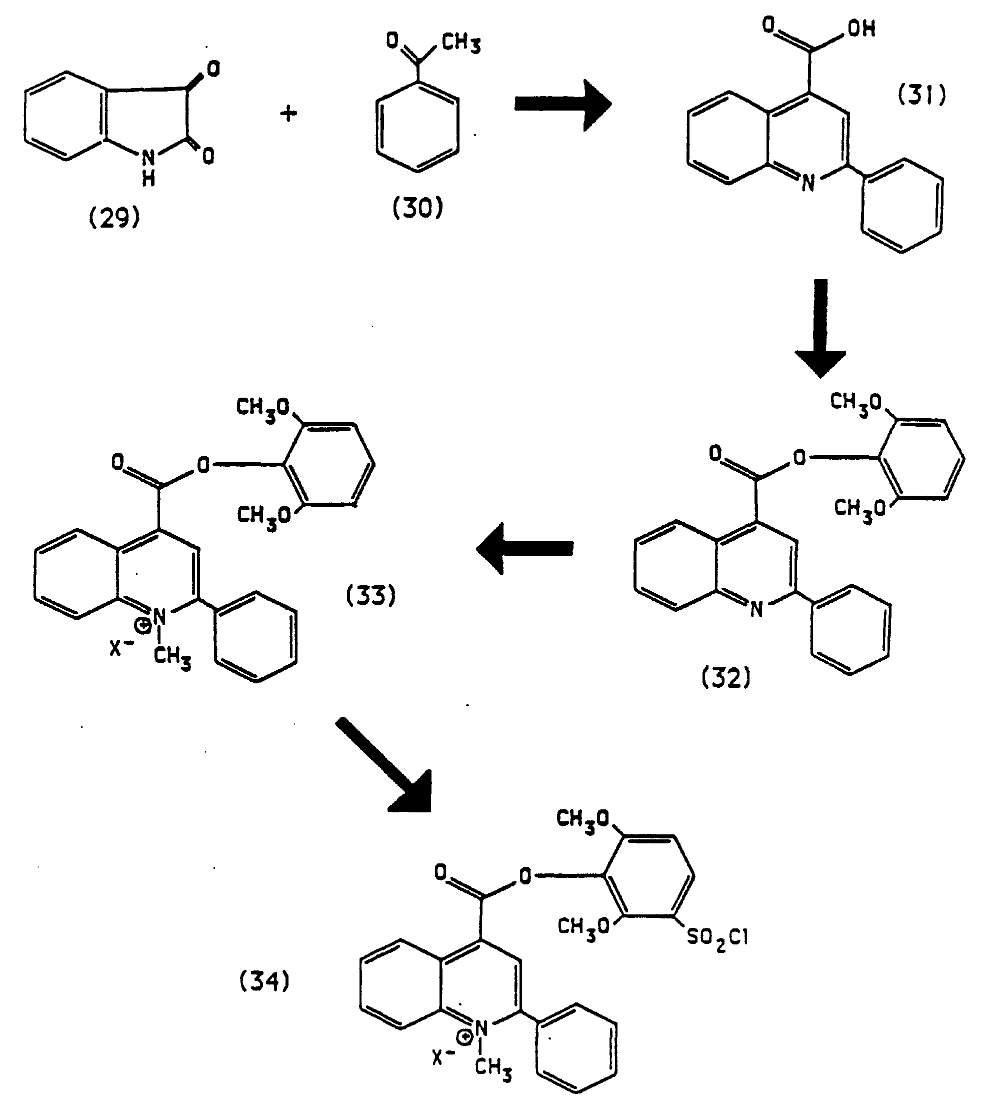

Acetophenone (29) (120 g, 1 mol) and isatin (30) (147 g, 1 mol) were refluxed for 10 hours in water and ethanol, with potassium hydroxide (17 g). 2-phenyl-quinoline-4-carboxylic acid (31) was recovered from ethanol as white needles (yield 84%).

2-phenyl-quinoline carboxylic acid (31) (735 mg, 3 mmoles) was dissolved in anhydrous pyridine (14 ml) and cooled in an icewater bath. p-Toluene sulfonyl chloride (1.15 g, 6 mmoles) was added and the mixture was stirred for 15 mins. 2,6-dimethoxy phenol (462 mg, 3 mmoles) was added and the mixture was stirred at room temperature for 15 hours. The solution was poured into ice water (300 ml) and the (2,6-dimethoxy)phenyl-2-phenyl-quinoline-4-carboxylate (32) was filtered. The solids were dried and purified on a silica gel column using chloroform/hexane (1:1) (yield = 50%).

The ester (32) (381 g, 1 mmole) was dissolved in anhydrous methylene chloride (3 ml) and methyl fluorosulfate (492 l, 0.69 g, 6 mmoles) was added. After stirring for 15 hours at room temperature under nitrogen anhydrous ether (20 ml) was added. The (2,6-dimethoxy)phenyl-2-phenyl-quinoline-4-carboxylate-N-methyl fluorosulfonate (33) was filtered and washed with ether and dried (yield = 95%).

In a dry two neck 25 ml round bottom flask the ester (33) (200 mg, 0.4 mmole) was suspended in anhydrous methylene chloride (5 ml). The suspension is cooled in a dry ice/CCl₄ bath under nitrogen. Chlorosulfonic acid (144 l, 2 mmole) was added and stirring continued at -20°C for 0.5 hours. The mixture was then allowed to warm slowly to room temperature and stirred for an additional 2 hours. Anhydrous ether (20 ml) was added and the precipitated (2,6-dimethoxy-3-chlorosulfonyl)phenyl-2-phenyl-N-methyl-quinolinium-4-carboxylate fluorosulfonate (34) was filtered and washed with ether and dried (yield = 90%).

### Example 10

Another moiety of the present invention is (2,6-dimethyl-4-bromo)phenyl-2-phenyl-1,4-dihydro-N-methyl-quinoline-4-carboxylate which has the following formula:

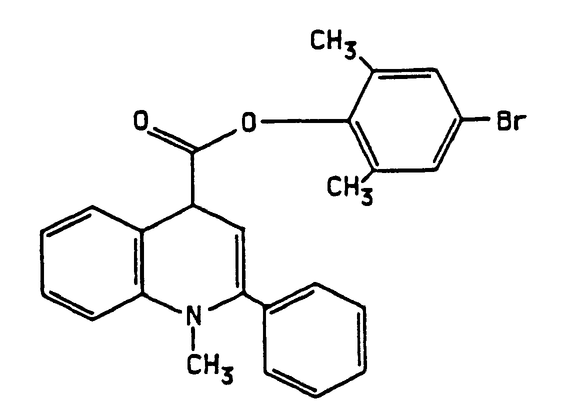

(2,6-dimethyl-4-bromo)phenyl-2-phenyl-1,4-dihydro-N-methyl-quinoline-4-carboxylate was synthesized from the (2,6-dimethyl-4-bromo)phenyl-2-phenyl-N-methyl-quinolinium-4-carboxylate by reduction with sodium cyanoborohydride. The unreduced quinolinium was obtained by the same procedure as the (2,6-dimethyl-4-bromo)phenyl acridinium moiety described above, with the substitution of quinoline for acridine.

The quinolinium ester (500 mg, 0.9 mmole) was dissolved in a 1:1 mixture of acetonitrile and 0.1 M phosphate buffer pH 5.2 (80 ml). A solution of sodium cyanoborohydride (56 mg, 0.9 mmole) in acetonitrile/buffer mixture (10 ml) was added. After stirring for 2 minutes, the mixture was acidified to pH 2.0 and acetonitrile (100 ml) was added. The solvents were removed in a rotary evaporator. The residue was suspended in water and was extracted with ethylacetate. Drying and evaporation of the ethyl acetate gave (2,6-dimethyl-4-bromo)phenyl-2-phenyl-1,4-dihydro-N-methyl-quinoline-4-carboxylate (yield = 70%).

### Example 11

Another moiety of the present invention is (2,6-dimethyl-4-bromo)phenyl-2-phenyl-1,2,3,4-tetrahydro-N-methyl-quinoline-4-carboxylate which has the following formula:

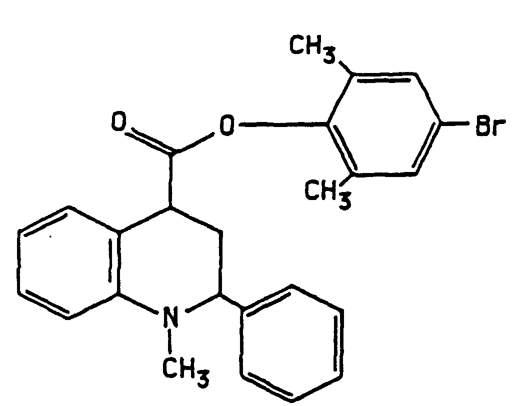

(2,6-dimethyl-4-bromo)phenyl-2-phenyl-1,2,3,4-tetrahydro-N-methyl-quinoline-4-carboxylate was synthesized from the (2,6-dimethyl-4-bromo)phenyl-2-phenyl-N-methyl-quinolinium-4-carboxylate by reduction with sodium cyanoborohydride. The unreduced quinolinium was obtained by the same procedure as the (2,6-dimethyl-4-bromo) phenyl acridinium compound described above, with the substitution of quinoline for acridine.

The quinolinium ester (500 mg, 0.9 mmole) was dissolved in a 1:1 mixture of acetnitrile and 0.1 M phosphate buffer pH 5.2 (80 ml). A solution of sodium cyanoborohydride (560 mg, 9.0 mmole) in acetonitrile/buffer mixture (20 ml) was added. After stirring for 30 minutes, the mixture was acidified to pH 2.0 with 0.1 N hydrochloric acid and stirred for an additional 5 minutes. Acetonitrile (100 ml) was added and the solvents were removed in a rotary evaporator. The residue was suspended in water and was extracted with ethylacetate. Drying and evaporation of the ethyl acetate gave (2,6-dimethyl-4-bromo)phenyl-2-phenyl-1,2,3,4-tetrahydro-N-methyl-quinoline-4-carboxylate (yield = 80%).

### Example 12

Another preferred moiety of the present invention is (2,6-dimethyl-4-trimethylammonio)phenyl-N-methyl-acridinium-9-carboxylate difluorosulfonate which has the following formula: 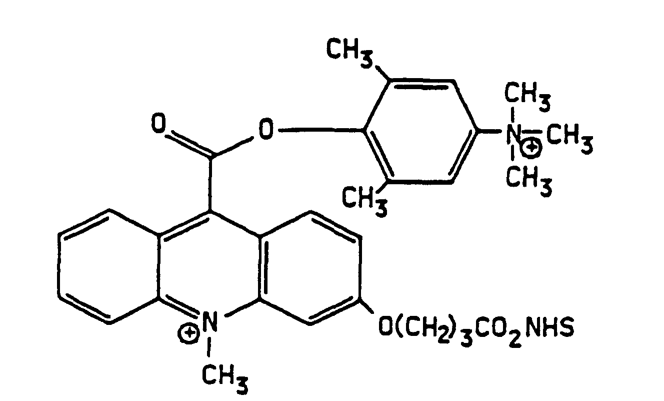 (2,6-dimethyl-4-trimethylammonio)phenyl-N-methyl-acridinium-9-carboxylate difluorosulfonate is synthesized according to the following scheme: 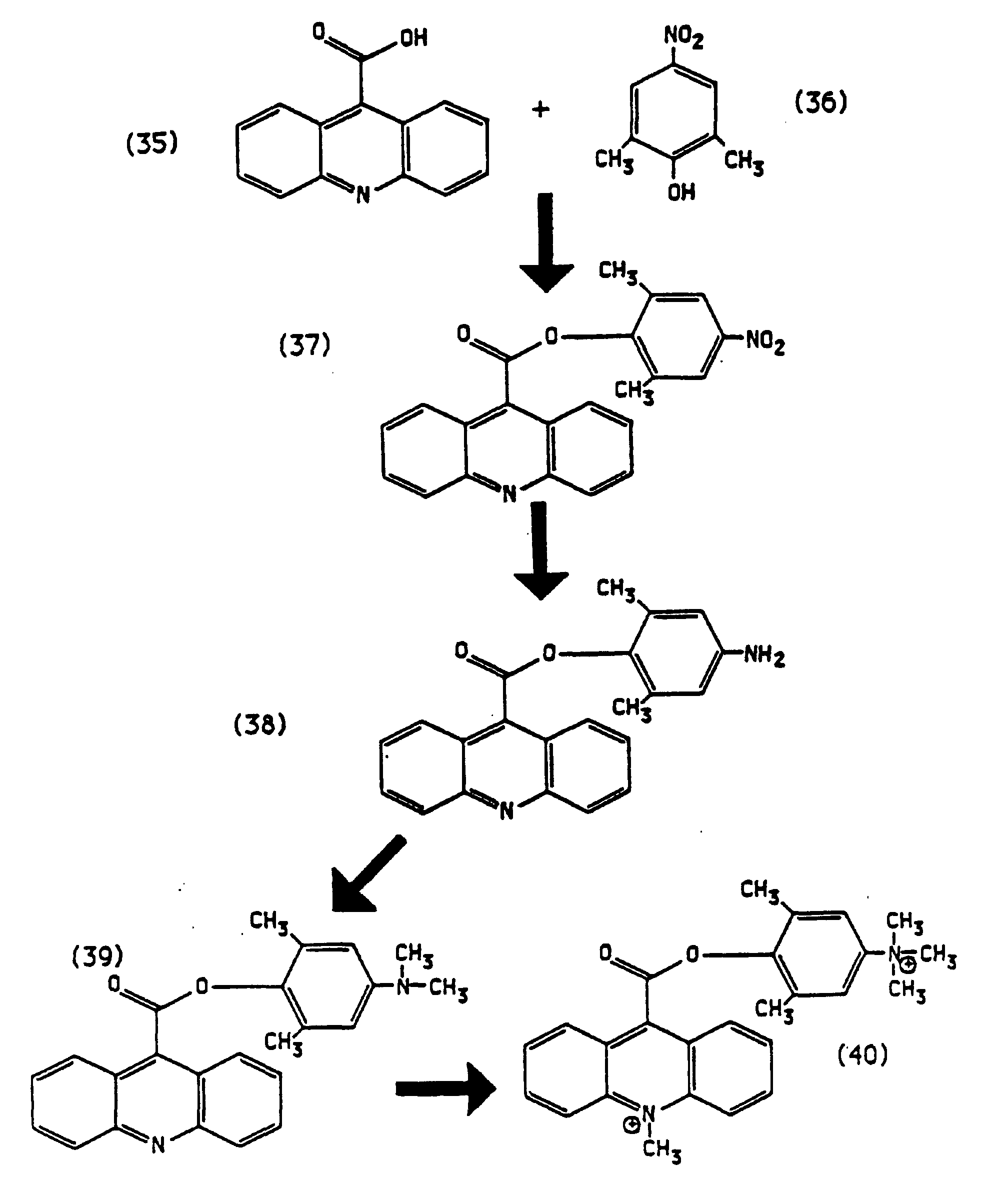 (2,6-dimethyl-4-trimethylammonio)phenyl-N-methyl-acridinium-9-carboxylate (35) was obtained by esterification of acridine-9-carboxylic acid with 2,6-dimethyl-4-nitrophenol (36). The product (37) was reduced to the (2,6-dimethyl-4-amino)phenyl-acridine-9-carboxylate (38) with zinc. Two methyl groups were introduced on the amino group by treatment with methyl iodide. Quatemization and acridinium formation was then accomplished using methyl fluorosulfate. These reactions are described in further detail in the following.

Acridine-9-carboxylic acid (35) (3.05 g, 0.014 moles) in a 250 ml round bottom flask was mixed with thionyl chloride (65 ml) and the mixture was refluxed for 2 hours with stirring. The excess thionyl chloride was removed in a rotary evaporator. The residue was treated with benzene (75 ml) and the solvent was removed in vacuo to remove traces of thionyl chloride. The residue of acridine-9-carbonyl chloride was mixed with pyridine (65 ml) and 2,6-dimethyl-4-nitrophenol (36) (2.25 g, 0.014 moles) was added. The mixture was warmed using a water bath (about 60° C) to dissolve all the solids. After 15 hours of stirring at room temperature the mixture was poured into 1 liter of water. The suspension was acidified with concentrated hydrochloric acid to pH 2.0. The solid product was filtered, washed with water and dissolved in chloroform. Drying (anhydrous sodium sulfate) and evaporation of chloroform gave the crude ester.

The crude ester was chromatographed on a silica gel column using CHCl₃/EtOAc 98:2 as solvent. The fractions with R_{f}value of 0.6 on TLC with the same solvent were pooled and evaporation of the solvents gave pure (2,6-dimethyl-4-nitro)phenyl-acridine-9-carboxylate (37) (yield = 30%).

The (2,6-dimethyl-4-nitro)phenyl ester (37) (1.16 g, 3.1 mmole) was dissolved in acetic acid (50 ml) by warming in an oil bath at about 65°C. Stannous chloride (1.5 g) was dissolved in concentrated hydrochloric acid (10 ml) and was added to the ester solution. The mixture was stirred for 45 minutes and was then poured into water (750 ml). Extraction with chloroform (3 x 200 ml) removed unreacted (2,6-dimethyl-4-nitro)phenyl ester. The aqueous layer was made basic with sodium bicarbonate and was extracted with chloroform (4 x 200 ml). Drying and evaporation of the chloroform gave (2,6-dimethyl-4-amino)phenyl-acridine-9-carboxylate (38) (yield = 25%).

The amino ester (38) (64 mg, 0.18 mmole) was dissolved in nitromethane (5 ml). Methyl iodide (1 ml) and pyridine (0.1 ml) were added. The mixture was stirred at room temperature for 15 hours. Methanol (2 ml) was added and the mixture was then stirred for an additional 2 hours. The solvents were evaporated and the residue was treated with water (10 ml) and was then extracted with chlroform (4 x 20 ml) after the solution was made basic. Drying and evaporation of the chloroform gave (2,6-dimethyl-4-dimethylamino)phenyl-acridine-9-carboxylate (39) (yield = 50%).

The dimethylamino ester (39) (154 mg. 0.41 mmole) was dissolved i methylene chloride (2 ml). Methyl fluorosupfate (265 1, 3.28 mmole) was added and the mixture was stirred at room temperature for 15 hours. Amhydrous ether (15 ml) was added and the precipitated solids were filtered and washed with ether. Drying gave (2,6-dimethyl-4-trimethylammonio)phenyl-N-methyl-acridinium-9-carboxylate (40) (yield = 50%). MS:FAB, thioglycerol matrix, m/e 400 (M⁺).

### LABELLING PROTEIN AND OTHER MATERIAL WITH CHEMILUMINESCENT MOIETIES

### Example 13

A conjugate of the present invention comprises progesterone bound to a β-D-thioglucose adduct of (2,6-dimethyl-3-chlorosulfonyl)phenyl-N-methyl-acridinium-9-carboxylate fluorosulfonate. The progesterone conjugate of the β-D-thioglucose adduct of (2,6-dimethoxy-3-chlorosulfonyl)phenyl-N-methyl-acridinium-9-carboxylate fluorosulfonate has the following formula: 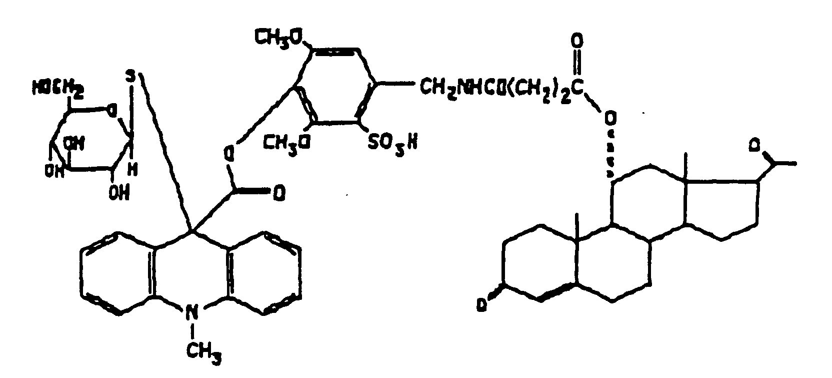 The progesterone conjugate is synthesized according to the following scheme:

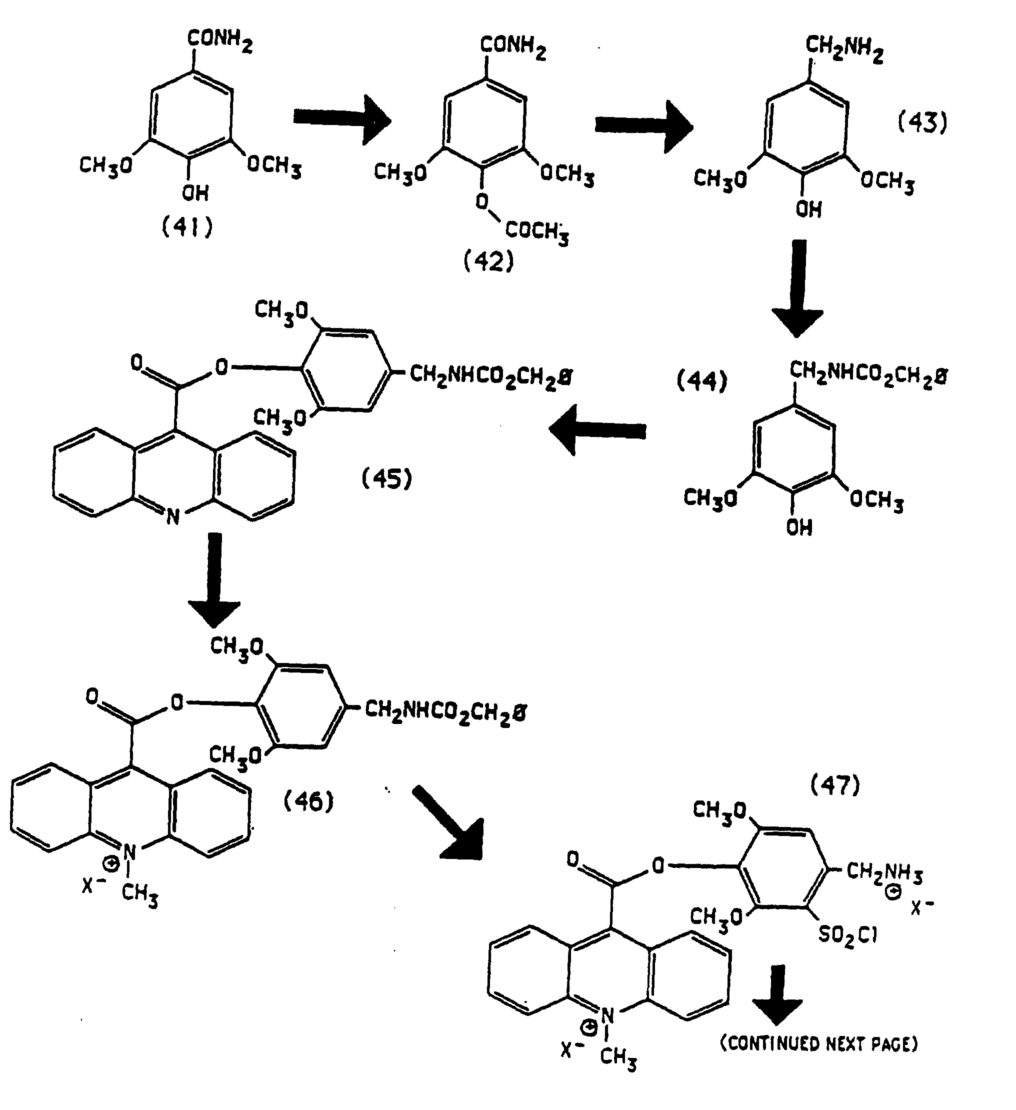

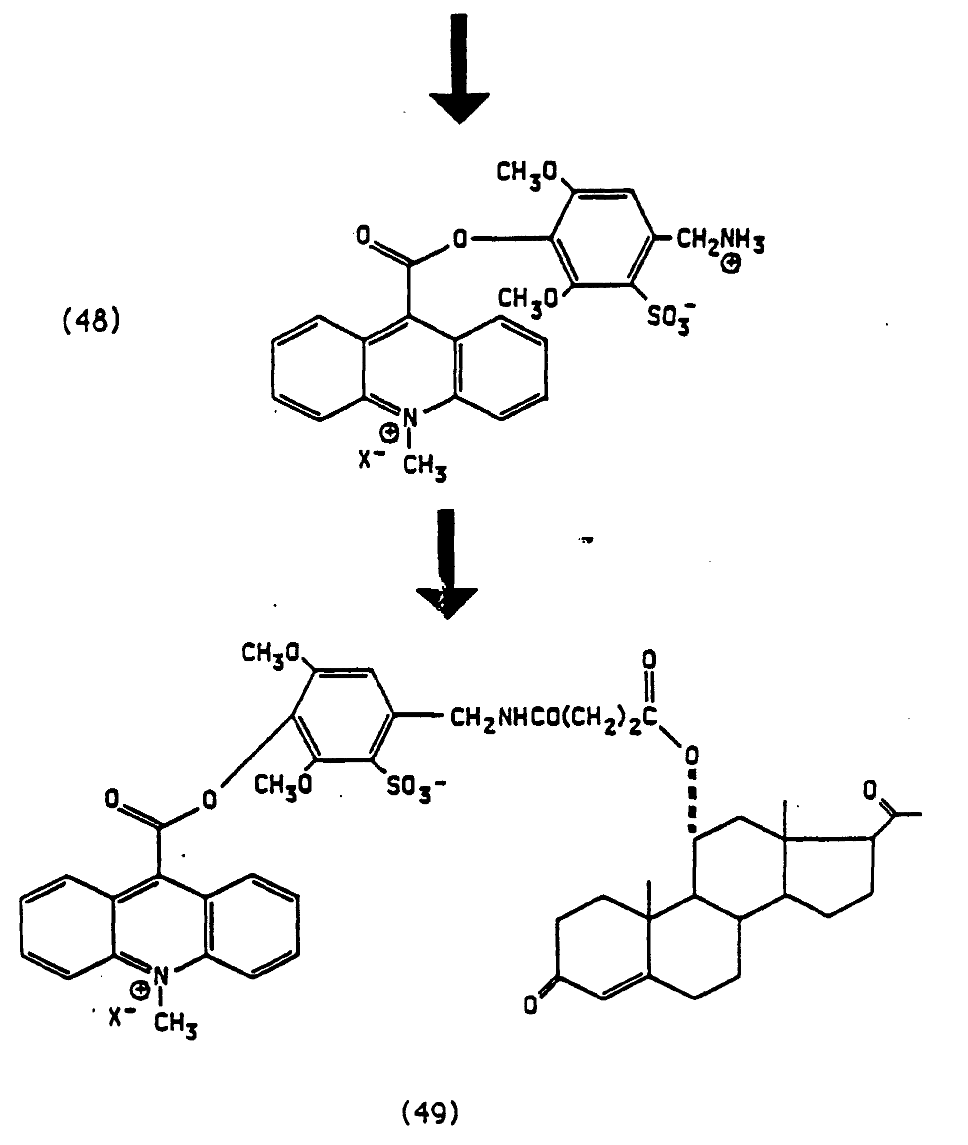

3,5-dimethoxy-4-hydroxy benzamide (35) (3.0 g, 15.2 mmole) was dissolved in anhydrous pyridine (15 ml) and the solution was cooled in a dry ice/CCl₄ bath. Acetyl chloride (1.4 ml, 1.54 g, 19.7 mmole) was added and the mixture was kept stirred at room temperature for 2 hours. Methanol (1 ml) and water (5 ml) were added and the solvents were removed under reduced pressure. The residue was treated with water (50 ml) acidified with dilute hydrochloric acid and was extracted with ethyl acetate. Washing with water, drying and evaporating of the ethyl acetate gave 2,6-dimethoxy-4-carbozamido-phenyl acetate (36) (2.2 g) which was recrystallized from ethyl acetate (yield = 60%).

The phenyl acetate (36) (1.27 g, 5.33 mmole) was dissolved in anhydrous tetrahydrofuran (125 ml). Diborane solution in THF (1.0 M, 10.9 ml, 10.9 mmoles) was added and the mixture was refluxed for 4 hours. After cooling to room temperature water (2 ml) and hydrochloric acid (1.0 N, 5 ml) were added. After stirring for 30 minutes the solvents were removed in vacuum. The residue was extracted with chloroform. The chloroform was then dried and removed in vacuo. 2,6-dimethoxy-4-aminomethyl phenol (37) was used in the next step without further purification.

To a solution of the crude amine (37) in anhydrous pyridine (10 ml) benzylchloroformate (1.050 ml, 1.25 g, 7.3 mmole) was added and the mixture was stirred for 3 hours at room temperature. Water (5 ml) was added and the solvents were removed in vacuo. To the residue water (30 ml) was added and the mixture was acidified with dilute HCl. Extraction with ethyl acetate, washing with water, drying and evaporation of the solvent gave 2,6-dimethoxy-4-(benzyloxycarbonyl amino)methyl phenol (38) as an oil (yield = 70% overall).

Acridine-9-carboxylic acid (754 mg, 3.38 mmole) was dissolved in anhydrous pyridine (14 ml). p-Toluene sulfonyl chloride (1.28 g, 6.76 mmole) was added and the mixture was stirred at room temperature for 30 minutes. 2,6-dimethoxy-4-(benzyloxy carbonyl amino)methyl phenol (38) (1.18 g, 3.76 mmole) was added and the mixture was stirred at room temperature for 15 hours. Water (10 ml) was added and solvents were removed in vacuo. The residue was dissolved in chloroform and the chloroform layer was washed successively with water, 0.1 N HCl and sodium bicarbonate solution. Drying and evaporating of chloroform gave the crude ester which was chromatographed on a silica gel column using CHCl₃/Ethyl acetate, 1:1. as the solvent. Evaporation of the solvents from the pooled fractions gave [2,6-dimethoxy-4-(benzyloxycarbonyl amino)methyl]phenyl-acridine-9-carboxylate (39) (yield = 22%).

The acridine (39) (296 mg, 0.57 mmole) was dissolved in anhydrous methylene chloride (5 ml). Methyl fluorosulfate (277 I, 3.4 mmole) was added and the mixture was stirred at room temperature for 5 hours. Anhydrous ether (25 ml) was added and the precipitated [2,6-dimethoxy-4-(benzyloxycarbonyl amino)methyl]phenyl-acridinium-9-carboxylate fluorosulfonate (40) was filtered and washed with ether and dried (yield = 99%).

The acridinium (40) (107 mg, 0.169 mmole) was suspended in anhydrous methylene chloride (2 ml). Chlorosulfonic acid (53 I, 92 mg, 0.797 mmole) was added after the flask was cooled in a dry ice/CCl₄ bath. It was stirred for 30 minutes and the bath was removed. After further stirring at room temperature for 1.5 hours anhydrous ether (20 ml) was added. The precipitated product was filtered and dried in vacuo. The (2,6-dimethoxy-4-aminomethyl-3-chlorosulfonyl)phenyl-acridinium-9-carboxylate fluorosulfonate (41) was directly used in the next reaction.

The sulfonyl chloride (41) (129 mg) was stirred at room temperature in a mixture of methanol (12.5 ml) and water (12.5 ml) for 3 hours. Acetonitrile (35 ml) was added and the solvents were evaporated. The residue was dried in vacuum over phosphorous pentoxide. The (2,6-dimethoxy-4-aminomethyl-3-oxosulfonyl)phenyl-acridinium-9-carboxylate fluorosulfonate (42) was used directly for the next reaction.

Progesterone hemisuccinate (90 mg, 0.209 mmole) and N-methylmorpholine (22 I, 209 mmole) were dissolved in anhydrous DMF (2 ml). The solution was chilled in dry ice/CCl₄ bath and isobutylchloroformate (30 I, 0.229 mmole) was added. After 2 minutes a solution of the acridinium (42) (101 mg, 0.143 mmole) in dimethylsulfoxide (2 ml) containing N-methylmorpholine (3.14 I, 0.28 mmole) was added. Stirring was continued at -20° C for 10 minutes and the cooling bath was removed. After stirring at room temperature for 7 hours, 3 drops of water were added. The solvents were removed in vacuo and ethyl acetate was added to the residue. The oily precipitate was washed repeatedly with ethyl acetate. Upon trituration with acetonitrile (2 ml) the oil separated as solids. The product was purified on HPLC using C₁₈ Dynamax semi-prep column (10 mm x 250 mm) (commercially available from Rainin Instrument Co., Inc., Woburn, Massachusetts) using CH₃CN/H₂O (0.1% trifluoroacetic acid), 55/45 as mobile phase at a flow rate of 2.75 ml/min. The peak apparatus at retention time of 6.00 minutes was collected. Evaporation of solvents gave the conjugate (43) (yield = 30%). MS: FAB, thioglycerol matrix, 895 (M⁺, without any counterions).

The progesterone conjugate (43) (1.1 mg) in a mixture of CH₃CN (1 ml) and H₂O (200 l) was treated with β-D-thioglucose (0.29 mg) as a solution in water (72 l). After 10 minutes the solvents were removed completely under vacuum to provide the β-D-thioglucose adduct depicted above.

### Example 14

The following procedure for attaching to protein is generally applicable to moieties of the present invention.

Mouse IgG (Sigma, 1 mg) was dissolved in 0.9 ml phosphate buffer (100 mM, pH 8.0, 150 mM). The solution was then divided into three equal portions of 0.33 mg/0.3 ml (0.0022 moles). About 0.3 mg of a moiety of the present invention was dissolved in about 0.4 ml DMF so as to obtain 0.022 moles of moiety in 15 I DMF.

0.022 moles of the compound of the present invention was mixed with 0.0022 moles of IgG in a plastic microcentrifuge tube. After 15 minutes, an additional 0.022 moles of compound was added to the microcentrifuge tube (compound to protein molar ratio was 20:1). After an additional 15 minutes, excess amounts of the compound of the present invention were quenched with lysine HCl solution (10 1 in 100 mM p_{*i*} buffer, pH 8.0) for 15 minutes.

Alternatively, aliquots of 0.0055 moles of the compound of the present invention was used instead of 0.022 moles (compound to protein molar ratio was 5:1).

Biorad glass columns (1 cm x 50 cm) (commercially available from Biorad, Chemical Division, Richmond, California) were packed with previously swelled and de-aerated Sephadex® G-50-80 in phosphate buffer (100 mM, pH 6.3, 150 mM NaCl. 0.001% TMS) to a bed volume of 45 ml. The reaction solution was run through the columns at a flow rate of 0.3-0.4 ml/min. 0.5 ml fractions were collected. Labelled protein fractions were detected by diluting 20 I from each fraction to 1 ml and determining the chemiluminescence produced with 30 I of the diluted solution. Labelled fractions were then pooled.

The pooled conjugate fractions were dialyzed to improve the purity of immunoreactive conjugate. The pooled fractions were dialyzed against 500 ml pH 6.3 phosphate buffer (100 mM, pH 6.3, 150 mM NaCl, 0.001% TMS) over a period of 24 hours with three buffer changes.

### Example 15

Moieties containing an unreduced heterocyclic ring or ring system can be converted to their equivalent reduced forms while such unreduced moieties are attached to protein or other material. This can be accomplished by using a reducing agent such as sodium cyanoborohydride. The procedure for reduction of an acridinium/IgG conjugate is described below. The same procedure is applicable to the reduction of other conjugates.

The IgG labelled with a representative acridinium (100 g) in phosphate buffer (400 l) (pH 6.0, 100 mM, 150 mM. NaCl, 0.001 % Thimerosal) was treated with a freshly prepared solution (10 containing sodium cyanoborohydride (10⁻⁷ moles). After two hours of incubation at room temperature the conversion of the acridinium label on the antibody to the acridan is complete as seen from the UV-Vis spectra indicating appearance of a band at 280 nm and disappearance of the band at 360 nm. This reduced forms retained all their immunological properties.

### ASSAY PROTOCOLS

### Example 16

### 1. Components

A) Labelled Antibody (conjugate): Affinity purified rabbit anti-prolactin conjugated to (2,6-dimethoxy-3-chlorosulfonyl)phenyl-N-methyl-acridinium-9-carboxylate fluorosulfonate. Storage buffer: 10 mM phosphate buffer, 100 mM NaCl pH 6.0, 0.001% Thimerosal, 0.4% BSA.
B) Capture antibody: Rabbit anti-prolactin (6 g/ml) as a solid phase on Nunc tubes (commercially available from Midland Scientific, Roseville, Minnesota).
C) Solid-phase coated tubes: Dried Nunc tubes were prepared as follows:
   1) 0.3 ml of the capture antibody per tube at 6 g/ml in PBS buffer (phosphate buffer saline, pH 7.2-7.4, 10 mM phosphate, 100 mM NaCl, 10 mM NaN₃) was pipetted into Nunc tubes.
   2) Tubes were incubated for 18-24 hours.
   3) Tubes were washed 2 times with the PBS buffer.
   4) Tubes were blocked with 2.0% BSA in PBS buffer. Incubate for ≤ 4 hours at room temperature.
   5) Tubes were washed 3 times with PBS buffer.
   6) Tubes were dried at room temperature.
   7) Tubes were stored in plastic freezer bags at 4° C.
D) Standards: Prepared in horse serum 0, 5, 30, 100 and 200 ng/ml/ml

### 2. Assay Protocol

1) 25 I of sample or standard was pipetted into the antibody-coated tubes.
2) 100 I of labelled antibody was added.
3) Tubes were vortexed gently.
4) Tubes were incubated for 1 hour at room temperature on a rotator.
5) Tubes were washed 3 times with deionized water.
6) Chemiluminescence was counted for 2 seconds [pump 1: 0.1 N HNO₃ + 0.25% H₂O₂ ; pump 2: 0.25 N NaOH + 0.125% CTAC] in a LumaTag™ Analyzer (commercially available from London Diagnostics, Eden Prairie, Minnesota).

### Example 17

### 1. Components

A) Progesterone Conjugate of the β-D-thioglucose adduct of (2,6-dimethoxy-3-chlorosulfonyl)phenyl-N-methyl-acridinium-9-carboxylate fluorosulfonate: 20 pg/ml progesterone conjugate in phosphate buffer (pH 6.0, 100 mM phosphate, 150 mM NaCl, 0.1% human serum albumin, 0.001% Thimerosal).
B) Primary antibody: Rabbit anti-progesterone (Cambridge Medical Diagnostics) in phosphate buffer (pH 6.0, 200 mM phosphate, 150 mM NaCl, 0.1% human serum albumin, 0.01% CHAPS, 5 g Danazol).
C) Solid-phase coated tubes: Dried Nunc tubes coated with 2.5 g of Goat anti-Rabbit fc and blocked with 0.5% BSA. Tubes were prepared as follows:
   1) Tubes were ncubated for 1 hour with 2.5 g/ml Goat anti-Rabbit fc (500 I) at room temperature.
   2) Tubes were washed 3 times with distilled water.
   3) Tubes were immediately incubated for 3 hours with 0.5% BSA (500 I) at room temperature.
   4) Tubes were washed 3 times with distilled water.
   5) Tubes were dried overnight at 40% relative humidity at room temperature.
   6) Tubes were stored in plastic freezer bags at 4° C.
D) Serum matrix: Antech steer serum.
E) Standards: 0, 0.13, 0.38, 1.31, 7.31 16.6 and 37.0 ng/ml.

### 2. Assay Protocol

1) 50 I of sample or standard was pipetted into the antibody-coated tubes.
2) 100 I of conjugate buffer was added.
3) 100 I of primary antibody buffer was added.
4) Tubes were vortexed gently.
5) Tubes were incubated for 2 hours at 37°C.
6) Tubes were decanted and washed with 150 mM NaCl in 0.1% Tween (1 ml) and then 3 times with distilled water.
7) Tubes were inverted and allowed to drain.
8) Chemiluminescence was counted for 2 seconds [pump 1: 0.1 N HNO₃ + 0.25% H₂O₂ ; pump 2: 0.25 N NaOH + 0.125% CTAC] in a LumaTag™ Analyzer (commercially available from London Diagnostics, Eden Prairie, Minnesota).

### Example 18

### 1. Components

A) Labelled Ab: Affinity purified goat anti-TSH conjugated to (2,6-dimethoxy-3-chlorosulfonyl)phenyl-N-methyl-acridinium-9-carboxylate fluorosulfonate.
B) Storage buffer: 100 mM phosphate, 0.145 M NaCl, 0.001% Thimerosal, 0.4% BSA, 0.1 mg/ml mouse-globulins, and 0.1 mg/ml goat-globulins, pH 6.0.
C) Capture antibody: Monoclonal-anti-TSH (2 g/ml) as a solid phase on Nunc tubes. Procedure for preparation of solid-phase Nunc tubes:
   1) 0.4 ml of the capture antibody at 2 g/ml in PBS buffer (phosphate buffer saline, pH 7.2-7.4, 10 mM phosphate, 100 mM NaCl, 10 mM NaN₃) was added in each tube.
   2) Tubes were incubated for 18-24 hours.
   3) Tubes were washed 3 times with the PBS buffer.
   4) Tubes were blocked with 2.0% BSA in PBS buffer and incubated for ≤ 4 hours at room temperature.
   5) Tubes were washed 3 times with PBS buffer.
   6) Tubes were dried at room temperature.
   7) Tubes were stored in plastic freezer bags at 4° C.
D) Standards: Prepared in horse serum.0, 0.5, 2.5, 10, 25 and 100 IU/ml

### 2. Assay Protocol

1) 200 I of sample was pipetted into the coated tubes.
2) 100 I of labelled antibody was added.
3) Tubes were vortexed gently.
4) Tubes were incubated for 2 hours at room temperature on a shaker.
5) Tubes were washed using a Biotomic washer (commercially available from Ocean Scientific, Inc., Garden Grove, Claifornia).
6) Chemiluminescence was counted for 2 seconds [pump 1: 0.1 N HNO₃ + 0.25% H₂O₂ ; pump 2: 0.25 N NaOH + 0.125% CTAC] in a LumaTag™ Analyzer (commercially available from London Diagnostics, Eden Prairie, Minnesota).

### Example 19

### 1. Components

A) Labelled Ab: Affinity purified rabbit anti-prolactin conjugated to (2,6-dimethyl-4-nitro )phenyl-N-methyl-acridinium-9-carboxylate fluorosulfonate. Storage buffer: 10 mM phosphate buffer, 100 mM NaCl pH 6.0, 0.001% Thimerosal, 0.4% BSA.
B) Capture antibody: Rabbit anti-prolactin (6 g/ml) as a solid phase on Nunc tubes.
C) Solid-phase coated tubes: Dried Nunc tubes were prepared as follows:
   1) 0.3 ml of the capture antibody per tube at 6 g/ml in PBS buffer (phosphate buffer saline, pH 7.2-7.4, 10 mM phosphate, 100 mM NaCl, 10 mM NaN₃) was pipetted into Nunc tubes.
   2) Tubes were incubated for 18-24 hours.
   3) Tubes were washed 2 times with PBS buffer.
   4) Tubes were blocked with 2.0% BSA in PBS buffer. Incubate for ≤ 4 hours at room temperature.
   5) Tubes were washed 3 times with PBS buffer.
   6) Tubes were dried at room temperature.
   7) Tubes were stored in plastic freezer bags at 4° C.
D) Standards: Prepared in horse serum 0, 5, 30, 100 and 200 ng/ml/ml

### 2. Assay Protocol

1) 25 I of sample or standard was pipetted into the antibody-coated tubes.
2) 100 I of labelled antibody was added.
3) Tubes were vortexed gently.
4) Tubes were incubated for 1 hour at room temperature on a rotator.
5) Tubes were washed 3 times with deionized water.
6) Chemiluminescence was counted for 2 seconds [pump 1: 0.1 N HNO₃ + 0.25% H₂O₂ ; pump 2: 0.25 N NaOH + 0.125% CTAC] in a LumaTag™ Analyzer (commercially available from London Diagnostics, Eden Prairie, Minnesota).

### STABILITY STUDIES

In accordance with the following stability studies, the moieties and conjugates of the present invention are preferably stored and used in assays at a pH between about 6.5 and about 7.5. The moieties and conjugates exhibit increased stability at other pHs under certain conditions; however, the increase is not condition-dependant within the preferred pH range.

### Example 20

Comparative stability was determined by comparing the number of days (t_{1/2})in which a moiety lost 50% of its chemiluminescence. Stability of certain moieties not linked to protein or other material was monitored at room temperature and at 4°C. Moieties were stored in buffer (pH 6.0, 50 mM p_{*j*}, 0.1% BSA). (2,6-dimethyl-4-nitro)phenyl-3-(3-succinimidyloxycarbonyl) propyloxy-N-methyl-acridinium-9-carboxylate fluorosulfonate showed no appreciable loss of counts after 98 days at both room temperature and at 4°C. (2,6-isopropyl-4-nitro)phenyl-3-(3-succinimidyl-oxycarbonyl) propyloxy-N-methyl-acridinium-9-carboxylate fluorosuifonate showed no appreciable loss of counts after 312 days at room temperature and 4°C. For (2,6-dimethyl-4-trimethylammonio)phenyl-N-methyl-acridinium-9-carboxylate difluorosulfonate t_{1/2} was 105 days at room temperature and t_{1/2} was not reached after 105 days at 4°C. For (2,6-dimethoxy-3-chlorosulfonyl)phenyl-N-methyl-acridinium-9-carboxylate fluorosulfonate, t_{1/2} was 50 days at room temperature. t_{1/2} at 4°C was not reached after 130 days. For (2.6-dimethoxy-3-chlorosulfonyl-4-propanoic)phenyl-N-methyl-acridinium-9-carboxylate fluorosulfonate, t_{1/2} was 24 days at room temperature and > 95 days at 4°C. For (2,6-dimethyl) phenyl-N-methyl-acridinium-9-carboxylate fluorosulfonate, t_{1/2} at room temperature was 50 days. In comparison, moieties not having the substitutions of moieties of the present invention had an average t_{1/2} less than 8 days at room temperature and approximately 32 days at 4°C.

### Example 21

The comparative stability of the phenyl-, (2.6-dimethyl)phenyl- and (2,6-dimethoxy-3-chlorosulfonyl)phenyl-, (2,6-dimethyl-3-chlorosulfonyl)phenyl-, (2,6-dimethyl-4-nitro)phenyl-, (2,6-dimethyl-4-trimethylammonio)phenyl-, and (2,6-dimethyl-4-bromo)phenyl- N-methyl-acridinium esters was observed in phosphate buffer (100 mM p_{*j*}, 150 mM NaCl, 0.001% Thimerosal, 0.005% human serum albumin) at pH 6.3 and pH 8.0 while incubated at 35°C and 45°C.

All of the substituted phenyl compounds, with the exception that the (2,6-dimethyl)phenyl- compound, showed increased stability in comparison with the naked compound. The (2,6-dimethyl)phenyl- compound, was less stable than the naked phenyl compound at pH 6.3 at 35°C, but showed increased stability at pH 8.0 at 35°C and at both pHs at 45 C.

### Example 22

The stability of (2,6-dimethyl-4-nitro)phenyl-3-(3 -succinimidyl-oxycarbonyl) propyloxy-N-methyl-acridinium-9-carboxylate fluorosulfonate and (2,6-dimethoxy-3-chlorosulfonyl)phenyl-N-methylacridinium-9-carboxylate fluorosulfonate mouse IgG conjugates at various temperatures and pHs was studied and compared to the stability of 4-(2-succinimidylcarboxyethyl)-phenyl-N-methyl acridinium-9-carboxylate fluorosulfonate IgG conjugates. 4-(2-succinimidylcarboxyethyl)-phenyl-N-methyl acridinium-9-carboxylate fluorosulfonate has the following formula: 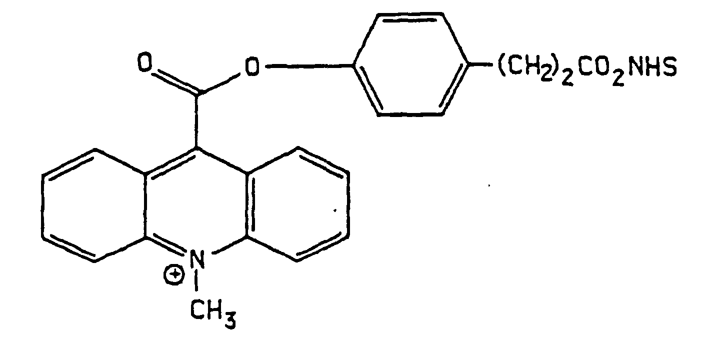 4-(2-succinimidylcarboxyethyl)-phenyl-N-methyl acridinium-9-carboxylate fluorosulfonate is different from the compounds of the present invention *in* that it does not have an electron withdrawing group or 2,6-substitutions on the phenyl ring. 4-(2-succinimidylcarboxyethyl)-phenyl-N-methyl acridinium-9-carboxylate fluorosulfonate has been published in the literature and is widely used in chemiluminescent applications.

As presented below, loss of counts in a certain conjugate results from the hydrolysis of the ester linkage in the chemiluminescent label. Therefore, as the stability of the labelling compound decreases, the rate of loss of counts in the conjugate decreases.

Mouse IgG conjugates of (2,6-dimethyl-4-nitro)phenyl-3-(3-succinimidyl-oxycarbonyl) propyloxy-N-methyl-acridinium-9-carboxylate fluorosulfonate, (2,6-dimethoxy-3-chlorosulfonyl)phenyl-N-methyl-acridinium-9-carboxylate fluorosulfonate and 4-(2-succinimidylcarboxyethyl)-phenyl-N-methyl acridinium-9-carboxylate fluorosulfonate were stored at 4°C. The conjugates were stored in three different buffer solutions at pH 6.3, 7.3 and 8.0.

The first buffer contained 100 mM p_{*i*}, 150 mM NaCl, 0.001% Thimerosal, 0.1% human serum albumin, 20 mg/L sheep IgG ("Standard Phosphate Buffer").

The second buffer was Standard Phosphate Buffer without sheep IgG.

The third buffer was Standard Phosphate Buffer without sheep IgG and with 0.1% bovine serum albumin instead of human serum albumin.

In each buffer at each pH (2,6-dimethyl-4-nitro)phenyl-3-(3-succinimidyl-oxycarbonyl)propyloxy-N-methyl-acridinium-9-carboxylate fluorosulfonate and (2,6-dimethoxy-3-chlorosulfonyl)phenyl-N-methyl-acridinium-9-carboxylate fluorosulfonate IgG conjugates exhibited increased stability when compared with 4-(2-succinimidylcarboxyethyl)-phenyl-N-methyl acridinium-9-carboxylate fluorosulfonate IgG conjugate.

Mouse IgG conjugates of (2,6-dimethyl-4-nitro)phenyl-3-(3-succinimidyl-oxycarbonyl) propyloxy-N-methyl-acridinium-9-carboxylate fluorosulfonate, (2,6-dimethoxy-3-chlorosulfonyl)phenyl-N-methyl-acridinium-9-carboxylate fluorosulfonate and 4-(2-succinimidylcarboxyethyl)-phenyl-N-methyl acridinium-9-carboxylate fluorosulfonate were stored at room temperature (about 23°C) ("RT") and 37°C. The conjugates were stored in Standard Phosphate Buffer at pH 6.3, 7.3 and 8.0.

At each pH and at room temperature and 37°C (2,6-dimethyl-4-nitro)phenyl-3-(3-succinimidyl-oxycarbonyl) propyloxy-N-methyl-acridinium-9-carboxylate fluorosulfonate and (2,6-dimethoxy-3-chlorosulfonyl)phenyl-N-methyl-acridinium-9-carboxylate fluorosulfonate IgG conjugates exhibited increased stability when compared with 4-(2-succinimidylcarboxyethyl)-phenyl-N-methyl acridinium-9-carboxylate fluorosulfonate IgG conjugate.

(2,6-dimethyl-4-nitro)phenyl-3-(3-succinimidyl-oxycarbonyl) propyloxy-N-methyl-acridinium-9-carboxylate fluorosulfonate, (2,6-dimethoxy-3-chlorosulfonyl)phenyl-N-methyl-acridinium-9-carboxylate fluorosulfonate and 4-(2-succinimidylcarboxyethyl)-phenyl-N-methyl acridinium-9-carboxylate fluorosulfonate IgG conjugates were stored at 37°C in azide buffer (50 mM p_{*j*}, 100 mM NaCl, 0.1% bovine serum albumin, 10 mM sodium azide) at pH 6.9, 7.0, 7.3 and 8.0.

At pHs above 7.3 both (2,6-dimethyl-4-nitro)phenyl-3-(3-succinimidyl-oxycarbonyl)propyloxy-N-methyl-acridinium-9-carboxylate fluorosulfonate and (2,6-dimethoxy-3-chlorosulfonyl)phenyl-N-methyl-acridinium-9-carboxylate fluorosulfonate IgG conjugates exhibited increased stability when compared with 4-(2-succinimidylcarboxyethyl)-phenyl-N-methyl acridinium-9-carboxylate fluorosulfonate IgG conjugate. At pH 6.9 and 7.0 only (2,6-dimethyl-4-nitro)phenyl-3-(3-succinimidyl-oxycarbonyl) propyloxy-N-methyl-acridinium-9-carboxylate fluorosulfonate IgG conjugate exhibited increased stability. (2,6-dimethoxy-3-chlorosulfonyl)phenyl-N-methyl-acridinium-9-carboxylate fluorosulfonate IgG conjugate was not significantly more stable than the 4-(2-succinimidylcarboxyethyl)-phenyl-N-methyl acridinium-9-carboxylate fluorosulfonate IgG conjugate at pH 6.9 and 7.0.

(2,6-dimethyl-4-nitro)phenyl-3-(3-succinimidyl-oxycarbonyl) propyloxy-N-methyl-acridinium-9-carboxylate fluorosulfonate, (2,6-dimethoxy-3-chlorosulfonyl)phenyl-N-methyl-acridinium-9-carboxylate fluorosulfonate and 4-(2-succinimidylcarboxyethyl)-phenyl-N-methyl acridinium-9-carboxylate fluorosulfonate IgG conjugates were stored at 37°C in azide buffer at pH 6.3.

(2,6-dimethyl-4-nitro)phenyl-3-(3-succinimidyl-oxycarbonyl) propyloxy-N-methyl-acridinium-9-carboxylate fluorosulfonate and 4-(2-succinimidylcarboxyethyl)-phenyl-N-methyl acridinium-9-carboxylate fluorosulfonate IgG conjugates were stored at room temperature and 4°C in azide buffer at pH 5.9.

At pHs less than 6.3 at 37°C both (2,6-dimethyl-4-nitro)phenyl-3-(3-succinimidyl-oxycarbonyl) propyloxy-N-methyl-acridinium-9-carboxylate fiuorosulfonate and (2,6-dimethoxy-3-chlorosulfonyl)phenyl-N-methyl-acridinium-9-carboxylate fluorosulfonate IgG conjugates exhibited decreased stability when compared with 4-(2-succinimidylcarboxyethyl)-phenyl-N-methyl acridinium-9-carboxylate fluorosulfonate IgG conjugate. At pH 5.9 at room temperature and 4°C (2,6-dimethyl-4-nitro)phenyl-3-(3-succinimidyi-oxycarbonyl)propyloxy-N-methy(-acridinium-9-carboxylate fluorosulfonate IgG conjugate exhibited decreased stability when compared with 4-(2-succinimidylcarboxyethyl)-phenyl-N-methyl acridinium-9-carboxylate fluorosulfonate IgG conjugate.

### Example 23

The comparative stability of the phenyl-, (2,6-dimethyl)phenyl- and (2,6-dimethyl-4-nitro)phenyl- N-methyl-acridan esters was observed in phosphate buffer (100 mM p_{*j*}, 150 mM NaCl, 0.001% Thimerosal, 0.005% human serum albumin) at pH 6.3 and pH 8.0 while incubated at 35°C and 45°C.

The (2,6-dimethyl)phenyl- and (2,6-dimethyl-4-nitro)phenyl- compounds showed increased stability in comparison with the naked phenyl compound.

### ADDITIONAL CHEMILUMINESCENT MOIETIES

### Example 24

A preferred chemiluminescent moiety of the present invention having an RnX group on the carbon to which the ester linkage is attached is (2,6-dimethoxy-3-chlorosulfonyl)phenyl-N-methyl-acridan-9-ethoxy-9-carboxylate, which has the following formula: 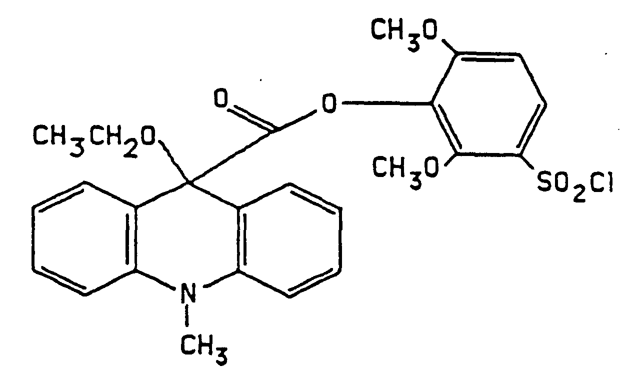 (2,6-dimethoxy-3-chlorosulfonyl)phenyl-N-methyl-acridan-9-ethoxy-9-carboxylate is synthesized from (2,6-dimethoxy-3-chlorosulfonyl)phenyl-N-methyl-acridinium-9-carboxylate by two alternative methods described below.

### 1. High Performance Liquid Chromatography (HPLC)

A C₁₈ column (Rainin, Dynamax 60A, 250mm x 10mm) was equilibrated with a mixture of ethanol and acetonitrile (up to 10%) containing about 0.05% of a tertiary amine (e.g., triethylamine). (2,6-dimethoxy-3-chlorosulfonyl) phenyl-N-methyl-acridinium-9-carboxylate was dissolved in the mobile phase and injected onto the column. The major fraction, eluting with a maximum absorbance at 280nm, was collected at a flow rate of 2.0-2.5 ml/min. The solvent was immediately completely removed under vacuum. The residue was then treated with a small amount of benzene to remove traces of alcohol and to remove moisture from the final product, (2,6-dimethoxy-3-chlorosulfonyl)phenyl-N-methyl-acridan-9-ethoxy-9-carboxylate. This HPLC method is the preferred method of synthesis, since it yields a much purer product. However, the HPLC method may not be appropriate for certain nucleophiles. Where the HPLC method does not work, the nucleophilic anion method described below can be used.

### 2. Treatment of Starting Compound with Nucleophilic Anion

(2,6-dimethoxy-3-chlorosulfonyl)phenyl-N-methyl-acridinium-9-carboxylate (0.013 mmole, 7.5 mg) was dissolved in absolute ethanol (3 ml) under nitrogen. A solution of potassium-t-butoxide in absolute ethanol (2 mg/ml) was added dropwise (using a gas tight syringe) to the vigorously stirred (2,6-dimethoxy-3-chlorosulfonyl)phenyl-N-methyl-acridinium-9-carboxylate solution until the yellow color of the solution was completely discharged. The ethanol was then completely removed under vacuum. The residue was treated with anhydrous ether (2ml) and approximately 1g anhydrous sodium sulfate. Separation of the ether solution and evaporation of the solvent yielded (2,6-dimethoxy-3-chlorosulfonyl)phenyl-N-methyl-acridan-9-ethoxy-9-carboxylate as a white solid (2.5 mg).

(2,6-dimethoxy-3-chlorosulfonyl)phenyl-N-methyl-acridan-9-methoxy-9-carboxylate has been produced using both the HPLC and nucleophilic anion synthetic procedures from (2,6-dimethoxy-3-chlorosulfonyl)phenyl-N-methyl-acridinium-9-carboxylate. (2,6-dimethyl-3-chlorosulfonyl)phenyl-N-methyl-acridan-9-methoxy-9-carboxylate has been produced using the nucleophilic anion synthetic method from (2,6-dimethyl-3-chlorosulfonyl)phenyl-N-methyl-acridinium-9-carboxylate.

### 3. Other Syntheses

Both synthetic procedures described above can be used to produce any of the compounds of the present invention having an RₙX group. If a nucleophile other than ethanol is to donate the RₙX group, the desired nucleophile is substituted for ethanol in the synthetic procedures. If a different acridinium, phenanthridinium, etc. is desired, the compound to which the RₙX group is to be added can be substituted for (2,6-dimethoxy-3-chlorosulfonyl)phenyl -N-methyl-acridinium-9-carboxylate in the synthetic procedures described above. In many instances, a change in the nucleophile will also require a change in solvent (e.g., if methanol is the desired nucleophile, ethanol cannot be used as a solvent because of competition for addition). In such instances, the new nucleophile can be used as a replacement solvent, if appropriate, or a non-nucleophilic, non-protic solvent (e.g., THF) can be substituted.

Other synthetic methods, including without limitation treatment with a nucleophilic solvent, can also be used to produce moieties having an RnX group.

### Example 25

The operability of the synthetic procedures described in Example 24, was confirmed by analysis of the products by UV-Vis spectroscopy, fast atom bombardment mass spectoscopy and 300 MHz proton NMR.

### Example 26

(2,6-dimethoxy-3-chlorosulfonyl)phenyl-N-methyl-acridan-9-ethoxy-9-carboxylate was used as a label in an assay for TSH as follows:

### 1. Components

A) Labelled Ab: Affinity purified goatanti-TSH was conjugated to (2,6-dimethoxy-3-chlorosulfonyl)phenyl-N-methyl-acridan-9-ethoxy-9-carboxylate as follows: a solution of the anti-TSH antibody (approximately 100ug) in bicarbonate buffer (0.1 M, pH 9.6) was treated with 25 moles excess of (2,6-dimethoxy-3-chlorosulfonyl)phenyl-N-methyl-acridan-9-ethoxy-9-carboxylate as a solution in DMF. The reaction mixture was purified on a fast flow Sephadex® G25 (superfine) column (Pharmacia) using an HPLC system. The protein peak was collected at a flow rate of approximately 0.75 ml/min with a mobile phase of phosphate buffer (pH 6.0) containing approximately 20% ethanol. After buffer exchange the labelled antibody preparation was diluted with storage buffer to provide approximately 100,000 counts/100 ul in a LumaTag™ Analyzer (commercially available from London Diagnostics, Eden Prairie, Minnesota) after 1:10 dilution.
B) Storage buffer: 100 mM phosphate, 0.145 M NaCl, 0.001% Thimerosal, 0.4% BSA, 0.1 mg/ml mouse-globulins, and 0.1 mg/ml goat-globulins, pH 6.0.
C) Capture antibody: Monoclonal-anti-TSH (2 ug/ml) as a solid phase on Nunc tubes. Procedure for preparation of solid-phase Nunc tubes:
   1) 0.4 ml of the capture antibody at 2 ug/ml in PBS buffer (phosphate buffer saline, pH 7.2-7.4, 10 mM phosphate, 100 mM NaCl, 10 mM NaN₃) was added to each tube. 2. Tubes were incubated for 18-24 hours.
   3) Tubes were washed 3 times with the PBS buffer.
   4) Tubes were blocked with 2.0% BSA in PBS buffer and incubated for < 4 hours at room temperature.
   5) Tubes were washed 3 times with PBS buffer.
   6) Tubes were dried at room temperature.
   7) Tubes were stored in plastic freezer bags at 4°C.
D) Standards: Prepared in horse serum 0, 0.05, 0.1, 0.5, 2.5, 10, 25 and 50 ulU/ml
E) Wash Solution: saline buffer containing BSA

### 2. Assay Protocol

1) 200 ul of sample was pipetted into the coated tubes.
2) 100 ul of labelled antibody was added.
3) Tubes were vortexed gently.
4) Tubes were incubated for 2 hours at room temperature on a shaker.
5) 1ml Wash Solution was added to each tube.
6) Tubes were washed using a Biotomic washer (commercially available from Ocean Scientific, Inc., Garden Grove, California).
7) Chemiluminescence was counted for 2 seconds [pump 1: 0.1 N HNO₃ + 0.25% H₂O₂ ; pump 2: 0.25 N NaOH + 0.125% CTACI in a LumaTag™ Analyzer (commercially available from London Diagnostics, Eden Prairie, Minnesota).
Addition of HNO₃ to the assay mixture containing the labelled antibody causes the C9 ethoxy group to cleave from the acridinium molecule before the chemiluminescent reaction is triggered by the addition of NaOH.

### Example 27

The comparative stability of (2,6-dimethoxy-3-chlorosulfonyl)phenyl-N-methyl-acridinium-9-carboxylate ("DMC") and (2,6-dimethoxy-3-chlorosulfonyl)phenyl-N-methyl-acridan-9-ethoxy-9-carboxylate ("DME") was compared at pH 9.6 at 25°C. The compounds were dissolved in DMF (Approximately 0.5 mg/ml). 10ul of the solution was then added to 1ml bicarbonate buffer (0.1M bicarbonate, 0.00025% Thimerosal, 0.1% bovine serum albumin). The solution was diluted further to provide approximately 300,000 counts in 10ul. The data from the stability study is shown in Fig. 36. DME was shown to be more stable than DMC over time.

The comparative stability of DMC-labelled TSH conjugate and DME-labelled TSH conjugate was compared at pH 6.0 at 25, 30 and 35°C. The DME-labelled TSH conjugate was shown to be more stable over time.

The present embodiments are to be considered in all respects as illustrative and not restrictive, the scope of the invention being indicated by the appended claims.

## Claims

1. A chemiluminescent compound having the general formula I: 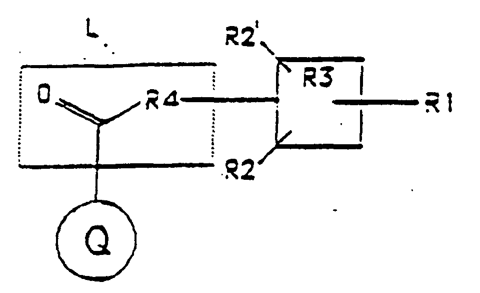 wherein:
R4 is such that the dashed box labelled L contains an ester, thioester or amide linkage;
Q represents a heterocyclic ring or ring system containing a carbon atom to which the linkage L is attached, wherein the heteroatom within the ring or ring system is in, or is capable of being in, an oxidation state which renders the carbon atom susceptible to attack by peroxide or motecular oxygen to form an intermediate which decays to produce chemiluminescence;
R3 represents an aryl ring or ring system containing at least one six-membered ring;
each of R2 and R2' individual represent groups which sterically hinder the hydrolysis of the linkage L;
R1 represents an electron withdrawing group which is -NO₂, -SO₂Cl, -Br or -N(CH₃)₃^{⊕};
one of R1, R2 and R2' may also represent a group capable of attaching the compound to a protein or nucleic acid; or a salt thereof.

2. A compound as claimed in claim 1 wherein each of R2 and R2' individually represent the group -R, -OR or -SR where R represents an aryl group.

3. A compound as claimed in claim 1 or 2 wherein R2 and R2' are in the ortho-positions on R3 when R3 is a phenyl ring with the ester linkage being attached at position 1.

4. A compound as claimed in any preceding claim wherein Q represents an acridinium, benz[a,b or c]acridinium, 1,2,4-triazole cation, isooxazole cation, isoihioazole cation, 1,2-azole cation, imidazole cation, beozimidazole cation, quinolinium, isoquinolinium, quinolizinium, a cyclic C3, C4 or C5-substituted quinolinium, pyridinium, pyrimidinium, pyridazinium, pyrazinium, phenanthridinium or quinoxalinium group, or a reduced form thereof.

5. A chemiluminescent compound having the general formula I: 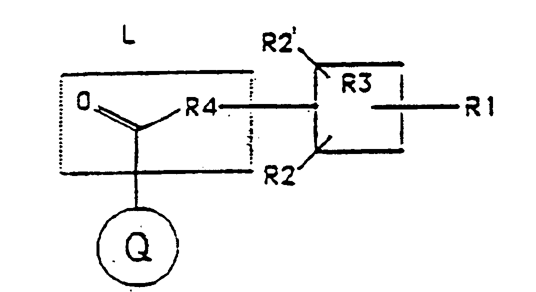 wherein:
R4 is such that the dashed box labelled L contains an ester, thioester or amide linkage;
Q represents a heterocyclic ring or ring system containing a carbon atom to which the linkage L is attached, wherein the heteroatom within the ring or ring system is in an oxidation state which renders the carbon atom susceptible to attack by peroxide or molecular oxygen to form an intermediate which decays to produce chemiluminescence, wherein the ring system is acridinium, benz[a, b or c]acridinium, a benzimidazole cation, quinolinium, isoquinolinium, quinolizinium, a cyclic C3, C4 or C5-substituted quinolinium, phenanthridinium or quinoxalinium; or a reduced form of such ring systems;
Q additional being substituted with one or more peri substituents each of which is located on carbon atom within the ring system Q which is capable of accepting a substituent and is nearest to the carbon atom to which the ester, thioester or amide linkage L is attached and which cause steric hinderance to that carbon atom;
R3 represents an aryl ring or ring system containing at least one six-membered ring;
R2 and R2' represent substituents that along with the peri substituent(s) sterically hinder hydrolysis of the linkage L;
R1 represents an electron withdrawing group having a σₚ value above 0.0 and wherein the additive σₚ value for R1, R2 and R2', wherein R2 and/or R2' are electron with drawing groups, is less than or equal to 1.0; and one of R1, R2 and R2' may also represent a group capable of attaching the compound to a protein or nucleic acid; or a salt thereof.

6. A compound as claimed in claim 5 wherein each peri substituent individually represents a methyl, ethyl or phenyl group.

7. A compound as claimed in claim 5 or 6 wherein Q represents an acridinium, phenanthridinium, quinolixinium or quinolinium group.

8. A compound as claimed in any of claims 5 to 7 wherein the peri substituents are placed in the following positions:
(a) on C1 and C8, where Q represents an acridinium;
(b) on C7 where Q represents a phenanthridinium group; or
(c) on C3 where Q represents a quinolinium group.

9. A compound as claimed in any of claim 5 to 8 wherein at least one peri substituent is -CH₃.

10. A compound as claimed in any of claims 5 to 9 wherein R₃ represents a phenyl group and the sum of all σₚ values for R1, R2 and R2', when they represent an electron withdrawing group, is less than or equal to 1.0.

11. A chemiluminescent compound having the general formula II: 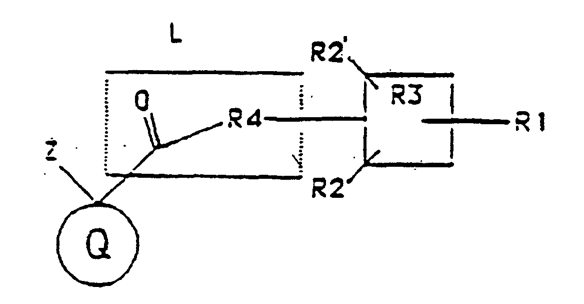 wherein:
R4 is such that the dashed box labelled L contains an ester, thioester or amide linkage;
Q represents a heterocyclic ring or ring system containing a carbon atom to which the linkage L is attached, wherein the heteroalom within the ring or ring system is in, or is capable of being in, an oxidation state which renders the carbon atom susceptible to attack by peroxide or molecular oxygen to form an intermediate which decays to produce chemiluminescence;
R3 represents an aryl ring or ring system containing at least one six-membered ring;
each of R2 and R2' individually represent substituents that sterically hinder hydrolysis of the linkage L;
R1 represents an electron withdrawing group having a σₚ value above 0,0 and wherein the additive σₚ value for R1, R2 and R2', wherein R2 and/or R2' are electron with drawing groups, is less than or equal to 1.0;
one of R1, R2 and R2' may also represent a group capable of attaching the compound to a protein or nucleic acid;
Z represents a substituent attached to the sp³ hybridised carbon atom to which the linkage L is attached and is:
- a hydrogen or halogen atom:
- CN;
- OR;
- NR₂;
- NR^{⊕}₃;
- SR;
- SR^{⊕}₂;
- S₂R;
- NRCO₂R;
- NHNR₂;
- NRNR₂;
- ONR₂;
- NHOR,
- CR(CN)₂;
- CR(COR)₂;
- CR₂NO₂;
- C ≡ COR;
- XRₙ;
- a drug or steroid molecule;
- 2 - oxazole;
- 1 - imidazole;
wherein:
R represents an alkyl group, an aryl group, an amino acid, a sugar or multiple R groups can represent 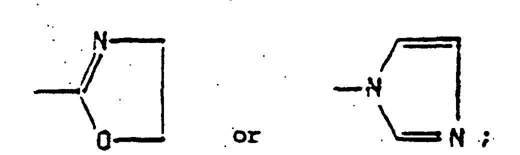
X represents an oxygen, nitrogen, sulphur or carbon atom;
n is an integer of from 1 to 3 whose value is determined by the valency of the atom represented by X; or a salt thereof.

12. A compound as claimed in claim 11 which is (2,6-dimethoxy-3-chlorosulfonyl)phenyl-N-methyl-acridan-9-ethoxy-9-carboxylate.

13. A compound as claimed in any claim 11 or 12 wherein when Z is RₙX it can be cleaved by treating the compound with an acid.

14. A compound as claimed in any preceding claim wherein Q represents an acridinium, benz[a,b or c]acridiniun, 1.2,4-triazole cation, isooxazole cation, isothioazole cation, 1,2-azole cation, imidazole cation, benzimidazole cation, quinolinium, isoquinolinium, quinoliziziun, a cyclic C3, C4 or C5-substitutedquinolinium, pyridinium, pyrimidiniun, pyridazinium, pyraziniun, phenanthridinium or quinoxalinium group, or a reduced form thereof.

15. A chemiluminescent compound having the general formula I: 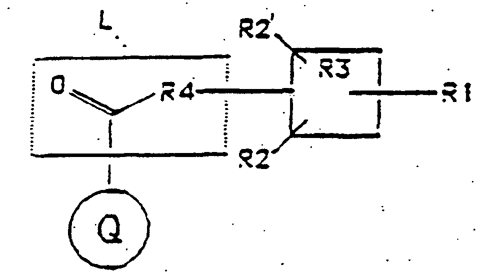 wherein:
R4 is such that the dashed box labelled L contains an ester, thioester or amide linkage;
Q represents a heterocyclic ring or ring system containing a carbon atom to which the linkage L is attached, wherein the heteroatom within the ring or ring system is in, or is capable of being in, an oxidation state which renders the carbon atom susceptible to attack by peroxide or molecular oxygen to form an intermediate which decays to produce chemiluminescence;
Ca additionalty being substituted by a substituant capable of being conjugated with a specific binding material; through an alkyl, alkenyl, alkynyl, alkylamino, oxoalkyl, thioalkyl or an alkyloxycarbonyl group joined to Q, the substitutent having at least one of the following function groups:
- COOR, where R represents an alkyl or aryl group;
- C(OR) = NH^{⊕} ₂, where R represents a residue of a functional alcohol;
- SO₂Cl,
- NCS; 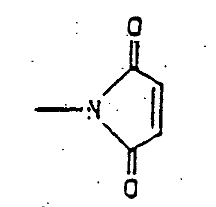 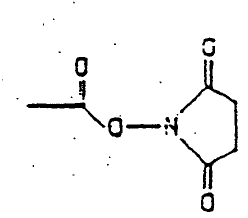 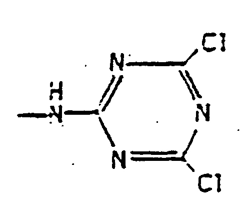
- N(CH₃)(CH₂)ₘCl, where m is at least one;
- N₃;
- NH₂; or
- an ion, sugar, polyamine, polyoxyethylene or polyoxybutylene:
R3 represents an aryl ring or ring system containing at least one six-membered ring;
each of R2 and R2' individually represent substituents that sterically hinder hydrolysis of the linkage L;
R1 represents an electron withdrawing group having a σₚ value above 0.0 and
wherein the additive σₚ value for R1, R2 and R2', where R2 and/or R2' are electron with drawing groups, is less than or equal to 10; or a salt thereof.

16. A compound as claimed in claim 15 wherein the substituent is bound through an oxoalkyl or alkyloxycarbonyl group.

17. A compound as claimed in claim 15 or 16 wherein the functional group is succinimide or N-carboxyl succinimide.

18. A compound as claimed in any of claims 15 to 17 wherein Q represents an acridinium, benz[a,b or c]acridinium, 1,2,4-triazole cation, isooxazole cation, isothioazole cation; 1,2-azole cation, imidazole cation, benzimidazole cation, quinolinium, isoquinolinium, quinolizinium, a cyclic C3, C4 or C5-substituted quinolinium, pyridinium, pyrimidinium, pyridazinium, pyrazinium, phenanthridinium or quinoxalinium group, or a reduced form thereof.

19. A compound as claimed in any preceding claim wherein R4 represents -O- so that L is an ester linkage.

20. A compound as claimed in any preceding claim wherein R3 represents a pheriyl group.

21. A compound as claimed in any preceding claim wherein R1 represents -NO₂, -SO₂Cl, or -N(CH₃)₃^{⊕}.

22. A compound as claimed in any preceding claim wherein R1 represents -SO₂Cl.

23. A compound as claimed in any preceding claim wherein R2 and R2' represent -CH₃, or -OCH₃.

24. A compound as claimed in any preceding claim wherein R₃ represents a phenyl group and the sum of all σₚ values for R1, R2 and R2', when they represent an electron withdrawing group, is less than or equal to 1.0.

25. A compound as claimed in any preceding claim wherein Q represents an acridinium group.

26. A compound according to any of claims 1 to 4, 19, 20 and 23 to 25 which is:

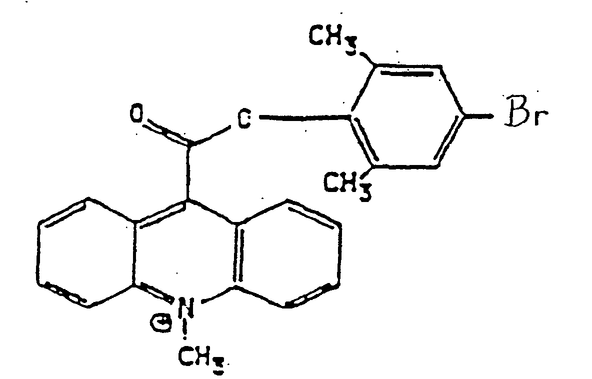

27. A compound according to any of claims 1 to 4 and 19 to 25 which is:

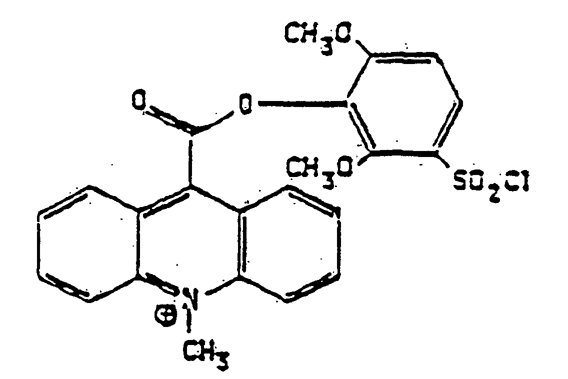

28. A compound according to any of claims 1 to 4 and 19 to 25 which is:

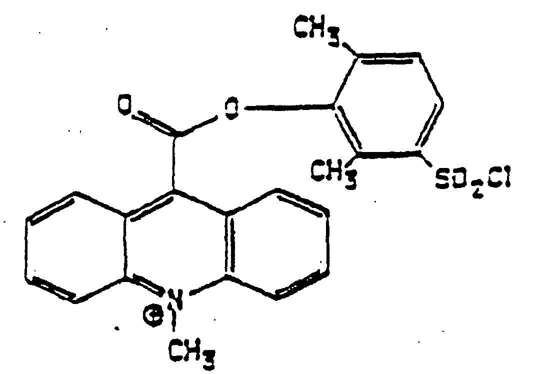

29. A compound according to any of claims 1 to 4, 19 to 21 and 23 to 25 which is:

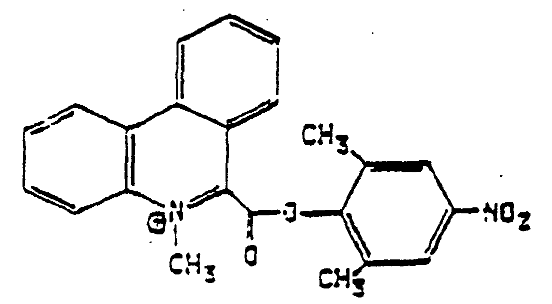

30. A compound according to any of claims 1 to 4, 19 to 21, 23 and 24 which is:

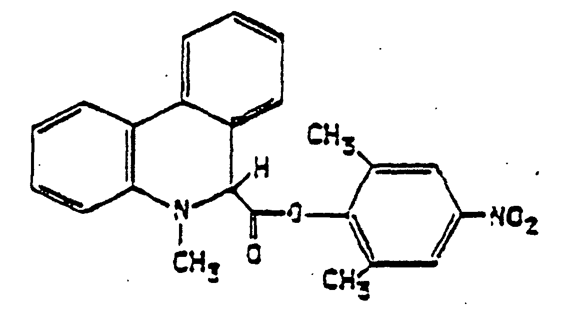

31. A compound according to any of claims 1 to 4 and 19 to 24 which is:

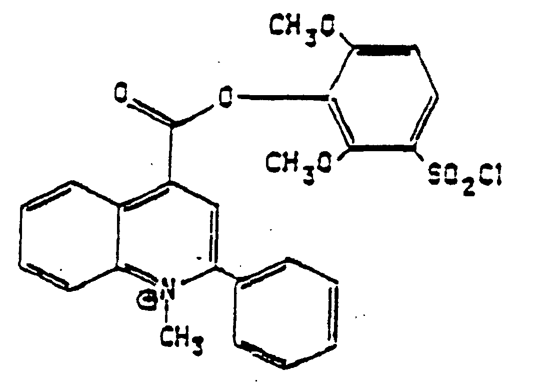

32. A compound according to any of claims 1 to 4 and 19 to 24 which is:

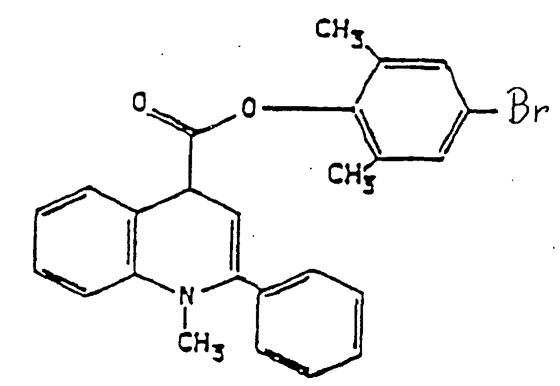

33. A compound according to any of claims 1 to 4, 19, 20, 23 and 24 which is:

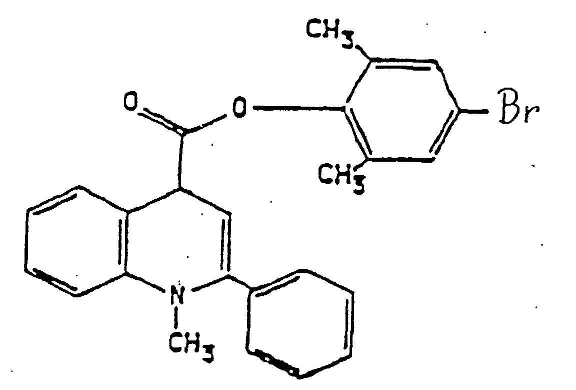

34. A compound as claimed in any of claims 11 to 14 which is (2,6-dimethoxy-3-chtorosulphonyl)phenyl-N-methyl-acridan-9-ethoxy-9-carboxylate, (2,6-dimethoxy-3-chlorosulphonyl)phenyl-N-methyl-acridan-9-methoxy-9-carboxylate or (2,6-dimethyl-3-chlorosulphonyl)phenyl-N-methyl-acridan-9-methoxy-9-carboxylate.

35. A compound according to any of claims 15 to 21 and 23 to 25 which is:

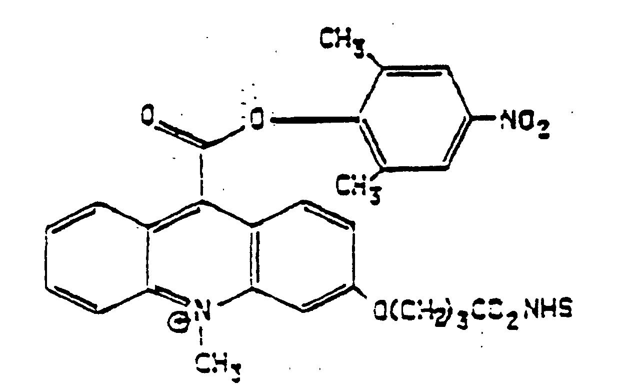

36. A compound according to any of claims 15 to 21 and 23 to 25 which is:

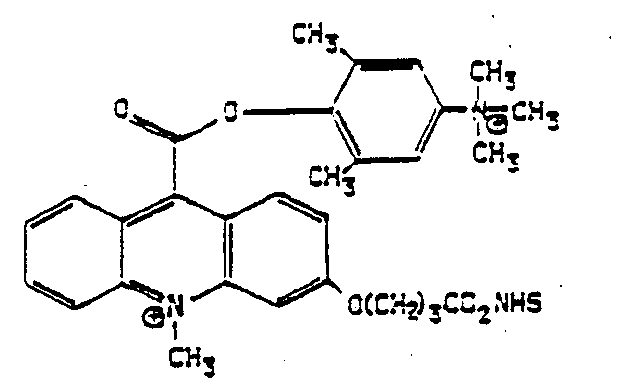

37. A compound according to any of claims 15 to 21 and 23 to 25 which is:

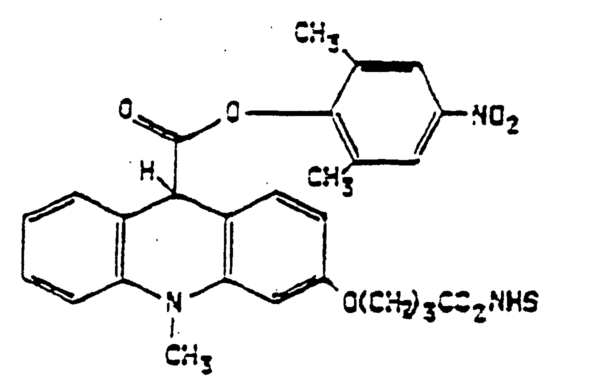

38. A compound according to any of claims 15 to 21 and 23 to 25 which is:

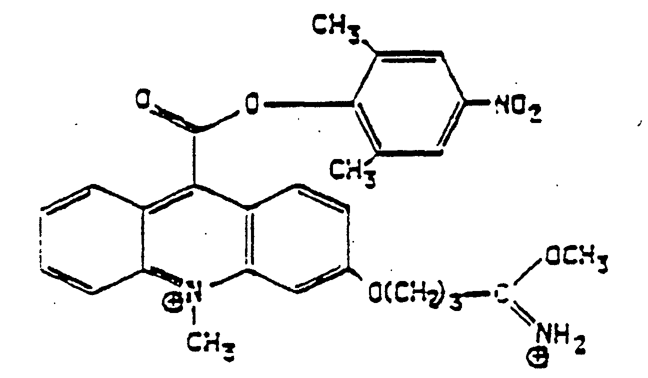

39. A conjugate comprising a chemiluminescent compound according to any of claims 1 to 38 bound to a specific binding partner for a biological, biochemical or chemical species.

40. A conjugate as claimed in claim 39 which can specifically bind by an immunoreaction, protein binding reaction or nucleic hybridisation.

41. A composition comprising a chemiluminescent compound as claimed in any of claims 1 to 38 or a conjugate as claimed in claim 39 or 40.

42. A specific binding assay kit comprising a vial containing a composition according to claim 41.

43. A specific binding assay for detecting the presence of an analyte in a sample wherein the assay utilises a chemiluminescent conjugate according to claim 39 or 40 or a chemiluminescent compound as claimed in any of claims 1 to 38 attached to a specific binding material where the presence of the analyte in the sample is proportional to the formation of one or more specific binding reaction products containing the conjugate, the assay comprising:
allowing under suitable conditions substantial formation of one or more specific binding reaction products containing the conjugate; and
measuring the chemiluminescence of either (i) one or more of the specific binding reaction products or (ii) the conjugate not contained in the binding reaction products.

44. An assay as claimed in claim 43 wherein the specific binding material is capable of specifically binding with the analyte and the specific binding reaction product is an analyte-conjugate complex, the assay comprising:
allowing under suitable conditions substantial formation of the analyte-conjugate complex; and
measuring the chemiluminescence of either (i) the analyte-conjugate complex or (ii) the conjugate not contained in the analyte-conjugate complex.

45. An assay as claimed in claim 44 which is a surface antigen assay and the specific binding material is an antibody which is specific for a surface antigen on a cell.

46. An assay as claimed in claim 44 or 45 which further utilizes a reactant which is capable of specifically binding with both (i) the analyte, to form an analyte-reactant complex, and (ii) with the specific binding material, to form a conjugate-reactant complex which is the specific binding reaction product, the assay comprising:
allowing under suitable conditions substantial formation of the analyte-reactant complex and conjugate-reactant complex: and
measuring the chemiluminescence of either (i) the conjugate-reactant complex, or (ii) the conjugate not contained in the conjugate-reactant complex.

47. An assay as claimed in claim 43 which is a competitive assay.

48. An assay as claimed in claim 46 or 47 wherein the reactant is attached to a solid phase or complexes containing the reactant are precipitated.

49. An assay as claimed in claim 43 which is a sequential saturation assay and the analyte is first reacted with the reactant before reaction of the conjugate with any remaining unreacted reactant.

50. An assay as claimed in claim 43 which is a competitive displacement assay where the conjugate competitively displaces analyte which has already bound to the reactant.

51. An assay as claimed in claim 43 which is a quenching assay and the reactant is attached to a quenching moiety.

52. An assay as claimed in claim 51 wherein the quenching moiety reduces or quenches the chemiluminescence of the compound when brought into close proximity to the compound.

53. An assay as claimed in claim 43 wherein the specific binding material is capable of specifically binding with the analyte and the assay further utilises a reactant capable of specifically binding with the analyte to form a reactant-analyte-conjugate complex, which is the specific binding reaction product, the assay comprising:
allowing under suitable conditions substantial formation of the reactant-analyte-conjugate complex; and
measuring the chemiluminescence of either (i) the reactant-analyte-conjugate complex, or (ii) the conjugate not contained in the reactant-anatyte-conjugate complex.

54. An assay as claimed in claim 53 which is a sandwich assay.

55. An assay as claimed in claim 53 or 54 wherein the reactant is attached to a solid phase.

56. An assay as claimed in any of claims 53 to 55 wherein the reactant is attached to a dipstick, bead, tube or paper or polymer sheet.

57. An assay as claimed in claim 55 or 56 wherein the reactant attached to the solid phase is reacted with the analyte in the sample after which the solid phase containing the complexed analyte is separated from the remaining sample and the complex is then reacted with the conjugate.

58. A specific binding assay for detecting the presence of an analyte in a sample wherein the assay utilises a chemiluminescent moiety comprising a chemiluminescent compound as claimed in any of claims 1 to 38, and the presence of the analyte in the sample is proportional to the formation of one or more specific binding reaction products not containing the chemiluminescent compound and the compound chemituminesces in proportion to the formation of the specific binding reaction products, the assay comprising:
allowing under suitable conditions substantial formation of the specific binding reaction products; and
measuring the chemiluminescence of the compound caused by formation of the specific binding reaction products.

59. An assay as claimed in claim 58 which further utilises a reactant capable of binding with the analyte to form an analyte-reactant complex and wherein the compound chemiluminesces in proportion to the formation of the analyte-reactant complex.

60. An assay as claimed in claim 59 which is an enzyme-substrate assay where formation of the enzyme-substrate complex triggers the chemiluminescent compound.

61. An assay as claimed in claim 60 wherein the analyte is the substrate glucose and the reactant is the enzyme glucose oxidase.

62. An assay as claimed in claim 61 wherein formation of the specific binding reaction product promotes or inhibits . chemiluminescence of the compound.

63. An assay as claimed in claim 58 wherein a first reactant, which is cross-reactive with the analyte, is attached to an enzyme close to its active site and a second reactant, which is specific for both the analyte and an immunoreactive material which is added to the sample and the enzyme in the presence of a substrate.

64. An assay as claimed in claim 63 wherein the second reactant binds to the first reactant to block the active site on the enzyme in such a way that the substrate cannot bind to the enzyme at the active site or the binding of the substrate at the active site is significantly decreased.

65. An assay as claimed in any of claims 43 to 64 which is an immunoassay, protein binding assay or nucleic acid hybridisation assay.

66. An assay as claimed in claim 65 which is a nucleic acid hybridisation assay where any double-stranded DNA or RNA is converted to a single stranded form before hybridisation and immobilised on a carrier or electrophoresed onto a gel matrix.

67. An assay as claimed in any of claims 43 to 66 which is a heterogeneous assay where the reaction products, whose formation is proportional to the presence of the analyte in the sample, are separated from other products of the reaction.

68. An assay as claimed in any of claims 43 to 66 which is a homogeneous assay where the reaction products are not separated from other products of the reaction and chemiluminescence is measured from the whole assay mixture.

69. An assay as claimed in any of claims 43 to 68 wherein measuring the chemiluminescence involves if necessary, treating the compou nd with an acid to cleave Z, it Z is present and when it represents and RₙX group, and triggering the chemiluminescence if the compound of formation of the reaction product has not already done so.

## Patentansprüche

1. Chemilumineszierende Verbindung mit der allgemeinen Formel I: 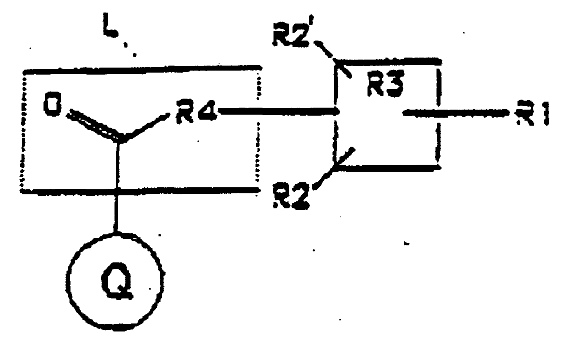 wobei:
R4 derart ist, daß der gestrichelte Kasten, der mit L bezeichnet ist, eine Ester-, Thioester- oder Amidbindung enthält;
Q einen heterocyclischen Ring oder ein heterocyclisches Ringsystem darstellt, der/das ein Kohlenstoffatom enthält, an welchem die Bindung L angelagert ist, wobei das Heteroatom in dem Ring oder Ringsystem sich in einem Oxidationszustand befindet oder befinden kann, der das Kohlenstoffatom für einen Angriff durch Peroxid oder molekularen Sauerstoff anfällig macht, um ein Zwischenprodukt zu bilden, das zur Erzeugung von Chemilumineszenz zerfällt;
R3 einen Arylring oder ein Arylringsystem darstellt, das mindestens einen sechsteiligen Ring enthält;
jedes von R2 und R2' für sich Gruppen darstellt, welche die Hydrolyse der Bindung L sterisch behindern;
R1 eine elektronenziehende Gruppe darstellt, die -NO₂, -SO₂Cl, -Br oder -N(CH₃)₃⊕ ist;
eines von R1, R2 und R2' auch eine Gruppe darstellen kann, welche imstande ist, die Verbindung an einem Protein oder einer Nucleinsäure anzulagern; oder ein Salz davon.

2. Verbindung nach Anspruch 1, wobei jedes von R2 und R2' für sich die Gruppe -R, -OR oder -SR darstellt, wobei R eine Arylgruppe darstellt.

3. Verbindung nach Anspruch 1 oder 2, wobei R2 und R2' sich in den ortho-Stellungen an R3 befinden, wenn R3 ein Phenylring, bei welchem die Esterbindung an Position 1 angehängt ist.

4. Verbindung nach einem der vorangehenden Ansprüche, wobei Q Acridinium, Benz[a,b oder c]acridinium, 1,2,4-Triazolkation, Isooxazolkation, Isothibazolkation, 1,2-Azolkation, Imidazolkation, Benzimidazolkation, Chinolinium, Isochinolinium, Chinolizinium, eine cyclische C3-, C4- oder C5-substituierte Chinolinium-, Pyridinium-, Pyrimidinium-, Pyridazinium-, Pyrazinium-, Phenanthridinium- oder Chinoxaliniumgruppe oder eine reduzierte Form davon darstellt.

5. Chemilumineszierende Verbindung mit der allgemeinen Formel I: 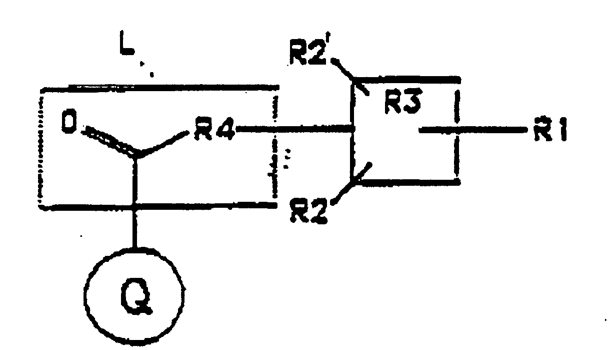 wobei:
R4 derart ist, daß der gestrichelte Kasten, der mit L bezeichnet ist, eine Ester-, Thioester- oder Amidbindung enthält;
Q einen heterocyclischen Ring oder ein heterocyclisches Ringsystem darstellt, der/das ein Kohlenstoffatom enthält, an welchem die Bindung L angelagert ist, wobei das Heteroatom in dem Ring oder Ringsystem sich in einem Oxidationszustand befindet oder befinden kann, der das Kohlenstoffatom für einen Angriff durch Peroxid oder molekularen Sauerstoff anfällig macht, um ein Zwischenprodukt zu bilden, das zur Erzeugung von Chemilumineszenz zerfällt; wobei das Ringsystem Acridinium, Benz[a,b oder c]acridinium, ein Benzimidazolkation, Chinolinium, Isochinolinium, Chinolizinium, ein cyclisches C3-, C4-oder C5- substituiertes Chinolinium, Phenanthridinium oder Chinoxalinium; oder eine reduzierte Form solcher Ringsysteme ist;
Q zusätzlich mit einem oder mehreren Perisubstituenten substituiert ist, von welchen jeder an einem Kohlenstoffatom in dem Ringsystem Q angeordnet ist, das imstande ist, einen Substituenten anzunehmen und sich am nächsten zu dem Kohlenstoffatom befindet, an welchem die Ester-, Thioester- oder Amidbindung L angelagert ist, und die eine sterische Behinderung bei diesem Kohlenstoffatom verursachen;
R3 einen Arylring oder ein Arylringsystem darstellt, das mindestens einen sechsteiligen Ring enthält;
R2 und R2' Substituenten darstellen, die gemeinsam mit dem/den Perisubstituenten die Hydrolyse der Bindung L sterisch behindern;
R1 eine elektronenziehende Gruppe darstellt, mit einem σₚ-Wert über 0,0 und wobei der zusätzliche σₚ - Wert für R1, R2 und R2', wobei R2 und/oder R2' elektronenziehende Gruppen sind, kleiner oder gleich 1,0 ist; und
eines von R1, R2 und R2' auch eine Gruppe darstellen kann, die imstande ist, die Verbindung an ein Protein oder eine Nucleinsäure anzulagern; oder ein Salz davon.

6. Verbindung nach Anspruch 5, wobei jeder Perisubstituent für sich eine Methyl-, Ethyl- oder Phenylgruppe darstellt.

7. Verbindung nach Anspruch 5 oder 6, wobei Q eine Acridinium-, Phenanthridinium-, Chinolixinium- oder Chinoliniumgruppe darstellt.

8. Verbindung nach einem der Ansprüche 5 bis 7, wobei die Perisubstituenten in den folgenden Positionen angeordnet sind:
(a) an C1 und C8, wobei Q ein Acridinium darstellt;
(b) an C7, wobei Q eine Phenanthridiniumgruppe darstellt; oder
(c) an C3, wobei Q eine Chinoliniumgruppe darstellt.

9. Verbindung nach einem der Ansprüche 5 bis 8, wobei mindestens ein Persisubstituent -CH₃ ist.

10. Verbindung nach einem der Ansprüche 5 bis 9, wobei R₃ eine Phenylgruppe darstellt und die Summe aller σₚ-Werte für R1, R2 und R2', wenn sie eine elektronenziehende Gruppe darstellen, kleiner oder gleich 1,0 ist.

11. Chemilumineszierende Verbindung mit der allgemeinen Formel II: 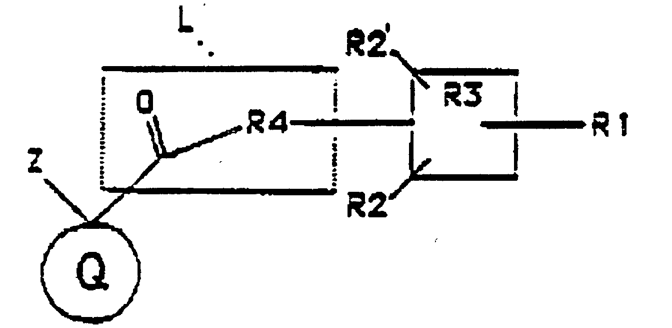 wobei:
R4 derart ist, daß der gestrichelte Kasten, der mit L bezeichnet ist, eine Ester-, Thioester- oder Amidbindung enthält;
Q einen heterocyclischen Ring oder ein heterocyclisches Ringsystem darstellt, der/das ein Kohlenstoffatom enthält, an welchem die Bindung L angelagert ist, wobei das Heteroatom in dem Ring oder Ringsystem sich in einem Oxidationszustand befindet oder befinden kann, der das Kohlenstoffatom für einen Angriff durch Peroxid oder molekularen Sauerstoff anfällig macht, um ein Zwischenprodukt zu bilden, das zur Erzeugung von Chemilumineszenz zerfällt;
R3 einen Arylring oder ein Arylringsystem darstellt, das mindestens einen sechsteiligen Ring enthält;
jedes von R2 und R2' für sich Substituenten darstellt, welche die Hydrolyse der Bindung L sterisch behindern;
R1 eine elektronenziehende Gruppe darstellt mit einem σₚ-Wert über 0,0 und wobei der zusätzliche σₚ-Wert für R1, R2 oder R2', wobei R2 und/oder R2' eltronenziehende Gruppen sind, kleiner oder gleich 1,0 ist;
eines von R1, R2 und R2' auch eine Gruppe darstellen kann, die imstande ist, die Verbindung an ein Protein oder eine Nucleinsäure anzulagem;
Z einen Substituenten darstellt, der an das sp³-hybridisierte Kohlenstoffatom angelagert ist, an welches die Bindung L angelagert ist, und folgendes ist:
- ein Wasserstoff- oder Halogenatom;
- CN;
- OR;
- NR₂;
- NR^{⊕}₃;
- SR;
- SR^{⊕}₂;
- S₂R;
- NRCO₂R;
- NHNR₂;
- NRNR₂;
- ONR₂;
- NHOR;
- CR(CN)₂;
- CR(COR)₂;
- CR₂NO₂;
- C ≡ COR;
- XRₙ;
- ein Arzneidrogen- oder Steroidmolekül;
- 2 - Oxazol;
- 1 - Imidazol;
wobei:
R eine Alkylgruppe, eine Arylgruppe, eine Aminosäure, einen Zucker darstellt oder mehrere R-Gruppen 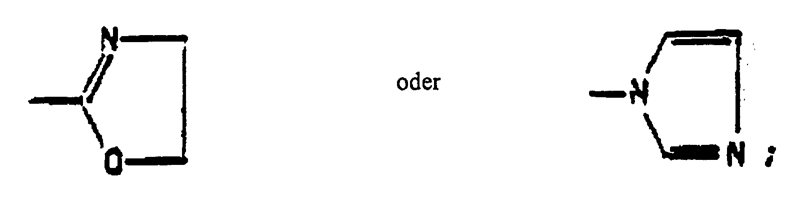
darstellen können;
X ein Sauerstoff-, Stickstoff-, Schwefel- oder Kohlenstoffatom darstellt;
n eine ganze Zahl von 1 bis 3 ist, deren Wert durch die Valenz des Atoms bestimmt wird, das durch X dargestellt ist; oder ein Salz davon.

12. Verbindung nach Anspruch 11, welche (2,6-dimethoxy-3-chlorsulfonyl)phenyl-N-methyl-acridan-9-ethoxy-9-carboxylat ist.

13. Verbindung nach einem der Ansprüche 11 oder 12, wobei, wenn Z RₙX ist, es durch Behandlung der Verbindung mit einer Säure gespalten werden kann.

14. Verbindung nach einem der vorangehenden Ansprüche, wobei Q ein Acridinium, Benz[a,b oder c]acridinium, 1,2,4-Triazolkation, Isooxazolkation, Isothioazolkation, 1,2-Azolkation, Imidazolkation, Benzimidazolkation, Chinolinium, Isochinolinium, Chinolizinium, eine cyclische C3-, C4- oder C5- substituierte Chinolinium-, Pyridinium-, Pyrimidinium-, Pyridazinium-, Pyrazinium-, Phenanthridinium- oder Chinoxaliniumgruppe oder eine reduzierte Form davon darstellt.

15. Chemilumineszierende Verbindung mit der allgemeinen Formel I: 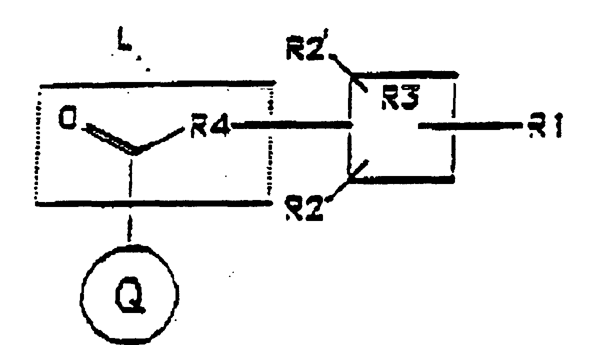 wobei:
R4 derart ist, daß der gestrichelte Kasten, der mit L bezeichnet ist, eine Ester-, Thioester- oder Amidbindung enthält;
Q einen heterocyclischen Ring oder ein heterocyclisches Ringsystem darstellt, der/das ein Kohlenstoffatom enthält, an welchem die Bindung L angelagert ist, wobei das Heteroatom in dem Ring oder Ringsystem sich in einem Oxidationszustand befindet oder befinden kann, der das Kohlenstoffatom für einen Angriff durch Peroxid oder molekularen Sauerstoff anfällig macht, um ein Zwischenprodukt zu bilden, das zur Erzeugung von Chemilumineszenz zerfällt;
Q zusätzlich durch einen Substituenten substituiert ist, der imstande ist, durch eine Alkyl-, Alkenyl-, Alkynyl-, Alkylamino-, Oxoalkyl-, Thioalkyl- oder eine Alkylaxycarbonyl - Gruppe, die an Q gebunden ist, mit einem spezifischen Bindungsmaterial konjugiert zu werden, wobei der Substituent mindestens eine der folgenden funktionelle Gruppen aufweist:
- COOR, wobei R eine Alkyl- oder Arylgruppe darstellt;
- C(OR)= NH^{⊕}₂, wobei R einen Rest eines funktionellen Alkohols darstellt;
- SO₂Cl;
- NCS; 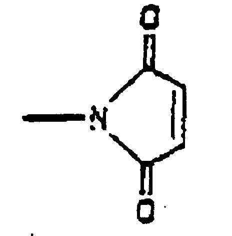 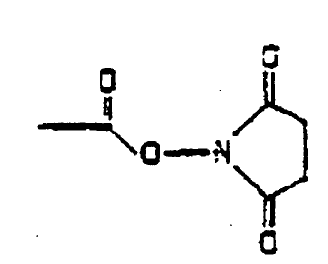 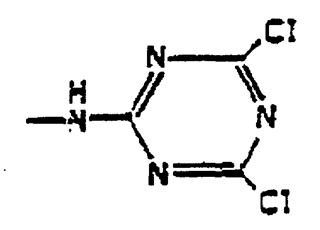
- N(CH₃)(CH₂)ₘCl, wobei m zumindest eins ist;
- N₃;
- NH₂; oder
- ein Ion, einen Zucker, ein Polyamin, Polyoxyethylen oder Polyoxybutylen;
R3 einen Arylring oder ein Arylringsystem darstellt, das mindestens einen sechsteiligen Ring enthält;
jedes von R2 und R2' für sich Substituenten darstellt, welche die Hydrolyse der Bindung L sterisch behindern;
R1 eine elektronenziehende Gruppe darstellt mit einem σₚ-Wert über 0,0 und wobei der zusätzliche σₚ-Wert für R1, R2 oder R2', wobei R2 und/oder R2' elektronenziehende Gruppen sind kleiner oder gleich 1,0 ist; oder ein Salz davon.

16. Verbindung nach Anspruch 15, wobei der Substituent durch eine Oxoalkyl- oder Alkyloxycarbonylgruppe gebunden ist.

17. Verbindung nach Anspruch 15 oder 16, wobei die funktionelle Gruppe Succinimid oder N-Carboxylsuccinimid ist.

18. Verbindung nach einem der Ansprüche 15 bis 17, wobei Q ein Acridinium, Benz[a,b oder c]acridinium, 1,2,4-Triazolkation, Isooxazolkation, Isothioazolkation, 1,2-Azolkation, Imidazolkation, Benzimidazolkation, Chinolinium, Isochinolinium, Chinolizinium, eine cyclische C3-, C4- oder C5- substituierte Chinolinium-, Pyridinium-, Pyrimidinium-, Pyridazinium-, Pyrazinium-, Phenanthridinium- oder Chinoxaliniumgruppe oder eine reduzierte Form davon darstellt.

19. Verbindung nach einem der vorangehenden Ansprüche, wobei R4 -Odarstellt, so daß L eine Esterbindung ist.

20. Verbindung nach einem der vorangehenden Ansprüche, wobei R3 eine Phenylgruppe darstellt.

21. Verbindung nach einem der vorangehenden Ansprüche, wobei R1 -NO₂, SO₂Cl oder -N(CH₃)₃^{⊕} darstellt.

22. Verbindung nach einem der vorangehenden Ansprüche, wobei R1 -SO₂Cl darstellt.

23. Verbindung nach einem der vorangehenden Ansprüche, wobei R2 und R2'-CH₃ oder OCH₃ darstellen.

24. Verbindung nach einem der vorangehenden Ansprüche, wobei R3 eine Phenylgruppe darstellt und die Summe aller σₚ-Werte für R1, R2 und R2', wenn sie eine elektronenziehende Gruppe darstellen, kleiner oder gleich 1,0 ist.

25. Verbindung nach einem der vorangehenden Ansprüche, wobei Q eine Acridiniumgruppe darstellt.

26. Verbindung nach einem der Ansprüche 1 bis 4, 19, 20 und 23 bis 25, die folgendes ist:

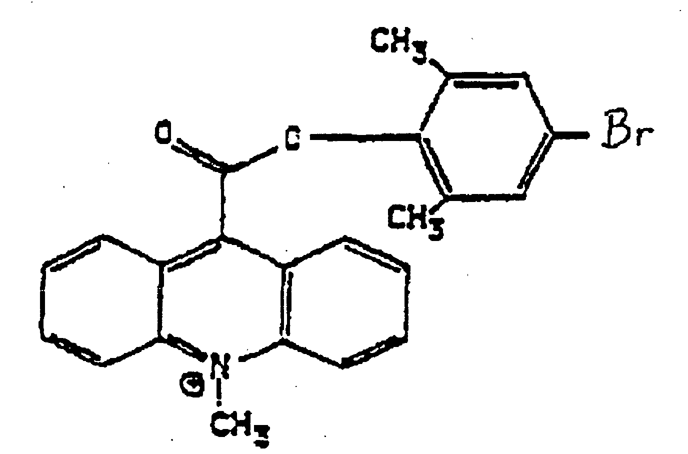

27. Verbindung nach einem der Ansprüche 1 bis 4 und 19 bis 25, die folgendes ist:

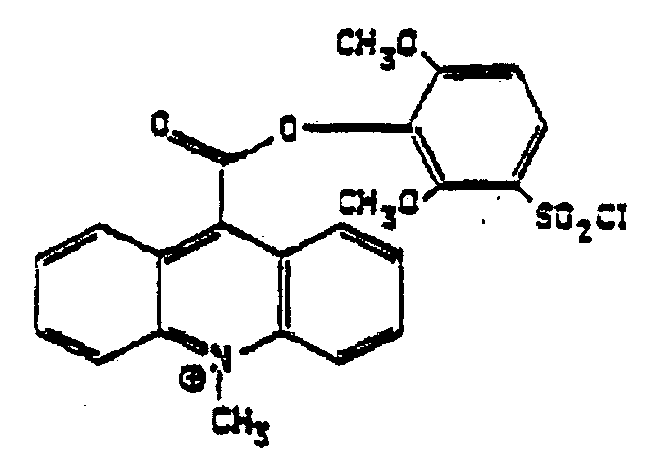

28. Verbindung nach einem der Ansprüche 1 bis 4 und 19 bis 25, die folgendes ist:

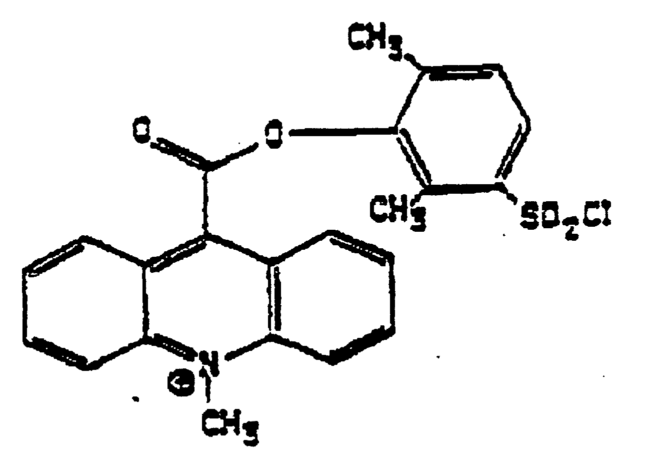

29. Verbindung nach einem der Ansprüche 1 bis 4, 19 bis 21 und 23 bis 25, die folgendes ist:

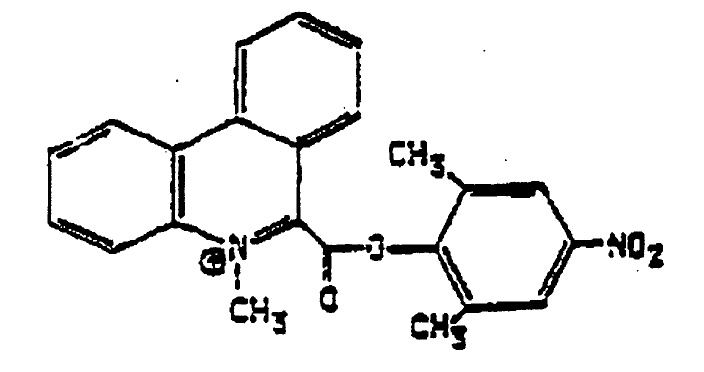

30. Verbindung nach einem der Ansprüche 1 bis 4, 19 bis 21, 23 und 24, die folgendes ist:

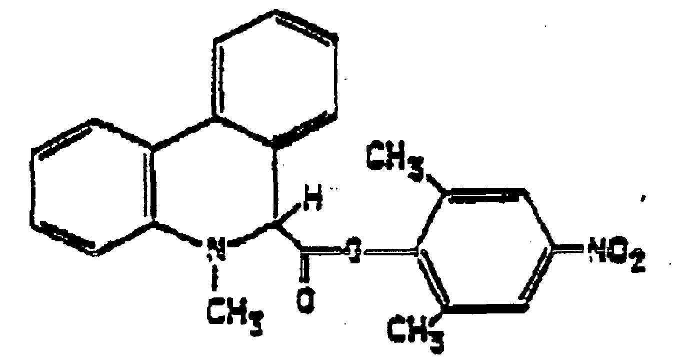

31. Verbindung nach einem der Ansprüche 1 bis 4 und 19 bis 24, die folgendes ist:

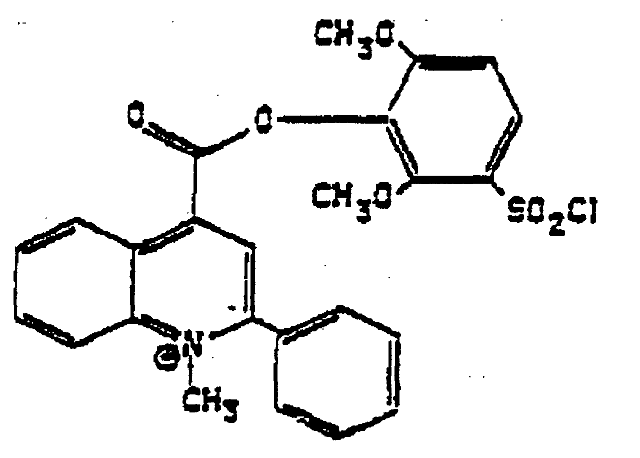

32. Verbindung nach einem der Ansprüche 1 bis 4 und 19 bis 24, die folgendes ist:

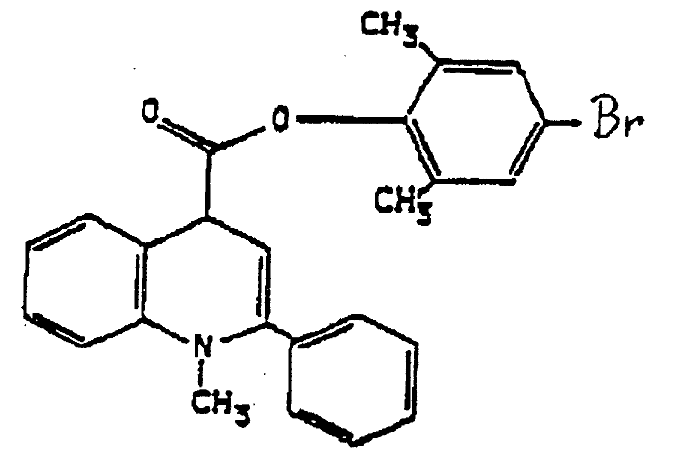

33. Verbindung nach einem der Ansprüche 1 bis 4, 19, 20, 23 und 24, die folgendes ist:

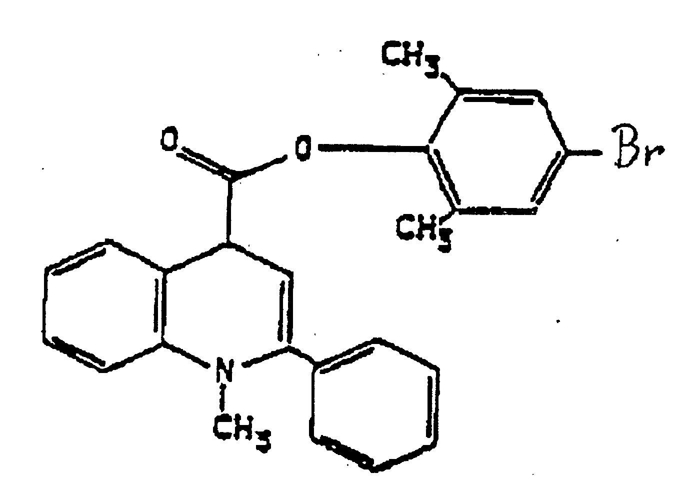

34. Verbindung nach einem der Ansprüche 11 bis 14, die (2,6-Dimethoxy-3-chlorosulphonyl)phenyl-N-methyl-acridan-9-ethoxy-9-carboxylat, (2,6-Dimethoxy-3-chlorosulphonyl)phenyl-N-methyl-acridan-9-methoxy-9-carboxylat oder (2,6-Dimethyl-3-chlorosulphonyl)phenyl-N-methyl-acridan-9-methoxy-9-carboxylat ist.

35. Verbindung nach einem der Ansprüche 15 bis 21 und 23 bis 25, die 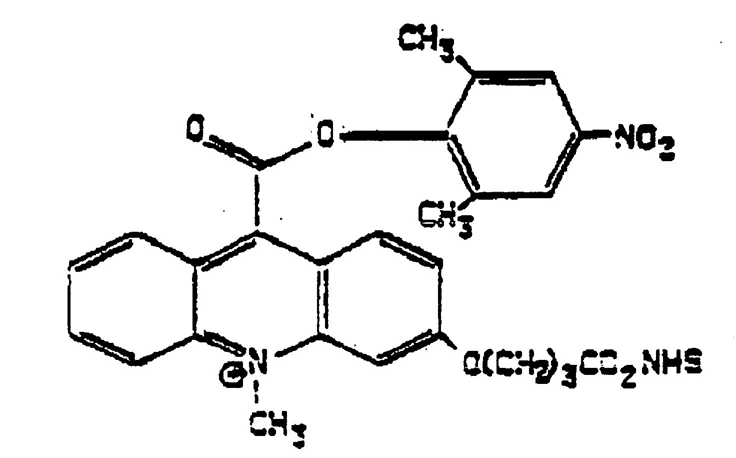 folgendes ist:

36. Verbindung nach einem der Ansprüche 15 bis 21 und 23 bis 25, die folgendes ist:

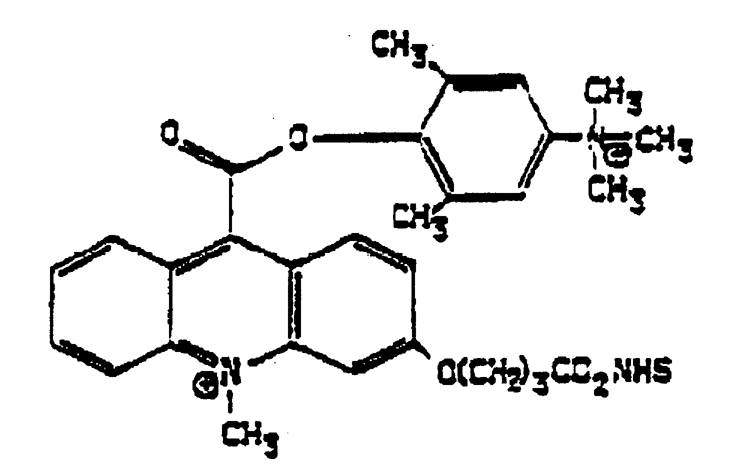

37. Verbindung nach einem der Ansprüche 15 bis 21 und 23 bis 25, die folgendes ist:

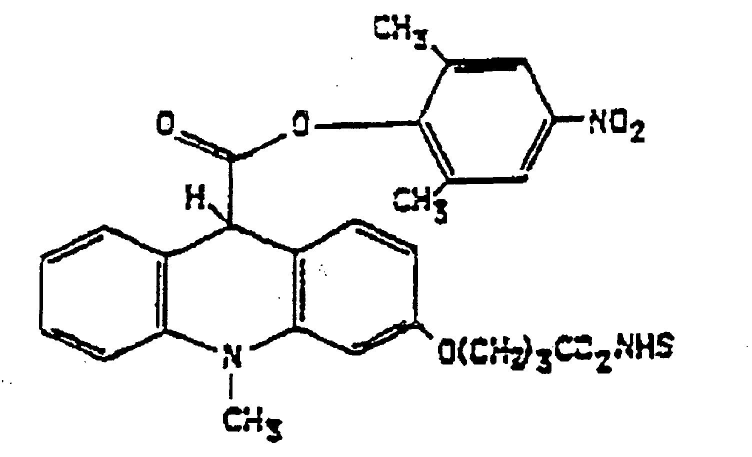

38. Verbindung nach einem der Ansprüche 15 bis 21 und 23 bis 25, die folgendes ist:

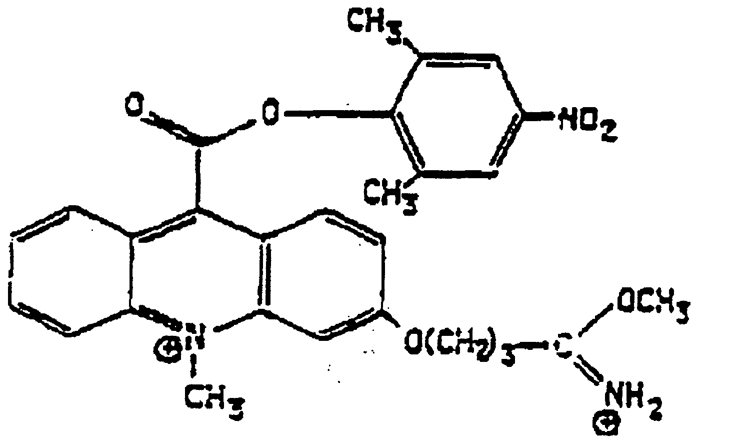

39. Konjugat, das eine chemilumineszierende Verbindung nach einem der Ansprüche 1 bis 38 umfaßt, die an einen spezifischen Bindungspartner für eine biologische, biochemische oder chemische Spezies gebunden ist.

40. Konjugat nach Anspruch 39, das spezifisch durch eine Immunreaktion, Proteinbindungsreaktion oder Nucleinhybridisierung binden kann.

41. Zusammensetzung, das eine chemilumineszierende Verbindung nach einem der Ansprüche 1 bis 38 oder ein Konjugat nach Anspruch 39 oder 40 umfaßt.

42. Spezifischer Bindungstestkit, der eine Ampulle aufweist, die eine Zusammensetzung nach Anspruch 41 enthält.

43. Spezifischer Bindungstest für den Nachweis der Gegenwart eines Analyts in einer Probe, wobei der Test ein chemilumineszierendes Konjugat nach Anspruch 39 oder 40 oder eine chemilumineszierende Verbindung nach einem der Ansprüche 1 bis 38, die an ein spezifisches Bindungsmaterial angelagert ist, verwendet, wobei die Gegenwart des Analyts in der Probe proportional zu der Bildung eines oder mehrerer spezifischer Bindungsreaktionsprodukte ist, die das Konjugat enthalten, wobei der Test umfaßt:
das Ermöglichen einer wesentlichen Bildung eines oder mehrerer spezifischer Bindungsreaktionsprodukte, die das Konjugat enthalten, unter geeigneten Bedingungen; und
das Messen der Chemilumineszenz entweder (i) eines oder mehrerer spezifischer Bindungsreaktionsprodukte oder (ii) des Konjugates, das nicht in den Bindungsreaktionsprodukten nicht enthalten ist.

44. Test nach Anspruch 43, wobei das spezifische Bindungsmaterial imstande ist, spezifisch mit dem Analyt zu binden und das spezifische Bindungsreaktionsprodukt ein Analyt-Konjugat-Komplex ist, wobei der Test umfaßt:
das Ermöglichen einer wesentlichen Bildung des Analyt-Konjugat-Komplexes unter geeigneten Bedingungen; und
das Messen der Chemilumineszenz entweder (i) des Analyt-Konjugat-Komplexes oder (ii) des Konjugates, das nicht in dem Analyt-Konjugat-Komplex enthalten ist.

45. Test nach Anspruch 44, der ein Oberflächenantigentest ist, wobei das spezifische Bindungsmaterial ein Antikörper ist, der für ein Oberflächenantigen auf einer Zelle spezifisch ist.

46. Test nach Anspruch 44 oder 45, der weiters einen Reaktionspartner verwendet, der zur spezifischen Bindung sowohl mit (i) dem Analyt zur Bildung eines Analyt-Reaktionspartner-Komplexes als auch mit (ii) dem spezifischen Bindungsmaterial zur Bildung eines Konjugat-Reaktionspartner-Komplexes, der das spezifische Bindungsreaktionsprodukt ist, imstande ist, wobei der Test umfaßt:
das Ermöglichen einer wesentlichen Bildung des Analyt-Reaktionspartner-Komplexes und Konjugat-Reaktionspartner-Komplexes unter geeigneten Bedingungen; und
das Messen der Chemilumineszenz entweder (i) des Konjugat-Reaktionspartner-Komplexes oder (ii) des Konjugates, das nicht in dem Konjugat-Reaktionspartner-Komplex enthalten ist.

47. Test nach Anspruch 43, der ein kompetitiver Test ist.

48. Test nach Anspruch 46 oder 47, wobei der Reaktionspartner an eine Festphase angelagert ist oder Komplexe, die den Reaktionspartner enthalten, ausgefällt sind.

49. Test nach Anspruch 43, der ein Sequenz-Sättigungstest ist und der Analyt zunächst mit dem Reaktionspartner vor der Reaktion des Konjugates mit dem verbleibenden, nicht reagierten Reaktionspartner zur Reaktion gebracht wird.

50. Test nach Anspruch 43, der ein kompetitiver Verdrängungstest ist, wobei das Konjugat den Analyt, der bereits an den Reaktionspartner gebunden ist, kompetitiv verdrängt.

51. Test nach Anspruch 43, der ein Löschungstest ist, wobei der Reaktionspartner an eine Löschungskomponente angelagert ist.

52. Test nach Anspruch 51, wobei die Löschungskomponente die Chemilumineszenz der Verbindung verringert oder löscht, wenn sie sehr nahe an die Verbindung herangebracht wird.

53. Test nach Anspruch 43, wobei das spezifische Bindungsmaterial zur spezifischen Bindung mit dem Analyt imstande ist und der Test weiters einen Reaktionspartner verwendet, der zur spezifischen Bindung mit dem Analyt zur Bildung eines Reaktionspartner-Analyt-Konjugat-Komplexes, der das spezifische Bindungsreaktionsprodukt ist, imstande ist, wobei der Test umfaßt:
das Ermöglichen einer wesentlichen Bildung des Reaktionspartner-Analyt-Konjugat-Komplexes unter geeigneten Bedingungen; und
das Messen der Chemilumineszenz entweder (i) des Reaktionspartner-Analyt-Konjugat-Komplexes oder (ii) des Konjugates, das nicht in dem Reaktionspartner-Analyt-Konjugat-Komplex enthalten ist.

54. Test nach Anspruch 53, der ein Sandwich-Test ist.

55. Test nach Anspruch 53 oder 54, wobei der Reaktionspartner an eine Festphase angelagert ist.

56. Test nach einem der Ansprüche 53 bis 55, wobei der Reaktionspartner an einen Tauchstab, eine Perle, ein Rohr oder ein Papier oder eine Polymerfolie angelagert ist.

57. Test nach Anspruch 55 oder 56, wobei der Reaktionspartner, der an die Festphase angelagert ist, mit dem Analyt in der Probe zur Reaktion gebracht wird, wonach die Festphase, die das zum Komplex gebildete Analyt enthält, von der übrigen Probe abgetrennt wird und der Komplex dann mit dem Konjugat zur Reaktion gebracht wird.

58. Spezifischer Bindungstest für den Nachweis der Gegenwart eines Analyts in einer Probe, wobei der Test eine chemilumineszierende Komponente verwendet, die eine chemilumineszierende Verbindung nach einem der Ansprüche 1 bis 38 umfaßt, und die Gegenwart des Analyts in der Probe proportional zu der Bildung eines oder mehrerer spezifischer Bindungsreaktionsprodukte ist, welche die chemilumineszierende Verbindung nicht enthalten, und die Verbindung im Verhältnis zu der Bildung der spezifischen Bindungsreaktionsprodukte chemiluminesziert, wobei der Test umfaßt:
das Ermöglichen einer wesentlichen Bildung der spezifischen Bindungsreaktionsprodukte unter geeigneten Bedingungen; und
das Messen den Chemilumineszenz der Verbindung, die durch die Bildung der spezifischen Bindungsreaktionsprodukte verursacht wird.

59. Test nach Anspruch 58, der weiters einen Reaktionspartner verwendet, der zur Bindung mit dem Analyt zur Bildung eines Analyt-Reaktionspartner-Komplexes imstande ist, und wobei die Verbindung im Verhältnis zu der Bildung des Analyt-Reaktionspartner-Komplexes chemiluminesziert.

60. Test nach Anspruch 59, der ein Enzym-Substrat-Test ist, wobei die Bildung des Enzym-Substrat-Komplexes die chemilumineszierende Verbindung triggert.

61. Test nach Anspruch 60, wobei der Analyt das Substrat Glucose ist und der Reaktionspartner das Enzym Gluxoseoxidase ist.

62. Test nach Anspruch 61, wobei die Bildung des spezifischen Bindungsreaktionsproduktes die Chemilumineszenz der Verbindung fördert oder hemmt.

63. Test nach Anspruch 58, wobei ein erster Reaktionspartner, der mit dem Analyt kreuzreaktionsfähig ist, an ein Enzym nahe seiner aktiven Stelle angelagert ist und ein zweiter Reaktionspartner, der sowohl für das Analyt als auch für ein immunreaktionsfähiges Material spezifisch ist, der Probe und dem Enzym in Gegenwart eines Substrates zugegeben wird.

64. Test nach Anspruch 63, wobei der zweite Reaktionspartner an den ersten Reaktionspartner zum Blockieren der aktiven Stelle an dem Enzym derart bindet, daß das Substrat nicht an das Enzym an der aktiven Stelle binden kann oder die Bindung des Substrates an der aktiven Stelle signifikant verringert ist.

65. Test nach einem der Ansprüche 43 bis 64, der ein Immuntest, Proteinbindungstest oder Nucleinsäure-Hybridisierungstest ist.

66. Test nach Anspruch 65, der ein Nucleinsäure-Hybridisierungstest ist, wobei jede doppelsträngige DNA oder RNA in eine einzelsträngige Form vor der Hybridisierung umgewandelt wird und auf einem Träger immobilisiert oder auf einer Gelmatrix einer Elektrophorese unterzogen wird.

67. Test nach einem der Ansprüche 43 bis 66, der ein heterogener Test ist, wobei die Reaktionsprodukte, deren Bildung proportional zu der Gegenwart des Analyts in der Probe ist, von anderen Produkten der Reaktion abgetrennt werden.

68. Test nach einem der Ansprüche 43 bis 66, der ein homogener Test ist, wobei die Reaktionsprodukte nicht von anderen Produkten der Reaktion abgetrennt werden und die Chemilumineszenz aus der ganzen Testmischung gemessen wird.

69. Test nach einem der Ansprüche 43 bis 68, wobei die Messung der Chemilumineszenz gegebenenfalls die Behandlung der Verbindung mit einer Säure zur Spaltung von Z beinhaltet, wenn Z vorhanden ist, und wenn es eine RₙX-Gruppe darstellt, sowie das Triggern der Chemilumineszenz, wenn die Verbindung zur Bildung des Reaktionsproduktes dies nicht bereits vorgenommen hat.

## Revendications

1. Composé chimioluminescent ayant la formule générale I : 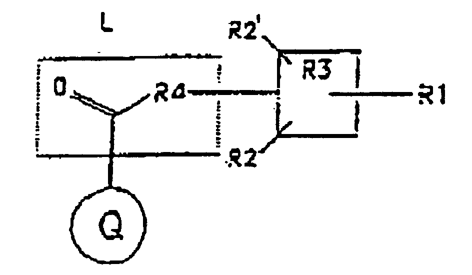 dans laquelle :
R4 est tel que la boite en traits interrompus marquée L contient une liaison ester, thioester ou amide ;
Q représente un hétérocycle ou un système hétérocyclique contenant un atome de carbone auquel la liaison L est attachée, où l'hétéroatome dans le cycle ou le système cyclique est dans, ou est capable d'être dans, un état d'oxydation qui rend l'atome de carbone susceptible d'être attaqué par un peroxyde ou de l'oxygène moléculaire pour former un intermédiaire qui se désintègre pour produire de la chimioluminescence ;
R3 représente un cycle ou un système cyclique aryle contenant au moins un cycle à six membres ;
chacun des R2 et R2' représente individuellement des groupements qui empêchent stériquement l'hydrolyse de la liaison L ;
R1 représente un groupement capteur d'électron qui est -NO₂, -SO₂Cl, -Br ou -N(CH₃)₃• ;
un des R1, R2 et R2' peut aussi représenter un groupement capable d'attacher le composé à une protéine ou un acide nucléique ; ou un sel de celui-ci.

2. Composé selon la revendication 1 dans lequel chacun des R2 et R2' représente individuellement le groupement -R, -OR ou -SR où R représente un groupement aryle.

3. Composé selon la revendication 1 ou 2 dans lequel R2 et R2' sont en position ortho sur R3, où R3 est un cycle phényle avec la liaison ester attachée en position 1.

4. Composé selon l'une quelconque des revendications précédentes dans lequel Q représente un groupement acridinium, un benz[a,b ou c] acridinium, un cation 1,2,4-triazole, un cation isooxazole, un cation isothioazole, un cation 1,2-azole, un cation imidazole, un cation benzimidazole, un groupement quinolinium, isoquinolinium, quinolizinium, quinolinium cyclique substitué en C3, C4 ou C5, pyridinium, pyrimidinium, pyridazinium, pyrazinium, phénanthridinium ou quinoxalinium, ou une forme réduite de ceux-ci.

5. Composé chimioluminescent ayant la formule générale I ; 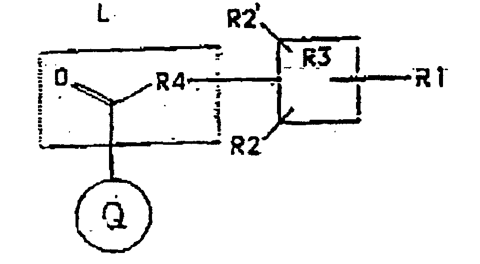 dans laquelle :
R4 est tel que la boîte en traits interrompus marquée L contient une liaison ester, thioester ou amide ;
Q représente un hétérocycle ou un système hétérocyclique contenant un atome de carbone auquel la liaison L est attachée, où l'hétéroatome dans le cycle ou le système hétérocyclique est dans un état d'oxydation qui rend l'atome de carbone susceptible d'être attaqué par un peroxyde ou de l'oxygène moléculaire pour former un intermédiaire qui se désintègre pour produire de la chimioluminescence, où le système cyclique est un groupement acridinium, benz[a,b ou c]acridinium, un cation benzimidazole, un groupement quinolinium, isoquinolinium, quinolizinium, quinolinium cyclique substitué en C3, C4 ou C5, phénanthridinium ou quinoxalinium ; ou une forme réduite de ces systèmes cycliques ;
Q étant de plus substitué avec un ou plusieurs péri substituants dont chacun est localisé sur un atome de carbone dans le système cyclique Q qui est capable d'accepter un substituant et est le plus proche de l'atome de carbone auquel la liaison L ester, thioester ou amide est attachée et qui provoque l'encombrement stérique de cet atome de carbone ;
R3 représente un cycle ou un système cyclique aryle contenant au moins un cycle à six membres ;
R2 et R2' représentent des substituants qui avec le(s) péri substituant(s) encombrent stériquement l'hydrolyse de la liaison L ;
R1 représente un groupement capteur d'électron ayant une valeur σₚ supérieure à 0,0, et où la valeur σₚ supplémentaire pour R1, R2 et R2', où R2 et/ou R2' sont des groupements capteurs d'électron, est inférieure ou égale à 1,0 ; et
un des R1, R2 et R2' peut aussi représenter un groupement capable d'attacher le composé à une protéine ou un acide nucléique ; ou un sel de ceux-ci.

6. Composé selon la revendication 5 dans lequel chaque péri substituant représente individuellement un groupement méthyle, éthyle ou phényle.

7. Composé selon la revendication 5 ou 6 dans lequel Q représente un groupement acridinium, phénanthridinium, quinolixinium ou quinolinium.

8. Composé selon l'une quelconque des revendications 5 à 7 dans lequel les péri substituants sont placés dans les positions suivantes :
(a) en C1 et C8, où Q représente un groupement acridinium ;
(b) en C7, où Q représente un groupement phénanthridinium ; ou
(c) en C3, où Q représente un groupement quinolinium.

9. Composé selon l'une quelconque des revendications 5 à 8 dans lequel au moins un péri substituant est -CH₃.

10. Composé selon l'une quelconque des revendications 5 à 9 dans lequel R3 représente un groupement phényle et la somme de toutes les valeurs σₚ pour R1, R2 et R2', quand ils représentent un groupement capteur d'électron, est inférieure ou égale à 1,0.

11. Composé chimioluminescent ayant la formule générale II : 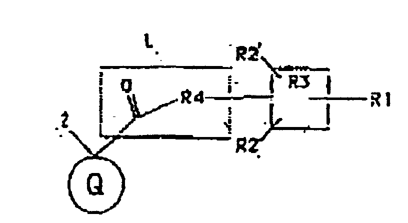 dans laquelle ;
R4 est tel que la boîte en traits interrompus marquée L contient une liaison ester, thioester ou amide ;
Q représente un hétérocycle ou un système hétérocyclique contenant un atome de carbone auquel la liaison L est attachée, où l'hétéroatome dans le cycle ou le système cyclique est dans, ou est capable d'être dans, un état d'oxydation qui rend l'atome de carbone susceptible d'être attaqué par un peroxyde ou de l'oxygène moléculaire pour former un intermédiaire qui se désintègre pour produire de la chimioluminescence ;
R3 représente un cycle ou un système cyclique aryle contenant au moins un cycle à six membres ;
chacun des R2 et R2' représente individuellement des substituants qui encombrent stériquement l'hydrolyse de la liaison L ;
R1 représente un groupement capteur d'électron ayant une valeur σₚ supérieure à 0,0, et où la valeur σₚ supplémentaire pour R1, R2 et R2', où R2 et/ou R2' sont des groupements capteurs d'électron, est inférieure ou égale à 1,0;
un des R1, R2 et R2' peut aussi représenter un groupement capable d'attacher le composé à une protéine ou un acide nucléique ;
Z représente un substituant attaché à l'atome de carbone hybridé sp³ auquel la. liaison L est attachée et est :
- un atome d'hydrogène ou d'halogène ;
- CN ;
- OR ;
- NR₂ ;
- NR₃• ;
- SR ;
- SR₂• ;
- S₂R ;
- NRCO₂R ;
- NHNR₂ ;
- NRNR₂;
- ONR₂ ;
- NHOR ;
- CR(CN)₂ ;
- CR(COR)₂ ;
- CR₂NO₂ ;
- C ≡ COR ;
- XRₙ ;
- une molécule de médicament ou de stéroïde ;
- 2-oxazole ;
- 1-imidazole ;
dans lequel :
R représente un groupement alkyle, un groupement aryle, un acide aminé, un sucre ou des groupements multiples de R peuvent représenter 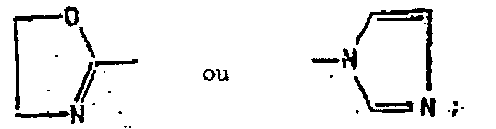
X représente un atome d'oxygène, d'azote, de soufre ou de carbone ;
n est un nombre entier de 1 à 3 dont la valeur est déterminée par la valence de l'atome représenté par X ; ou un sel de ceux-ci.

12. Composé selon la revendication 11 qui est le (2,6-diméthoxy-3-chlorosulfonyl)phényl-N-méthyl-acridan-9-éthoxy-9-carboxylate.

13. Composé selon l'une quelconque des revendications 11 ou 12 dans lequel, quand Z est RₙX, il peut être clivé en traitant le composé avec un acide,

14. Composé selon l'une quelconque des revendications précédentes dans lequel Q représente un groupement acridinium, benz[a, b ou c]acridinium, un cation 1,2,4-triazole, un cation isooxazole, un cation isothioazole, un cation 1,2-azole, un cation imidazole, un cation benzimidazole, un groupement quinolinium, isoquinolinium; quinolizinium, quinolinium cyclique substitué en C3, C4 ou C5, pyridinium, pyrimidinium, pyridazinium, pyrazinium, phénanthridinium ou quinoxalinium, ou une forme réduite de ceux-ci.

15. Composé chimioluminescent ayant la formule générale I ; 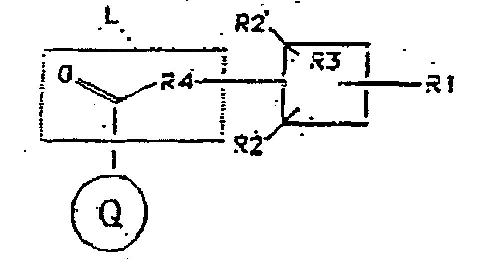 dans laquelle :
R4 est tel que la boite en traits interrompus marquée L contient une liaison ester, thioester ou amide ;
Q représente un hétérocycle ou un système cyclique contenant un atome de carbone auquel la liaison L est attachée, où l'hétéroatome dans le cycle ou le système cyclique est dans, ou est capable d'être dans, un état d'oxydation qui rend l'atome de carbone susceptible d'être attaqué par un peroxyde ou de l'oxygène moléculaire pour former un intermédiaire qui se désintègre pour produire de la chimioluminescence ;
Q étant de plus substitué par un substituant capable d'être conjugué avec un matériau de liaison spécifique, par un groupement alkyle, alcényle, alcynyle, alkylamino, oxoalkyle, thioalkyle ou alkyloxycarbonyle lié à Q, le substituant ayant au moins un des groupements fonctionnels suivants :
- COOR, où R représente un groupement alkyle ou aryle ;
- C(OR) = NH₂•, où R représente un résidu d'un alcool fonctionnel ;
- SO₂Cl ;
- NCS ; 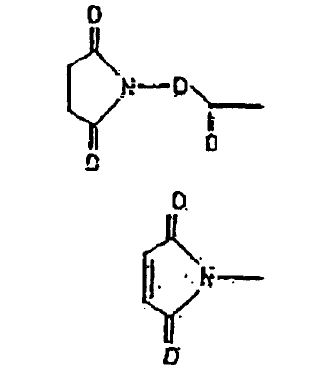 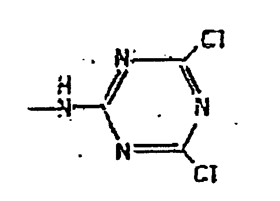
- M(CH₃) (CH₂)ₘCl, où m est au moins 1 ;
- N₃ ;
- NH₂ ; ou
- un ion, un sucre, une polyamine, un polyoxyéthylène ou un polyoxybutylène ;
R3 représente un cycle ou un système cyclique aryle contenant au moins un cycle à six membres ;
chacun des R2 et R2' représente individuellement des substituants qui encombrent stériquement l'hydrolyse de la liaison L ;
R1 représente un groupement capteur d'électron ayant une valeur σₚ supérieure à 0,0, et où la valeur σₚ supplémentaire pour R1, R2 et R2', où R2 et/ou R2' sont des groupements capteurs d'électron, est inférieure ou égale à 1,0 ; ou un sel de ceux-ci.

16. Composé selon la revendication 15 dans lequel le substituant est lié par un groupement oxoalkyle ou alkyloxycarbonyle.

17. Composé selon la revendication 15 ou 16 dans lequel le groupement fonctionnel est un groupement succinimide ou N-carboxysuccinimide.

18. Composé selon l'une quelconque des revendications 15 à 17 dans lequel Q représente un groupement acridinium, benz[a,b ou c]acridinium, un cation 1,2,4-triazole, un cation isooxazole, un cation isothioazole, un cation 1,2-azole, un cation imidazole, un cation benzimidazole, un groupement quinolinium, isoquinolinium, quinolizinium, quinolinium cyclique substitué en C3, C4 ou C5, pyridinium, pyrimidinium, pyridazinium, pyrazinium, phénanthridinium ou quinoxalinium, ou une forme réduite de ceux-ci.

19. Composé selon l'une quelconque des revendications précédentes dans lequel R4 représente -O- si bien que L est une liaison ester.

20. Composé selon l'une quelconque des revendications précédentes dans lequel R3 représente un groupement phényle.

21. Composé selon l'une quelconque des revendications précédentes dans lequel R1 représente -NO₂, -SO₂Cl ou -N(CH₃)₃•.

22. Composé selon l'une quelconque des revendications précédentes dans lequel R1 représente -SO₂Cl.

23. Composé selon l'une quelconque des revendications précédentes dans lequel R2 et R2' représentent -CH₃, ou -OCH₃.

24. Composé selon l'une quelconque des revendications précédentes dans lequel R3 représente un groupement phényle et la somme de toutes les valeurs de σₚ pour R1, R2 et R2', quand ils représentent un groupement capteur d'électron, est inférieure ou égale à 1,0.

25. Composé selon l'une quelconque des revendications précédentes dans lequel Q représente un groupement acridinium.

26. Composé selon l'une quelconque des revendications 1 à 4, 19, 20 et 23 à 25 qui est :

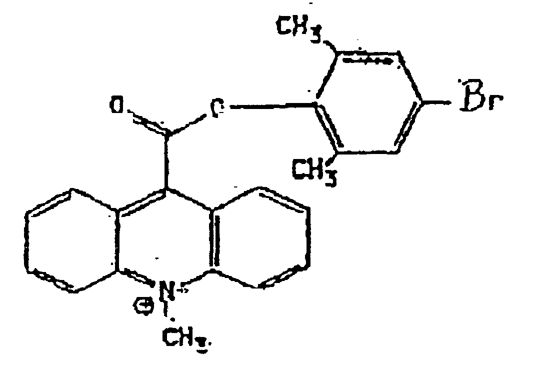

27. Composé selon l'une quelconque des revendications 1 à 4 et 19 à 25 qui est :

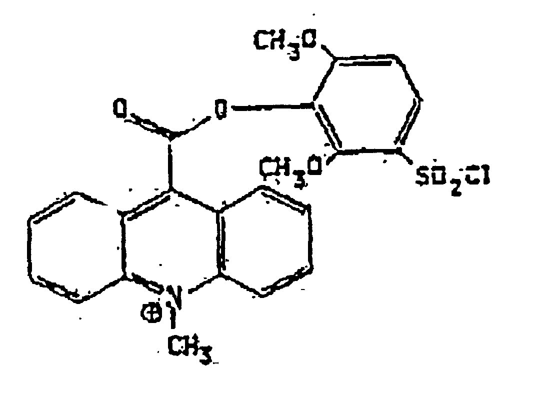

28. Composé selon l'une quelconque des revendications 1 à 4 et 19 à 25 qui est :

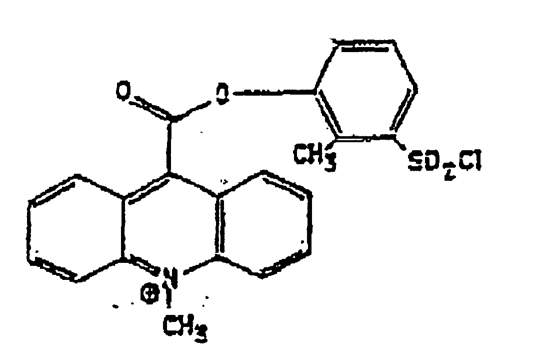

29. Composé selon l'une quelconque des revendications 1 à 4, 19 à 21 et 23 à 25 qui est :

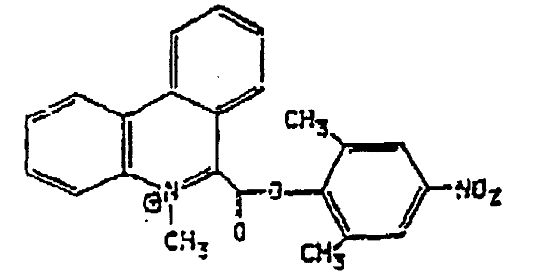

30. Composé selon l'une quelconque des revendications 1 à 4, 19 à 21, 23 et 24 qui est :

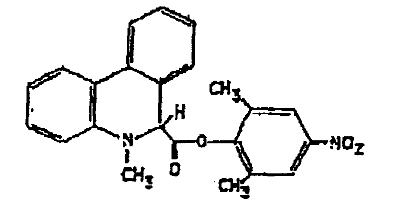

31. Composé selon l'une quelconque des revendications 1 à 4 et 19 à 24 qui est :

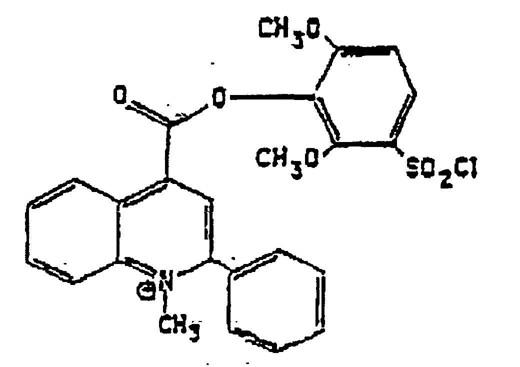

32. Composé selon l'une quelconque des revendications 1 à 4 et 19 à 24 qui est :

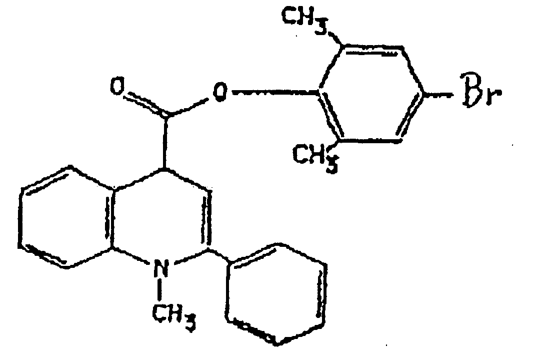

33. Composé selon l'une quelconque des revendications 1 à 4, 19, 20, 23 et 24 qui est :

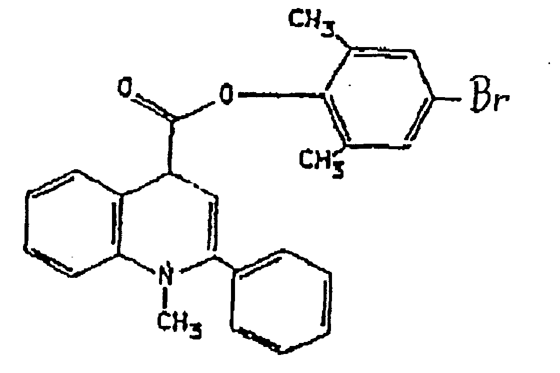

34. Composé selon l'une quelconque des revendications 11 à 14 qui est le (2,6-diméthoxy-3-chloro-sulfonyl)phényl-N-méthylacridan-9-éthoxy-9-carboxylate, le (2,6-diméthoxy-3-chlorosulfonyl)phényl-N-méthylacridan-9-méthoxy-9-carboxylate ou le (2,6-diméthyl-3-chloro-sulfonyl)phényl-N-méthylacridan-9-méthoxy-9-carboxylate.

35. Composé selon l'une quelconque des revendications 15 à 21 et 23 à 25 qui est ;

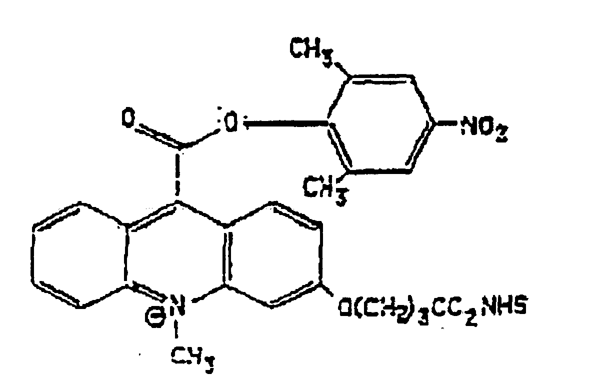

36. Composé selon l'une quelconque des revendications 15 à 21 et 23 à 25 qui est :

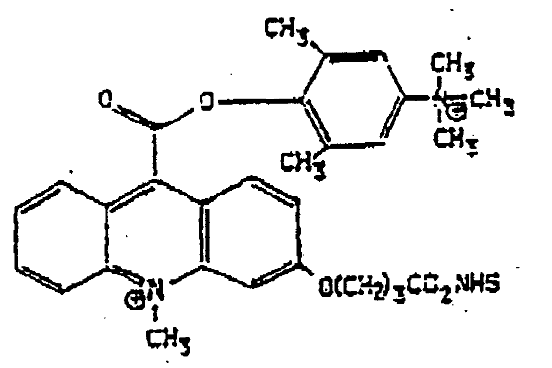

37. Composé selon l'une quelconque des revendications 15 à 21 et 23 à 25 qui est ;

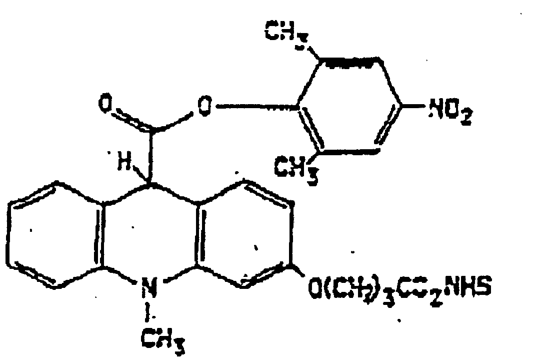

38. Composé selon l'une quelconque des revendications 15 à 21 et 23 à 25 qui est ;

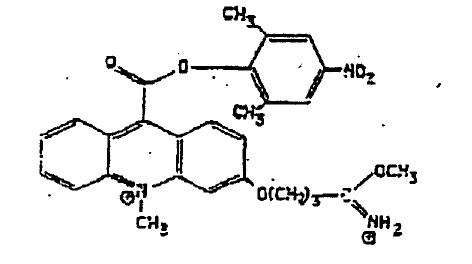

39. Conjugué comprenant un composé chimioluminescent selon l'une quelconque des revendications 1 à 38 lié à un partenaire de liaison spécifique pour une espèce biologique, biochimique ou chimique.

40. Conjugué selon la revendication 39 qui peut se lier spécifiquement par une réaction immunologique, une réaction de liaison de protéine ou par hybridation nucléique.

41. Composition comprenant un composé chimioluminescent selon l'une quelconque des revendications 1 à 38 ou un conjugué selon la revendication 39 ou 40.

42. Trousse d'essai de liaison spécifique comprenant un flacon contenant une composition selon la revendication 41.

43. Essai de liaison spécifique pour la détection de la présence d'un analyte dans un échantillon dans lequel l'essai utilise un conjugué chimioluminescent selon la revendication 39 ou 40 ou un composé chimioluminescent selon l'une quelconque des revendications 1 à 38 lié à un matériau de liaison spécifique où la présence de l'analyte dans l'échantillon est proportionnelle à la formation d'un ou plusieurs produits de réaction de liaison spécifique contenant le conjugué, l'essai étant effectué :
en permettant, sous des conditions appropriées, la formation substantielle d'un ou plusieurs produits de réaction de liaison spécifique contenant le conjugué ; et
en mesurant la chimioluminescence de soit (i) un ou plusieurs produits de réaction de liaison spécifique soit (ii) le conjugué non contenu dans les produits de réaction de liaison.

44. Essai selon la revendication 43 dans lequel le matériau de liaison spécifique est capable de se lier de manière spécifique avec l'analyte et le produit de réaction de liaison spécifique est un complexe analyte-conjugué, l'essai étant effectué :
en permettant, sous des conditions appropriées, la formation substantielle du complexe analyte-conjugué ; et
en mesurant la chimioluminescence de soit (i) le complexe analyte-conjugué soit (ii) le conjugué non contenu dans le complexe analyte-conjugué.

45. Essai selon la revendication 44 qui est un essai d'antigène de surface et le matériau de liaison spécifique est un anticorps qui est spécifique pour un antigène de surface sur une cellule.

46. Essai selon la revendication 44 ou 45 qui utilise en outre un réactif capable de se lier de manière spécifique avec à la fois (i) l'analyte, pour former un complexe analyte-réactif, et (ii) avec le matériau de liaison spécifique, pour former un complexe conjugué-réactif qui est le produit de réaction de liaison spécifique, l'essai étant effectué :
en permettant, sous des conditions appropriées, la formation substantielle du complexe analyte-réactif et le complexe conjugue-réactif ; et
en mesurant la chimioluminescence de soit (i) le complexe conjugué-réactif, soit (ii) le conjugué non contenu dans le complexe conjugué-réactif.

47. Essai selon la revendication 43 qui est un essai compétitif.

48. Essai selon la revendication 46 ou 47 dans lequel le réactif est attaché à une phase solide ou des complexes contenant le réactif sont précipités.

49. Essai selon la revendication 43 qui est un essai de saturation séquentiel et l'analyte est d'abord mis à réagir avec le réactif avant la réaction du conjugué avec le restant de réactif qui n'a pas réagi.

50. Essai selon la revendication 43 qui est un essai de déplacement compétitif où le conjugué déplace de manière compétitive l'analyte qui est déjà lié au réactif.

51. Essai selon la revendication 43 qui est un essai d'étouffement et le réactif est attaché à une partie jouant le rôle d'étouffoir.

52. Essai selon la revendication 51 dans lequel la partie étouffoir réduit ou éteint la chimioluminescence du composé quand elle est amenée à étroite proximité du composé.

53. Essai selon la revendication 43 dans lequel le matériau de liaison spécifique est capable de se lier de manière spécifique avec l'analyte et l'essai utilise en outre un réactif capable de liaison spécifique avec l'analyte pour former un complexe réactif-analyte-conjugué qui est le produit de réaction de liaison spécifique, l'essai étant effectué ;
en permettant, sous des conditions appropriées, la formation substantielle du complexe réactif-analyte-conjugué ; et
en mesurant la chimioluminescence de soit (i) le complexe réactif-analyte-conjugué, soit (ii) le conjugué non contenu dans le complexe réactif-analyte-conjugué.

54. Essai selon la revendication 53 qui est un essai en sandwich.

55. Essai selon la revendication 53 ou 54 dans lequel le réactif est attaché à une phase solide.

56. Essai selon l'une quelconque des revendications 53 à 55 dans lequel le réactif est attaché à une tigette, une bille, un tube ou une feuille de papier ou de polymère.

57. Essai selon la revendication 55 ou 56 dans lequel le réactif attaché à la phase solide est mis à réagir avec l'analyte dans l'échantillon après séparation de la phase solide contenant l'analyte complexé de l'échantillon restant et le complexe est alors mis à réagir avec le conjugué.

58. Essai de liaison spécifique pour détecter la présence d'un analyte dans un échantillon dans lequel l'essai utilise une partie chimioluminescente comprenant un composé chimioluminescent selon l'une quelconque des revendications 1 à 38, et la présence de l'analyte dans l'échantillon est proportionnelle à la formation d'un ou plusieurs produits de réaction de liaison spécifique ne contenant pas le composé chimioluminescent et le composé émet de la chimioluminescence proportionnelle à la formation des produits de réaction de liaison spécifique, l'essai étant effectué :
en permettant, sous des conditions appropriées, la formation substantielle des produits de réaction de liaison spécifique ; et
en mesurant la chimioluminescence du composé causée par la formation des produits de réaction de liaison spécifique.

59. Essai selon la revendication 58 qui utilise en outre un réactif capable de liaison avec l'analyte pour former un complexe analyte-réactif et dans lequel le composé émet de la chimioluminescence proportionnelle à la formation du complexe analyte-réactif.

60. Essai selon la revendication 59 qui est un essai enzyme-substrat où la formation du complexe enzyme-substrat provoque la chimioluminescence du composé.

61. Essai selon la revendication 60 dans lequel l'analyte est le substrat glucose et le réactif est l'enzyme glucose oxydase.

62. Essai selon la revendication 61 dans lequel la formation du produit de réaction de liaison spécifique favorise ou inhibe la chimioluminescence du composé.

63. Essai selon la revendication 58 dans lequel un premier réactif, qui est en réaction croisée avec l'analyte, est attaché à une enzyme près de son site actif et un second réactif, qui est spécifique à la fois de l'analyte et d'un matériau immunoréactif qui est ajouté à l'échantillon et l'enzyme en présence d'un substrat.

64. Essai selon la revendication 63 dans lequel le second réactif se lie au premier réactif pour bloquer le site actif sur l'enzyme de sorte que le substrat ne peut se lier à l'enzyme au site actif ou la liaison du substrat au site actif est diminuée de manière significative.

65. Essai selon l'une quelconque des revendications 43 à 64 qui est un essai immunologique, un essai de liaison de protéine ou un essai d'hybridation d'acide nucléique.

66. Essai selon la revendication 65 qui est un essai d'hybridation d'acide nucléique où tout ADN ou ARN double brin est converti en une forme simple brin avant l'hybridation et est immobilisé sur un support ou transféré par électrophorèse sur une matrice de gel.

67. Essai selon l'une quelconque des revendications 43 à 66 qui est un essai hétérogène où les produits de réaction, dont la formation est proportionnelle à la présence de l'analyte dans l'échantillon, sont séparés des autres produits de la réaction.

68. Essai selon l'une quelconque des revendications 43 à 66 qui est un essai homogène où les produits de réaction ne sont pas séparés des autres produits de la réaction et la chimioluminescence est mesurée à partir du mélange total de l'essai.

69. Essai selon l'une quelconque des revendications. 43 à 68 dans lequel la mesure de la chimioluminescence inclut, si nécessaire, le traitement du composé avec un acide pour cliver Z, si Z est présent et quand il représente un groupement RₙX, et en provoquant la chimioluminescence si le composé de formation du produit de réaction n'est pas déjà de ce type.
